# EUROPEAN PATENT SPECIFICATION

(11) **EP 1 976 996 B1**
(45) Date of publication and mention of the grant of the patent: **14.01.2015**
(21) Application number: 06827161.8
(22) Date of filing: 31.10.2006
(51) Int. Cl.: C12P 19/04

(54) **HIGH VISCOSITY DIUTAN GUMS AND METHODS OF PRODUCING**
HOCHVISKOSE DIUTANGUMMIS UND HERSTELLUNGSVERFAHREN
GOMMES DE DIUTANE DE VISCOSITÉ ÉLEVÉE ET PROCÉDÉS DE PRODUCTION DE CELLES-CI

(30) Priority: 01.11.2005 US 264268; 01.11.2005 US 264279
(43) Date of publication of application: 08.10.2008
(60) Divisional application: 11185331.3
(73) Proprietor: CP Kelco U.S., Inc., Atlanta, GA 30339 (US)
(72) Inventor: PATEL, Yamini, N., San Diego, CA 92128 (US); COLEMAN, Russell, San Diego, CA 92101 (US); MATZKE, Steven, San Diego, CA 92111 (US); HARDING, Nancy, California 92129 (US)
(74) Representative: Kiernan, Lisa
(86) International application number: PCT/US2006/042456
(87) International publication number: WO 2007/053612

(56) References cited:
- EP-A- 1 586 652
- WO-A-01/29196
- WO-A-2006/096269
- US-A- 5 175 278
- US-A- 5 854 034
- US-A- 6 103 671
- US-A1- 2004 259 839
- US-B1- 6 696 561
- YAMAZAKI M ET AL: "LINKAGE OF GENES ESSENTIAL FOR SYNTHESIS OF A POLYSACCHARIDE CAPSULE IN SPHINGOMONAS STRAIN S88" JOURNAL OF BACTERIOLOGY, AMERICAN SOCIETY FOR MICROBIOLOGY, US, vol. 178, no. 9, 1 May 1996 (1996-05-01), pages 2676-2687, XP002035687 ISSN: 0021-9193
- COLEMAN RJ, PATEL YN, HARDING NE.: "Identification and organization of genes for diutan polysaccharide synthesis from Sphingomonas sp. ATCC 53159." J IND MICROBIOL BIOTECHNOL, vol. 35, no. 4, 22 January 2008 (2008-01-22), - 22 January 2008 (2008-01-22) pages 263-274, XP002526660

### SEQUENCE LISTING

<110> Harding, Nancy
   Patel, Yamini
<120> HIGH VISCOSITY DIUTAN GUMS
<130> 05-506, 05-506A
<160> 45
<170> FastSEQ for Windows Version 4.0
<210> 1
   <211> 26278
   <212> DNA
   <213> Sphingomonas sp. ATCC53159
<400> 1
   gatcaacggc gccttgctcg gacggcacaa attcgtcctg gtcaatgtgt ccacggtcgc 60
   ctcttcgata ctgttccagc tgttcccgct tgtcgtcgcc tggatgatcg gcccggacct 120
   gcgaacgctg ctgatcgccg cgctcgttgg ccgggcggtt ccgatgatcg gcatgctgcc 180
   cgcgctgtat cgaaaccttt tgcgcggcaa cacgccgcgt tttcacgcca gcgaggcgcg 240
   cttcctgata ggctatggcg ggtgggcctc gctcacgacc gtggtagcga ccgtgctcat 300
   gatggcggac cgcttcctga ttggcgcact tcttgggccc gtcgccgtga ccatctacac 360
   ggcccccctg caactcgcac agcgcgtatc gctgctgccc tccgcactgt ccgccgcgct 420
   gttcccgcgc ctgcccagcg cgacgccggc ggagcgcatg gcgcttcaga tccgctcgct 480
   gtcgctgatc atgggcggcc ttaccgggat gatcggcggc ggactattgc tggccgcgcc 540
   gtttctcgat ctctggatcg gcaagtcgct cggccatgcg ggaacgccgg tcgcgctctt 600
   cctgttcttc ggcgcatggt ggaatgcgct ggcgatcatt tcgttcagcg gcctgcaggc 660
   gagcggacgg ccgaaagcga gcgcgatcgt ccagggggca gagctgctac ccgtgttgat 720
   cgcgctgtat gcagggatcc gatggggcgg cgtgaccggc gccgcagcgg tctttctggg 780
   acgctccgcc ctggatttcg tcctgttgac ctggcaggca ggcctgctcc gccagacggt 840
   gaagcaagta tccgtatgcg gcgccgttct caccgtcgcg atgctcgtgg gcgcgaccta 900
   tcgctattcg gtgccgctct ggtgcgtact cagcgcctgc tgcctggtcg cgctggcagc 960
   ctgctcctgg tggacattgg cgcgccagga caaggcactg ctgattggac gattgagccg 1020
   aattctacca aagcagcggc aactcgacct atagcctttc cgcaatgcac cgatggacca 1080
   caccaacccg ttttaattga cacacacaaa tgctacaccg acaaagacac aggccgagag 1140
   cgatatagaa gcgctatgcc tagccccagc gtcataaaga tgaacgggtc attgtcacct 1200
   tgcgacagga ctgaccgcgt atttaaaaga acagccagga aagttgctac ggcgagctca 1260
   agcgggtagc catctccgct catcttaaga ccacgaaacg cgagcaaaat cattaacgta 1320
   atcatcgtgc cgtatagcga aacaaaaccc agcaagccgt aatcagccgc tacggacagg 1380
   aaaccactgt cgatcgatag gaagccttgc tgattacgcc acccgacagc gccagcaccc 1440
   tctcccgggc catagccgaa gaaagggcgg cgagcgatgg caggcacgcc caagcgaaac 1500
   tgctcctgcc tgccttgatt gctaagttga gaagcgcctc caccgagaac acggttgtgg 1560
   acggcaggca cgaacatgac cgccagcgac agcgccacca tcaaggcggg atacgtcaac 1620
   gtcagcgaaa tgccgacaag cccgcccttt gtggtccgcc accgccgaat tgcccaaata 1680
   agcaaataca cggtatgcgc caccaatccc cccaccattg ccagtcgaga accgctaaga 1740
   aatccggacg caactacaag aaaatcgaag aaaatccaaa atgccaatct ccctacgcca 1800
   cgggaattcg ctatacggtg cagcacgaaa ggaatcgtca aagccgtcaa ctctccccag 1860
   acaagcggac tgctgaaagt cgtcaaaacg cggtaagtac cccggaaacc gggcgtaagc 1920
   actacggtaa gaaactgctc atcaacgcgc aggaagctcg gaatcgagta ggcccagagg 1980
   acgtgcttca cccggaactc cagcacgcca atcgccatca gcacgcccac gcaccaaaac 2040
   aagcgcgtaa cccaccactc cggggtgcgc gtgtcggtcc cgatcagcca tagcgagatg 2100
   aatgccatcg gcgtcaccgt cagaacgatg ccaatcaacc gcggaattgt ttgcgaggcc 2160
   gctggggtcg caatggaggc gacgatctgg accataatga aggcaagcaa tagtcgcgat 2220
   gggatcggcg ccgcccgcat aatcgccgcc atctcggatc gaaacttttt cgagaccgaa 2280
   agcgagatca tgagcgtgag caatgcgatc gaaccgatca tccgcctgat cgagatccaa 2340
   ggcaaaccac caacgctgag cgcaagatag ttcggccaca cgagcgccgc caccatatag 2400
   gcgaggtata gttttgccag caggcgagta ggcgcctgcc gcgcctcggg tagcgcccag 2460
   atcactacga gcgccatcag aacgaggggc acggccggga tcgccagcat ctggagcggc 2520
   agaactgcgg cgagcaggcc gtagactgcg gcaagaaaca tcacgctgac cagcagaacg 2580
   gtacgccgcg ccgcgatcgt cacgcctgat cgctcggctt tgtagacggg cagtaccggg 2640
   atcgctggct ttgtcagaaa ccgaaccagt cgcaacctgc gaagccgctg catcgctccg 2700
   tggaaggccg ggcgacgaaa cgccgaggta gtcgtcatct gcaagtcccc aacaagtccc 2760
   caagaggcgc tgccgctcgc atgatcgaag ggttcgcgaa aagcaaggtc gatacgccgc 2820
   actccctgcg atgtgccgcc ggatcgcagg agggcacggg cggcgccggc gcaaggccgc 2880
   tcaccgcccg cccccgctca ggcgcggtac aggttgtact gatccgccgt agcgctcagt 2940
   gtcgccgcgc tgcggattgc gcccatcgcc cccgcggtca tcatgtcgac accgatcttg 3000
   ctgacgagcg cgatctgcga ggacgcggca gtacctatag acagcgtact gcccaccgtg 3060
   gccaccgtcg caagcggcgt tgccgtgcta gcggcgccgg cacccgccag cagcgcagcg 3120
   gcctgcgcgg ccgcgccggt gacgaggctg tccttgaccg tcgccgccgc gctggcgctc 3180
   gacgcggtca ccagcgcctg cacctgggcg gcgctgatcg cgccatcgcg gatctcgatg 3240
   tcgccgaccg ttccgctgaa tgcggtcgag aacgggctgc cgacatacag cccccaggat 3300
   tcggcgggcc gggtcgtgcc ggtcatcgtc gctgtgccgc gttgcatgcc gtctacgtac 3360
   agaatcgcgg ttttccgcgt gctgtcgtag gtcagggcga tcttgtgtgt ggcagcatca 3420
   agcagcttgg cgccgctcgt caccattgtc tggctgacgc ccgcggcgtt gcgcatggtg 3480
   aagctcagtt ccccattggc ctgcagcgaa accgaccagc tctggaagat gccaagaatt 3540
   tgcccggccg tggccgtagc cgagtcccgc ttgaggtcga agctgagcgt gaacgccgac 3600
   aatgcgtaaa tctgccgcga atagctccgg tttagttcca cccccgtgcc cgtcgagacg 3660
   tggaaggcgc tgcccacgac cgccgacacg tccaccgcct ttgtcgtctg gccggtattc 3720
   cagtgcgaaa ggtccacgac gccgctgttg ctgaacgaca gatcgagcag cagcgacgga 3780
   tttgccgcct tcgcagtcga cagttcggta gtcacctgag cggcagcagc gctcgacacg 3840
   ggcggctggt acccgacgcc gggaacgatc aaatcgctga gccgcgccgt agccccatcg 3900
   ttgaggccat agatcttgcg gatcgttgcc gagtcactcg tcagcgtacg attgcctgtc 3960
   tgcacgatat tgctcgagga gcttgtgacg gtgatcaggt ccgcaacatt gttcttgatc 4020
   gtcgcgccat tggttttgtc gaggcgaatc caaaatgatg tgccatccac ttgcgatatc 4080
   acgctattgg attcgatatt gacattaacg ccgttaacaa cgttgatacc gtggtaataa 4140
   ccattcagat agataagatt gtttttgatg tttacattga catagggaag attaccggcc 4200
   tcgtcattca tgaaaatccc ttgcgcgcca gagcccgcgc cctgcatgat gacgttattg 4260
   gagatggtga tgttggtatt gcccttgacc ttgcccgccg tgaagaactg aatggcgtcg 4320
   ggatgttcgg tgcccacggg aaacaggttc gtgaacgaat ttccgtcgat gacaagattg 4380
   ttcatctcag tgaagttcgt atgatcgcgc cggttgtcgt ggaagctgct gttctggacc 4440
   accatgccat cgacgttgta ggcctcaagg cccagaccga agtggtcgat agacgaattc 4500
   tgcatcgtca ccgacgtgct gttgcgcacg aacaagcccg cccccttcga gagcgaaggg 4560
   tcaccagtgc cgccgctgaa ccgcacgccg tccaaaacga tgttggccga accctggatc 4620
   gtattcagtc gattccagtc atcggcgggc ttgtaatcgg tcgcagcgac catgtttttg 4680
   acggtaacgt tgctactgtt cccgatcacc agcttttgga tattgaccgg gttcgacgag 4740
   tcgagcgact caattgtcac catgctggta aacgtcttgg tcattacagt gagatctgtg 4800
   tagaccccgg cggcaagctt gatggtttcg ccacccttcg ccgccgcgat tgcagcattc 4860
   aactccgtct gattcttgac aatgatatcc ggcatgttga cttaccccgt acgcacgaac 4920
   ccgggccgat attgaccctt ccattgtcat aaataccaga acagccatga aatttgctcg 4980
   aagggataca gttaagaact cccttctacg gggccgcatg ccgggcccat gcacgcccga 5040
   ctttcgccgg caccgtctcg acggcgcaac acagtgcagc tactagggtg cgatgcagat 5100
   gctcccaacg cccgatgtca gcatactcgt ggtcgctttc aactcgaccg agtatatcga 5160
   agactgcctg cgcggcatcg ccgaaggagc gggcaagacc ccccacgaag ttctgctgat 5220
   cgacaatggc gacgggcgaa ccgaagcgct ggtccggcag cggttccacc acgtccgcat 5280
   cgttcccagt gagggcaata ttggtttcgg ggccggcaat aatcgcctgg cagcgcaggc 5340
   tgccggcccg ctcctgctgc tcgtcaaccc cgatgccatt ccccagcccg gcgcaatcga 5400
   tcagttggtc acctttgcca aacagcatcc cgaggcggcg gcatggggcg gccgttccta 5460
   ctcgcccagc ggcgatctag aacccgcaaa tttcatgtcc ctgccgacgc ccgccgactt 5520
   tctgacggcg attttcaacg cgcgtgcgct acgcagcggc gggctgcaag aaggcgcgac 5580
   cacccccgga gcggtcgagg tgttgaatgg cggcttcatg atggtacgca ccgatgtctg 5640
   gcaggcgatc ggcggttttg acgagagctt ttttctttat tcggaagaga tcgatctctt 5700
   ccagcgaatc cgcacgttgg ggcacaaggt gctcgtcgac ccctcggtca aagtggtaca 5760
   caatacgggg agtggtcagt cgatgtccca gaaccgcctg atgtatctca cgaccgggcg 5820
   catgcactat gcgcgaaagc attttggcgc actcggcacc cttgccaccg ggtgcgcgct 5880
   ttggctgatc gccgccaaat acacgttggt cggggcggca ctctggcgcc tgtcgccgcg 5940
   gacgggcacg cgatacaaag agctgagcaa cgggtggcgt gccgtattta gcaatcctgg 6000
   ccgatggtgg agcggctatc cgcgtcgcta aaagtccagc tccccccccc ctaaaggcgc 6060
   cgttgggagg cggacgcatc gttgcaacaa cgcgcccgcc tttcagacct tcagttcccc 6120
   gccggcgttg cgccgctgcc gcgaagctgc ggcggtgcgc tgtagccggc ctgatatttc 6180
   acggtttccc gcgccttctt caggcggtcg ttgagctgtg cgtcagccgc cttgccgaag 6240
   cgctcggtac gcagcccgct gagcgcgatc tcgcgcgcct ggtcggccgg caccggcagc 6300
   accgtggtcg acgtgatgat attcgcggtc agtccctgct gggtcggcag gatgaacatc 6360
   tcctgtgccg gcagcgacgc gatcttggca gcgatttccg gcggcagcgc agcggtgtcg 6420
   atctgcgacg gcgcgcgacg gaactggaca ttgtccgccg agagcttggc ggttagctgg 6480
   tccagcgtct tcagcggcgc gaattgcttg agctttgcgg ccgagctcgg cggagcgaag 6540
   acgacctgat cgatcgcgta gatcttgcgc tgcgcgaacc gctccggatg cgcggcctga 6600
   tatttctcga tctcggcatc ggtcggctgg gcgatgccgc cggcgatctt gtcgcgcagc 6660
   atggcggtga ggatcagctc gtcggcccgg cgctcctgga tcaggaaggc aggcgtcttg 6720
   tccagcttct gctcgcgggc gaccttggcg aggatcttgc gctcgatgat gcgctgcagc 6780
   gccagctgct cggccagctt gcgatcggtc cccgggggta cctgggaggc ctgcagttcg 6840
   gcattcagct cgaagacggt gatttcttcg ccatcgacgc tggcgaccac ctgccccttg 6900
   tcgagcttgc cgcccttgcc gccacatccg gagacggcca gcgcggccgc agccaccgcc 6960
   gtaaccaggt acaatttctt catgaagacc tccccgccgg cacggaattg cgcacggcac 7020
   aaacttctac ttgaacctat tcggacgggc gggcatccgc aatagcgttg gcagtgcagc 7080
   atggttctaa gcggagccag gcggcaacaa gggggacgag atggcagaag cgaacgcggt 7140
   agatggaaag gcctccaagc cgctgaaaat gtgccttgca gcgtcgggcg gcggccatct 7200
   ccggcaaatc ctcgatctgg aatcggtgtg gcgcgaacac gattatttct tcgttactga 7260
   agataccgcg ctcggccgga gccttgccga aaaacatccc gtcgaactgg tggagcacta 7320
   tgcgctcggc caggccaagc tgggccatcc cttgcgcatg ctgggcggcg catggcgcaa 7380
   cctgcgccag agcctttcga tcctgcgccg gcacaagccg gatgtggtga tttccaccgg 7440
   cgcgggcgca gtctatttca ccgcgctgct cgccaaactg tcgggcgcca agttcgtcca 7500
   tatcgaaagc ttcgcgcgct tcgaccaccc gtctgccttc ggcaagatgg tgaagggcat 7560
   cgcgacggtg acgatcgtcc agtcggcggc gctgaaagaa acctggcctg atgccgagct 7620
   gttcgatccg ttccgcctgc tcgatacacc gcgcccgccc aagcaggcgc taatcttcgc 7680
   gacggtcggc gccaccctgc ccttcccgcg gctggtgcag gcagtgctcg acctgaagcg 7740
   cgccggcggg ctgccgggca agctgatcct gcaatatggc gaccaggacc tgcccgatcc 7800
   cggcatcccc gacgtcgaga tccgccgtac catcccgttc gacgatctgc agctgctgct 7860
   gcgcgatgcg gatatggtga tatgccacgg cggcaccgga tcgctggtca cggcgctgcg 7920
   cgccggctgc cgggtcgtcg cctttccgcg ccgccacgat ctgggcgagc attatgacga 7980
   tcaccaggaa gagatcgccc agaccttcgc cgaccggggc ctgctccagg cggtgcgcga 8040
   cgagcgccag ctcggcgccg ctgtggaagc ggccaaggca accgagccgc agctggcgac 8100
   caccgaccac acggccctcg cggcgcggct gcgccagctg ctggcgcagt ggagtgccaa 8160
   gcgatgagca cgccccggat cagcgtcgtc atcccgcact ataacgatcc gcaatccttg 8220
   cggctctgcc tggatgcgct ggagcggcag acgatcggtc gcgacgcgtt cgagatcatc 8280
   gtcggcgaca acaattcgcc ctgtgggctc gcggcggtgg aggcggcggt cgccggacgt 8340
   gcgcggatcg tgaccattct ggaaaagggg gcgggccccg cgcgcaacgg ggcggcagcc 8400
   gcagcgcgtg gcgagatcct cgcctttacc gacagtgact gcgtggtgga gcccggctgg 8460
   ctggcgggcg gcacgaccag ggtcgcgcct ggccgtttca tcggcgggca catgtatgtg 8520
   cgcaagcccg aagggccgcc gaacggcgcc gaggcgctgg agatggcgct ggcgttcgac 8580
   aatgaaggct atgtgcggcg cacccagttc acggtcaccg caaacctgtt cgtgatgcgc 8640
   gccgatttcg aacgggtcgg cggcttccgc gttggcgtgt ccgaggatct ggaatggtgc 8700
   caccgggcga tcgccagcgg cctcaccatc aactatgcac cggatgcatc ggtgggccac 8760
   ccgccccggc ccgactggtc ggccctgctg gtgaagacgc ggcgcatcca gcgcgaactc 8820
   tatctgttca acatcgagcg gccgaagggc aggctgcgct ggctggtccg ttccgtggcg 8880
   caaccggcga tgatcccaca ggacgtggcc aagatcctgc gcacaccggg taccaagggc 8940
   gcgcgcctcg ctgcggtcac cacgctggtc cggctgcggc tgtggcgcgg cggcgccggc 9000
   ttgttgcagt tgctcggccg cgacatctga tcgaccggcg atcggccgac gagcgcgtcg 9060
   ccggccgatc gcattgcatc agacggtggc cagcgcgtct tccagcgtgc cgctgtcgag 9120
   ccgcaggcgg ccgatcatca gccacagata gaccggcagc gtatcgtcgg tgaagcggaa 9180
   gcggcaatcg ccgtcctgcg tttcggattc gaggccgagt tgaccggtga gctcgcccag 9240
   ctcctgctcg acctgcgccg ccgtgatgtg cgcgcccggc agcagatcca ccacggcttg 9300
   gccgctgaac cagccatccg ccgagcgcga ggcctcgccc agcgccgcga cgagtggatc 9360
   gtagcggccg ccgacgaact tgcgcatctc gatcaccgcg cgcggcggca tgcggccctc 9420
   gatctcaagg atcgcctggt cgagcgcacg acgcagatgc ccggcgtcga ccgtgaggcg 9480
   gccctggtcc agggcttcca gcgcggaatg gtggcacagc agccgcgcga aatagggcga 9540
   ccccagcgcg agcaggtgga tcatgtgagt caggtccgga tcgaagcgaa cgcccgaggc 9600
   ggtttcgccg agcgcgatca tctcctgcac ctccgattcc tccagccggg gcatcggcag 9660
   gccgatgacg ttgcggcgga tcgacggcgc ataaccgatc agctcctgca ggttcgaggc 9720
   gacgcccgcg atcaccagct ggacgcgcgc cgaacggtcc gacaggttct tgatcagctc 9780
   ggcgacctgc tgacggaagg cggaatcgct gacgcgatca tattcgtcga ggatgatcag 9840
   cacgcgtgtg cccgtgatgt cggcgcacag gtcggccagt tcgccgggcc cgaagctgcc 9900
   cgtcggcagg cggtcggcca agttgccgcc gctctccgcc tcgccggcgt tgggcgccac 9960
   gccgcgatgg aacagcagcg gcacgtcttc cagcacggcg cggaagacat cgctgaaatt 10020
   cgcgttcgca ccgcaggtcg catagctgac gatatagctg gattcgcggg cgacatcggt 10080
   cagcacgtgg agcagcgagg tcttgccgat gccgcgctcg ccatagagca cgacatggct 10140
   gcgctggctc tcgatcgagg agattaggcg cgccagcacg ccgaggcgcc cggcgaagct 10200
   cgaccgatcg gccaccggct gggtgggtgt gaagaaggtc gccagcgcga accgggcgcg 10260
   cgtgatctcg cggcgctcgt cgcggcggcg atccagcggg cggtccagcg cggaggcacg 10320
   gaaggttggg aaatccgggc gaccacggcc gctatgggca tcgcgatgcg gcaccactgt 10380
   cgcagtcagc gggaaatagc cctcttcttc aggttcttct cgacggccga acggccacaa 10440
   gaatctcagc gcggaaccta cagccactcg aacacctctt aaattcgtgc gccatcggca 10500
   ccgacggcgc accctggttc gcgccccctg gcgccccctc ctaacgaacc cacgccttgc 10560
   ctggcctatc ggcgcttgaa gaactcgtac ggtttgatca ccaaggcgat gtacgccagg 10620
   accagagcga tcgtcaaaat tgcaaagacg tgataattct cattgcccag ataattggcg 10680
   acggcgcaac cgactgcggg cggcaaatag ctgatcatcg tgtcccggac tgccgaatcg 10740
   gcttgggacc gttgcaggaa tataacgatc aggccggcaa atatcgcgat ggtgacccaa 10800
   tcatagggcg tctgcatgca tgtcctttct attcgacacc ggaatcgaac catttccggc 10860
   gacgctattg cacgcactag cagtgcgcgc ggccgctcgc taggtagcgc cgcaccggat 10920
   aaaccgacgt taagatggcg cggctcgatc gaaatggagt caaacgggct tgcccggccg 10980
   accgaagcat ggcgccatgg cgcatgcacc gtattgtgac cacgcaaacc gcgagggtca 11040
   ttcgatgcgg ttgcttgtac aggaggccat tgataatgaa gccgagaccc gggggaacct 11100
   ttatgcaagt aaatttcaat cgacaggctc gcaagctcgg tgccggcaat gcgctcgcgc 11160
   ggggggggcc cgtgcttgcg ctgcttgcga ccgcggcatg gacacaacct gcgctggcgc 11220
   agcgacaggc atttgagtcc cgcccctccg gtagcgagcg acaggtcgat attcgcgcga 11280
   cggggtcgct ggaatatgac gacaacgtcg tgctgaacga ccagcggatc acggacggcg 11340
   cgcgtggcga tgtgatcgca tcgcccgggc tggacgtgac cctagttctg ccccgcgcca 11400
   ccgggcagct ctacctcacc ggcaatgtcg gatatcgctt ttacaagcga tataccaact 11460
   ttaaccgcga gcagatctcg ctcaccggcg gcgcagatca gcggttcgcc tcctgcgtcg 11520
   tgcacgggga agtcggctat cagcgccacc tcaccgacct gtccagcatc ttgatccagg 11580
   acaccacgcc tgcgctcaac aacaccgaag aggcccggca gtacaccgcg gatatcggct 11640
   gcggcgcgac ctacggcctg cggcctgccg tttcctacac ccgcaacgaa gtgcgcaaca 11700
   gccttgccga gcgccgatac gcggactcga ataccaacac ctttaccgca cagcttggcc 11760
   tgacttcgcc tgccctgggg accgtggcgg tatttgggcg tatgtccgac agcagctatg 11820
   tccatcgcgt ccttcccggc attaccggcc aggacgggat gaagagctac gcggccggcg 11880
   tccagctcga gcgctcggtg gccaaccgac tccatttcaa cggctcggtg aattacaccg 11940
   aggttgaccc aaagctcgca tccaccaaag gattcaaggg cgtaggattt aacgtttccg 12000
   gcgattatgc tggtgatcag tacagcctcc aattgctggc ttcacgatcg ccccagcctt 12060
   cacttcttct gttcgtgggt tacgagattg tgacagcggt ttcggcgaat gcgacgcgcc 12120
   ggctgagcga tcgcattcag atatcgctgc aaggcagccg aacctggcgc gagctcgcgt 12180
   cttcgcggct gctcaccaac gtgccgattt ccggcaacga caacacctcg acgttgttcg 12240
   cctccgctac cttccggccg aatcgccggc tgagctttgt gctgggtgcc ggccttcagc 12300
   ggcgcaccag caacacgcag ctatacagtt acagctccaa acgcatcaat ctctcgacgt 12360
   cgctttcgct ctgacaaggg ccgtaatcat gcatatcaag aatcgcttcg tgaatatctc 12420
   gacgttggcc atcgccgccg cgctggccac gccggcggcg gcgcagatcc ccacgcggtc 12480
   cgtgcccgcg ccggcccgcc cgcggcctgc aacgccgccg gcgcaacagc agaaccaggc 12540
   gccgtcgacg cccgcagcgg caaccccggc gcagaccgcc gcaaccgttg cccctgcagc 12600
   aaccgcaccc gcaggttaca aaatcggcgt ggacgacgtg atcgaggccg acgtgctcgg 12660
   ccagaccgac ttcaagacgc gcgcccgtgt gcaggcggac ggcacggtga ccctgcccta 12720
   tctgggcgcc gtgcaggtca agggcgagac cgcgacctcg ctcgccgaaa agctggccgg 12780
   gctgctgcgc gccggcggct attatgccaa gccgatcgtc agcgtcgaaa tcgtcggttt 12840
   cgtcagcaac tatgtgacgg tgctgggcca ggtgaacagt tccggcctgc agccggtcga 12900
   ccgcggctat cacgtttccg agatcatcgc ccgtgccggc ggcctgcgcc ccgaagcggc 12960
   cgatttcgtc gttctcaccc gcgccgatgg ctccagcgcc aagctggact acaagaagct 13020
   cgcccaaggt ggccccaatg acgatccgat ggtgacgccc ggggacaagg tctttgtccc 13080
   ggaagtcgag catttctaca tttatggtca aattaacgcg cctggcgtat acgcgattcg 13140
   atcggacatg acgctccgtc gcgcgctggc ccagggcggt gggcttgccc ccgcaggctc 13200
   cgtcaagcgt gtgaaggtca cgcgggatgg caatgaactc aagttgaagc tggacgatcc 13260
   gattctccca ggcgacacga tcgtcatcgg cgaacgattg ttctgatctt ggcaacgatg 13320
   gcagcggacg aggcccacca gtgaatatca ttcagttctt ccgcattctg tgggtgcgcc 13380
   gatggatcat cctcccggcg tttctcgttt gcgttaccac tgccaccatt gtggtccagt 13440
   ttctgcccga acgctacaag gccactacgc gggtggtgct cgacacgttt aagcccgatc 13500
   ccgtcaccgg acaggtgatg agctcgcagt tcatgcgcgc ctatgtcgag actcagaccc 13560
   agctgatcga ggactatgcg accgccggtc gcgtggtcga cgaactgggc tgggtgaatg 13620
   atccggcgaa catctccgcg ttcaacaact cgtccgcggc tgccaccggc gacatccgcc 13680
   gctggctcgc caagcagatc atcgacaata ccaaggccga tgtgatggag gggagcaaca 13740
   tcctcgaaat cacctattcg gacagctcgc ccgagcgcgc cgaacgcatc gccaacctga 13800
   tccgcacctc gttcctcgcc cagtcgctcg ccgccaagcg ccaggccgcg accaagtcgg 13860
   ccgactggta cgcccagcag gccgaagctg cccgcgattc gctcgctgcg gcggtccagg 13920
   cccgcaccga tttcgtgaag aagaccggca tcgtgctgac cgaaaccggc gccgacctgg 13980
   aaacccagaa gctccagcag atcgaggggc agacgacgac cgccaccgcc ccggttgcca 14040
   tggcccccag cggcatgggc ccggcgcaga tgcagctcgc ccagatcgac cagcagatcc 14100
   agcaggcagc gaccagccta ggtccgaacc acccaacttt ccaggccttg cagcggcagc 14160
   gcgaagtgtt cgccaaggca gcggcggcgg aacgcgcgca ggcgaacggc gtatccggtc 14220
   cggcacgcgg ggccatcgaa agcgcagcca acgcccagcg cgcgcgggtt ctcggcaatc 14280
   gtcaggatgt cgacaagctt acgcagctgc agcgtgacgt ctcgctgaag caggatcagt 14340
   acatgaaggc ggcacagcgc gtcgccgatc tgcggctgga agcaagcagc aacgatgtcg 14400
   gcatgtcgac gctcagcgaa gcatcggcgc cggaaacgcc ctattacccc aaggtgccgc 14460
   tcatcatcgg tggtgcagcc ggcttcggcc tcgggctcgg tctgctggtc gcgctgctcg 14520
   tcgagctgct cggccgccgc gtccgcagcc ccgaggatct ggaagttgcg atcgatgcac 14580
   cggtgctggg cgtgatccag agccgcgcct cgcttgccgc ccgccttcgc cgcgcccaag 14640
   aaaccctcgg cgaaggtgcc gacacgcacg gagcttcagt aaactgatgg acgcgatgac 14700
   cagcgaaccg ctgcccgaag gcgatcgtcc gagcgccgtg ccgaccacgc cggatacgat 14760
   cggcatgctc gaataccagc tcgtcctctc cgatccgacc gggatcgagg cggaagcgat 14820
   ccgcgcgcta cgcacgcgca tcatgaccca gcacctccgc gagggccggc gcgcgctcgc 14880
   gatctgcgcc gcctcggcgg gatccggctg cagcttcacc gccgtcaatc tggcgacggc 14940
   gctggcgcag atcggcgtta agactgcgct ggtcgatgcc aatctgcgcg atcccagcat 15000
   cggcgcagcc ttcggcctcg ccgccgacaa gcccggcctg gccgattatc tcgcctcggg 15060
   cgatgtcgac ctcgcctcga tcatccatgc gacccgcctc gaccagctct cgatcatccc 15120
   ggccgggcat gtcgagcaca gcccgcagga actgctcgcg tccgaacagt tccatgatct 15180
   ggcgacgcag ctgctgcgcg agttcgacat cacgatcttc gacaccacgg cgtccaacac 15240
   ctgcgccgac gcgcagcgtg tcgcgcatat cgccggctat gcgatcatcg tggcgcgcaa 15300
   ggatgcgagc tacatccgcg acgtgaacac gctcagccgc acgctgcgtg cagaccgcac 15360
   caacgtcatc ggctgcgtac tgaacggcta ttgatttgga ccatatggca gcgaccgcga 15420
   tgacgcggca gcaggagagg aagggcggtg gctattggct ggccgttgcc ggtcttgccg 15480
   cgctaaccat cccgaccttc atcaccctgg gtcgcgaggt ttggagtgcg gaaggcggcg 15540
   tgcagggtcc gatcgtgctc gccacgggcg cctggatgct ggcccgccag tgctcgacga 15600
   tcgaggcgct acgccgcccc ggcagcgtgc tgctcggcgc gctgttcctg ctggcgacgc 15660
   ttgccttcta caccgttgga cgggtgttcg acttcatcag tgtcgaaacc ttcggactgg 15720
   tcgcgaccta tctggtcgtc gcctatctct atttcggtgc cagggtgctc cgtgccgcct 15780
   ggttcccggt gctgtggctg ttcttcctgg tgccgccgcc cggctgggcc gtcgaccgca 15840
   tcaccgcacc gctcaaggag ttcgtctcct atgcggcaac gggcctgctt tcctgggtgg 15900
   attatccgat cctgcgccag ggcgtgacac tgttcgtcgg cccctatcag ctgctcgtcg 15960
   aagatgcctg ttcgggtctg cgctcgctgt ccagcctggt cgtcgtgacg ctgctctaca 16020
   tctacatcaa gaacaagccg tcctggcgct acgcggcgtt catcgcagcg ctggtgatcc 16080
   cggtggcagt ggtgaccaac gtcctgcgga tcatcatcct ggtactgatc acctatcatc 16140
   tgggcgacga ggcggcgcag agcttcctcc acgtctccac cggcatggtg atgttcgtgg 16200
   tcgccctgct ttgcatcttc gcgatcgact gggtggtcga gcaacttctt ctcctgcgtc 16260
   ggaggcatca tgttcaaccg gcgtgacctg ctgatcggcg caggctgctt cgccgccgct 16320
   ggcgcctcgc tcggcctgaa gccgcaccgg cggatggacc tgctgggcgg caccaagctc 16380
   gacacgctga tgcccaaggc attcggcgca tggaaggcag aggataccgg ttcgctgatc 16440
   gcgccggcgc gcgaaggcag cctggaggac aagctctaca accaggtggt cacccgcgcc 16500
   ttctcccgcg cggacggtgc ccaagtgatg ctgctgatcg cctatggcaa cgcccagacc 16560
   gatctactgc agctgcaccg gccggaaata tgctacccgt tcttcggctt caccgtggtg 16620
   gaaagccatg agcagaccat cccggtgacg ccgcaggtga cgatccccgg tcgcgcgctg 16680
   accgccacca acttcaaccg caccgagcag atcctctact ggacccgcgt cggcgaatat 16740
   ctgccgcaga acggcaatca gcagatgctc gcgcggctga agagccaggt ccagggctgg 16800
   atcgtcgacg gtgtgctggt gcgcatctcg acggtgacgc ccgaggcgga agatggcctg 16860
   agcgccaatc tcgatttcgc gcgcgagctg gtgaagacgc tcgacccgcg cgtgctgcgc 16920
   ccgctgctcg ggaacgggct cacacggcag ctcggtcacc aggtctgaac cggtgcgccg 16980
   cacgcggcgc ccccggcaac aaaaaaggag cggcgcgggc cgccgccgct ccctctcctt 17040
   ctcatgcggc gccctgccct caccgctcgt gcagcgcgtc actccccgtc tcgagcacgg 17100
   gccccaccag atagctgaac agggttcgct tgccggtgac gatgtccgcg ctcgcgagca 17160
   tccccggccg cagcggcacc tgtgcgccat gggccagcac atacccgcgc gccagcgcga 17220
   tccgcgcctt gtagaccggc ggctggttct ccttcatctg caccgcctcg gggctgatgc 17280
   ccgccaccgt gccgggaatc atgccgtagc gggtataggg aaaggcctgc agcttcacct 17340
   ttaccggcat gccgatgtgg acgaagccga tgtcgctgtt gtcgaccatc acctcggcct 17400
   cgagccgggc attgtcggga accaggctga ggagcggctt ggccccttcc accacgccgc 17460
   cttcggtgtg gacctgcagc tgcgagacgg taccgctcac cggcgcgcgc agttcgcgga 17520
   acgagctgcg cagattcgcc ttggcgacgt cctcgccgcg ggcacgcacc tcgtcctgcg 17580
   ccttgaccag atcctgcagc acctgcgccc gcgcctcctc gcgcgtcttg gccgacaggc 17640
   tggagacgct cagcgactgc tggccgagtt tggcgagcgt agcgcgcgcc gccgtcaggt 17700
   cctgccgctc ggcgatcagc tggcgacgca tctccacgac gcgcagcttc gagacatagc 17760
   ccttggcggc catcgtctcg ttcgcggcga tctgctgttc gagcagcggc agcgactgtt 17820
   cgagcttccg cacctgtgcc tgcgcctcgg ccgcggccga gacggcggca ccgcgatcgg 17880
   agcggccgcc ggccagcgcc gcctcgatct ggcccagccg ggcgcgggcg aggccgcgat 17940
   gcgtcgccac ttcgcccggg ctggcggcgg caggcgcgac gaagcggaag cccctgccgt 18000
   ccagcgcgtc gatgatcgcc tggttgcgtg cggcgtcgag ctgggcgctg agcagcgcca 18060
   ccttcgcctg tgccgcctcc gccgacgaca cggtcgggtc gagcgtgatc agcacctggc 18120
   ccttggcgac cttctgcccc tcgcccacca ggatgcggcg gacgatcccc gattcgggcg 18180
   actggacgat cttggtctcg ccgatcggcg cgatccgccc ctgcgtcggc gcgacgactt 18240
   cgaccttgcc gatcgccagc caggcggcgg tgatcgccag cccggccagc atcaccttgg 18300
   cggtaagccg cgcggtgggc gaaaccggcc gctcgatgat ctccagcgcg gcaggcagga 18360
   aggcggtgtc ataagcgtcg acgcgggcag gcagcacggt atcgcgcatg cgggcgagcg 18420
   ggccgccgcg gcgcatcgga acaacggcgt tcatgcggca atctccccat agccgccctg 18480
   gcggcggtgc aggtcggcat agcggccgcc caggcgcaac aattcgtcgt gtcggccgct 18540
   ctcgacgatg cggccctgtt cgagcgtgat gatccggtcg cagctgcgca ccgcgctcag 18600
   gcgatgcgcg atcaccacga gcgtgcggcc ggccgagatg gcgcgcaggt tgttctggat 18660
   cagctcctcg ctctcggcat cgagcgccga ggtcgcttcg tcgaacacca ggatgcgcgg 18720
   attgccgacg agcgcgcggg cgatggcgag ccgctggcgc tggccgccgg agagattgac 18780
   gccgcgctcg acgatctcgg tgtcatagcc gcgcggctgg cgcaggatga aatcatgcgc 18840
   gccggccagc gtcgccgccg cgacgacatt ctcgaacggc atggcggggt tggagagcgc 18900
   gatgttctcg cggatcgagc ggctgaacag cagattctcc tgcagcacga cgccgatctg 18960
   gcgacgcagc caggcgggat cgagctgcgc cacgtcgacc tcgtcgacca gcacgcggcc 19020
   gagattcggc aggttgagcc gctggagcag cttggccagc gtcgacttgc ccgagcccga 19080
   cgaaccgacg atgccgagcg aggtgcccgc cggaatgtcg agcgtgatgt cgctcagcac 19140
   cggcggctgg tcctcggcat agcggaagct gacattctcg aagcgaatcg caccgcgcag 19200
   caccggcagc gtcgccgccg aggccgggcg cggttccacc ggatggttga gcacgtcgcc 19260
   cagccgctcg accgagatgc gcacctgctg gaaatcctgc cacagctgcg ccatgcggat 19320
   caccggcccg gacacgcgct gggcgaacat gttgaacgcc accagcgcgc ctacgctcat 19380
   cgcgccgccg atcaccgcct tggcgccgaa gaacaggatc gccgcgaagc tcagcttcga 19440
   gatcagctcg atcgcctggc tgccggtgtt ggcggtattg atcagccgct gcgacgcggc 19500
   ggtatgggcg gcgagctggc gctcccagcg attctgccag tgcggctcga ccgcggtcgc 19560
   cttgatcgtg tggatgcccg agacgctctc gacgagcagc gcgttgctgg cggagctctt 19620
   ctcgaacttg tcctccaccc gcgcgcggag cggcccggcg acgctgaacg atacgatcgc 19680
   ataggcgatc agcgacacga gcacgatgcc cgagagcatc ggcgagtaga acagcatcgc 19740
   ggcgaggaac acgaaggtga acagcgggtc caccatcacc gtcagcgagg cgctggtaag 19800
   gaattcgcgg atcgtctcga gctggcggac gcgggtgacg gtgtcgccca cgcggcgctt 19860
   ctcgaaatag gcgagcggca gcgccagcag gtggtggaac agccgggcac ccagctcgac 19920
   gtcgatcttc tgcgtcgtct cggtgaacag gcgggtgcgg atccagccga gcgccacttc 19980
   ccacaccgaa accgccagga aggcgaaggc gagcacgctc agcgtgctca tgctgttgtg 20040
   gatcagcacc ttgtcgatca cgctctggaa caacagcggc gcggcgaggc cgagcaggtt 20100
   gagcgcgagg gtgatgccga gcacctcgag gaacagcgtg cgatagcgcc ggaactgcgc 20160
   ggtgaaccag gagaggccga accgcagcgg ccgtcccgcc accgcgcggg tggtgagcag 20220
   caccagcgcg ccggaccaga tcgcgtccag cgcgtcccgg tcgacctgtt ccggggcatg 20280
   gcccgggcgc tggatgatca cgccatgttc ggtcaggccg ccgatcacga accagccttc 20340
   gggcccgtcg gcgatcgcgg gcagcggctg gcgggcgagt ccgccgcgcg gcacctcgac 20400
   ggccttggcg cgcacgccct gctggcgctt ggccaggagg atcaggtcgt cggcgcttgc 20460
   cgcctcggca tggcccagcg cgtggcgcag ctgttcgggc gtgatggcga tgttgtgcgc 20520
   gccgagcagc agcgacaacg ccaccagtcc ggattcgcgc agctccgcct cgcgctccgc 20580
   cgccccatgg gccgcgagcg cgctctgcag ggtggcctgc atttcgtcgc gtgtcatttc 20640
   cggaactctg cctccatggc gatactgaga gcgccatgat gaagaaggct ggtaaagact 20700
   cacttaatcc tagcttttct ggtatttacc cgtagctgcc gacccgattt gggacaggcc 20760
   tggcttagca ggtccttaaa ctcgaccgac tataccgcga cgccgaggag ggggaggatt 20820
   ggcgccgcat cgcgcggcga aacgcgggtg cgtcgcaaca tttcgccgga gtcgatccgt 20880
   cgcgaatgct gcacccgcga acgcaatgac ggccgccacg caatccggct tgatcccggg 20940
   cggcggatcg cgataagccg cgccacggtc gccaaaactc gtcgaaataa ccgacaaaac 21000
   cacggcatat ggctggatat tgcagcgttt gccctgcgtt tccgtcgttc aaccgccctt 21060
   cgaatcaggc aggcccagcg tgaccatgat tgatcttcct cttggaacgg cacactttgg 21120
   tcgacacgga gacttccggt cgggcaattg tcccgttata gtgcaatgca acaggccgaa 21180
   tcggccgctg tcggcgtgca cattccgttg agggagcccg atgaggcaat gaacgctttc 21240
   gaagcacagc gcgcctttga ggagcaactt cgggcgcatt cccgggttac gccatctgcc 21300
   gctcccgtgt ggcgtcgctc gacgctgcgg atggtcctct ataccgagtt gctgctgctg 21360
   gacagtctct cgatcctggc cggattccac gtcgcggcgg gcacgcgcga cggcaactgg 21420
   ctgtcgctgg cgggcatcaa cgtcggcgtc ttcctgctgc cgatcgctct cggcaccgcg 21480
   ctcgcaagcg gcacctactc gctgaactgc ctgcgctacc cggtcagcgg cgtgaagagc 21540
   atcttctcgg cattcttctt ctcgatcttc gtcgtcctgc tcggcagcta cctgctgacg 21600
   gccgagctgc cgctgtcccg cgtgcagctg gcggagggcg cgatcctctc gctggtcctc 21660
   ctgatggtgg gccgcctgat gttccgccgc cacgtccgcg cggttaccgg cggcaggctg 21720
   ctcgacgaac tggtcatcat cgacggcgtc tcgctcgacg tcgcgggcaa tgcggtcgcg 21780
   ctcgacgcgc ggatcatcaa tctctcgccg aacccgcgcg atccgcaaat gctgcatcgc 21840
   ctgggcacca ccgtgatcgg gttcgaccgg gtgatcgtcg cctgcaccaa ggagcatcgc 21900
   gcggtctggg cgctgctgct caagggcatg aacatcaagg gcgagatcct cgtcccccag 21960
   ttcaatgcgc tgggcgcgat cggcgtggac gcctttgacg ggaaggatac gctggtcgtc 22020
   tcgcagggcc cgctcaacat gcccaaccgc gcgaagaagc gcgcgctcga tctcgcgatc 22080
   accgtaccgg ccgtgctcgc gctggcgccg ctgatgatcc tggtggcgat cctgatcaag 22140
   ctggagagcc cgggcccggt gttgttcgcg caggatcgcg tcggccgcgg caaccggctg 22200
   ttcaagatca tgaagttccg ctcgatgcgc gtaacgctgt gcgacgcgaa cggcaacgtc 22260
   tcggccagcc gcgacgacga tcgcatcacc aaggtcggcc gcttcatccg caagaccagc 22320
   atcgacgaac tgccgcagct gctgaacgtg ctgcgcggcg acatgagcgt cgtcggcccg 22380
   cggccgcatg cgctgggctc gcgcgccgcc gatcacctgt tctgggaaat cgacgagcgc 22440
   tactggcacc gccacacgct caagccgggc atgaccggtc tggcccaggt gcgcggtttc 22500
   cgcggggcga ccgatcgccg cgtcgatctg accaaccggc tccaggcaga catggaatat 22560
   atcgacggat gggatatctg gcgcgatatc acgatcctgt tcaagacgct gcgggtgatc 22620
   gtgcattcga acgcattctg atccgcgcac gacgctgggc cgcagcctcg atccgcaaat 22680
   ggattgacag cggcccggct tccgttttct cgtttgattt tcgttgcggc cggtccgcgc 22740
   catgggggat tactgaatga agggcatcat ccttgcgggg ggcagcggga cgcgcctgta 22800
   ccccgcaacg ctatcgatct cgaagcagct gcttcccgtc tatgacaagc cgatgatctt 22860
   ctatccgctg tcggtgctga tgctcaccgg catccgggac atcctgatta tctccacccc 22920
   gcgcgacctg ccgatgttcc aggcgctgct gggcgacggc tcggccttcg gcatcaacct 22980
   cagctatgcc gagcagccct cccccaacgg gctggccgaa gcgttcatca tcggcgcgga 23040
   tttcgtcggc aacgatccca gcgcgctgat cctgggcgac aacatctatc acggcgaaaa 23100
   gatgggcgag cgctgccagg cagccgcagc gcaggcagcg cagggcggtg caaacgtctt 23160
   cgcctatcat gtcgacgacc ccgagcgcta cggcgtggtc gcgttcgacc cggagacggg 23220
   cgtcgccacc agcgtcgagg aaaagccggc cgagcccaag tccaactggg cgatcaccgg 23280
   cctgtatttc tacgacaagg acgtggtcga catcgccaag tcgatccagc cctcggcgcg 23340
   cggcgaactc gagatcaccg acgtcaaccg cgtttacatg gagcgcggcg acctgcacat 23400
   cacgcgcctc ggccgcggct atgcctggct cgacaccggc acgcatgaca gcctgcacga 23460
   agccggctcg ttcgttcgca cgctcgagca tcggacgggc gtgaagatcg cctgcccgga 23520
   ggaaatcgcc ttcgaaagcg gctggctcgg cgccgaagac ctgctcaagc gcgccgccgg 23580
   cctcggcaag accggctatg ccgcctatct ccgcaaggtt gcgaccgcag catgacccag 23640
   gtccatcatc acgaactgtc cggcgtcatc gagttcacgc cgcccaaata tggcgaccac 23700
   cgcggcttct tctccgaagt gttcaagcag tcggtgctcg atgccgaagg cgtcgaggca 23760
   cgctgggtgc aggacaatca gagcttctcg gcggccccgg gcacgatccg cggcctgcat 23820
   ctccaggcgc cgcccttcgc ccaggccaag ctggtccgcg tgttgcgcgg cgcgatcttc 23880
   gacgtcgcgg tcgacatccg tcgcggctcg cccacctatg gcaaatgggt cggcgtcgag 23940
   ctctcggccg agaagtggaa ccagctgctg gtccccgccg gctatgcgca cggcttcatg 24000
   acgctcgttc cggattgcga gatcctctac aaggtcagcg ccaaatattc gaaggattcg 24060
   gagatggcga tccgttggga cgatcccgat ctcgccatcg cctggccgga catcggcgtc 24120
   gagccggtcc tctccgaaaa ggacgcggtc gccacgccct tcgccgaatt caacaccccc 24180
   ttcttctatc agggctgagc catgcagcag accttcctcg tcaccggcgg cgccggcttc 24240
   atcggctcgg cggtggtgcg ccacctcgtc cgccagggcg cgcgcgtcat caatctcgac 24300
   aagctcacct atgccggcaa cccggcctcg ctgactgcga tcgagaacgc gcccaactat 24360
   cgcttcgtcc atgccgacat cgccgacacc gcgacgatcc taccgctgct gcgcgaggag 24420
   caggtcgatg tggtgatgca cctcgccgcc gagagccatg tcgatcgctc gatcgacggc 24480
   cctggcgagt tcatcgagac caatgtcgtc ggcaccttca agctgctcca gtcggcgctg 24540
   caatattggc gcgagctgga gggcgagaaa cgcgacgcgt tccgcttcca ccacatctcc 24600
   accgacgaag tgttcggcga cctgccgttc gacagcggca tcttcaccga agagacgccc 24660
   tatgatccct cctcgcccta ttcggcgtcg aaggcggcga gcgaccatct ggtgcgcgcc 24720
   tggggccaca cctatggcct gccggtggtg ctgtcgaact gctcgaacaa ttacgggccg 24780
   ttccacttcc ccgagaagct gatcccgttg accatcctca acgcgctcga gggcaagccg 24840
   ctgccggtct acggcaaggg cgagaatatc cgcgactggc tgtatgtcga cgatcacgcc 24900
   aaggcgctgg cgaccatcgc caccaccggc aaggtcggcc agagctacaa tgtcggcggc 24960
   cgcaacgagc ggaccaacct gcaggtggtc gagacgatct gcgacctgct cgaccagcgc 25020
   attccgctgg ccgacggtcg caagcgccgc gaactgatca ccttcgtcac cgatcgcccc 25080
   ggccatgacc gccgctacgc gatcgacgcg accaagctcg agaccgagct gggctggaag 25140
   gctgaggaga atttcgacac cggcatcgcc gcgacgatcg actggtatct ggcgaacgag 25200
   tggtggtggg gcccgatccg ctccggcaaa tatgccggcg agcggctggg gcagaccgcc 25260
   tgatgcgtat cctcgtcacc gggcatgacg gccaggtcgc ccagtcgctg gccgagcagg 25320
   cggtgggcca cgagctggtc ttcaccacct accccgaatt cgatctctcc aagccggaga 25380
   cgatcgaggc cggtgtggcg cgggtgcacc cggacctgat cgtctccgcc gccgcctaca 25440
   cggcggtcga caaggcggaa agcgaacccg agctggcgat ggcgatcaac ggcgacggtc 25500
   ccggcgtgct ggcgcgcgcg ggcgcgaaga tcggcgcgcc gatcatccac ctgtcgaccg 25560
   attatgtgtt cgacggcagt ctcgaccgcc cttggcgcga ggacgatccc accggcccgc 25620
   tcggcgtcta tggcgcgacc aagctggccg gcgagcaggc ggtgcaggcc tcgggtgcca 25680
   ccaacgccgt gatccggctg gcctgggtct acagcccgtt cggcaacaat ttcgtcaaga 25740
   cgatgctccg cctcgccgag acgcgcgacg cgctgaacgt cgtggaggac cagtggggct 25800
   gccccagttc ggcgctggac atcgcgaccg cgatcctgac ggtggtcggg cactggcagc 25860
   aggacggcgc gacgagcggc ctctaccatt tcgccggcac cggcgagacc aactgggccg 25920
   acttcgcatc gacgatcttc gccgagagcg ccaagcgcgg tggcccctcg gccaccgtca 25980
   ccggcattcc cagctcgggc tatccgactc cggccacgcg cccggccaat tcgcggctgg 26040
   actgcacccg cttcgcggag accttcggct accgggcgcc tgcctggcag gattcgctga 26100
   acgtcgtact ggatcgcctg ctcggctgat ccgaaacggg gggcctcagc gccccccgcc 26160
   atgctcccgt tcgcgcgccg gcaatgcctc tagcaccgcg cgctttccct taggactcag 26220
   ctcgctccag ccggcgattt ccttgggcga ccgccagcac cccaggcaca gccggatc 26278
<210> 2
   <211> 1053
   <212> DNA
   <213> Sphingomonas sp. ATCC53159
<220>
   <221> CDS
   <222> (1)...(1053)
   <223> dpsS (partial)
<400> 2
   atc aac ggc gcc ttg ctc gga cgg cac aaa ttc gtc ctg gtc aat gtg      48
   Ile Asn Gly Ala Leu Leu Gly Arg His Lys Phe Val Leu Val Asn Val
    1               5                  10                  15
   tcc acg gtc gcc tct tcg ata ctg ttc cag ctg ttc ccg ctt gtc gtc      96
   Ser Thr Val Ala Ser Ser Ile Leu Phe Gln Leu Phe Pro Leu Val Val
               20                  25                  30
   gcc tgg atg atc ggc ccg gac ctg cga acg ctg ctg atc gcc gcg ctc      144
   Ala Trp Met Ile Gly Pro Asp Leu Arg Thr Leu Leu Ile Ala Ala Leu
           35                  40                  45
   gtt ggc cgg gcg gtt ccg atg atc ggc atg ctg ccc gcg ctg tat cga      192
   Val Gly Arg Ala Val Pro Met Ile Gly Met Leu Pro Ala Leu Tyr Arg
       50                  55                  60
   aac ctt ttg cgc ggc aac acg ccg cgt ttt cac gcc agc gag gcg cgc      240
   Asn Leu Leu Arg Gly Asn Thr Pro Arg Phe His Ala Ser Glu Ala Arg
    65                  70                  75                  80
   ttc ctg ata ggc tat ggc ggg tgg gcc tcg ctc acg acc gtg gta gcg      288
   Phe Leu Ile Gly Tyr Gly Gly Trp Ala Ser Leu Thr Thr Val Val Ala
                   85                  90                  95
   acc gtg ctc atg atg gcg gac cgc ttc ctg att ggc gca ctt ctt ggg      336
   Thr Val Leu Met Met Ala Asp Arg Phe Leu Ile Gly Ala Leu Leu Gly
               100                 105                 110
   ccc gtc gcc gtg acc atc tac acg gcc ccc ctg caa ctc gca cag cgc      384
   Pro Val Ala Val Thr Ile Tyr Thr Ala Pro Leu Gln Leu Ala Gln Arg
           115                 120                 125
   gta tcg ctg ctg ccc tcc gca ctg tcc gcc gcg ctg ttc ccg cgc ctg
   432
   Val Ser Leu Leu Pro Ser Ala Leu Ser Ala Ala Leu Phe Pro Arg Leu
       130                 135                 140
   ccc agc gcg acg ccg gcg gag cgc atg gcg ctt cag atc cgc tcg ctg
   480
   Pro Ser Ala Thr Pro Ala Glu Arg Met Ala Leu Gln Ile Arg Ser Leu
   145                 150                 155                 160
   tcg ctg atc atg ggc ggc ctt acc ggg atg atc ggc ggc gga cta ttg
   528
   Ser Leu Ile Met Gly Gly Leu Thr Gly Met Ile Gly Gly Gly Leu Leu
                   165                 170                 175
   ctg gcc gcg ccg ttt ctc gat ctc tgg atc ggc aag tcg ctc ggc cat
   576
   Leu Ala Ala Pro Phe Leu Asp Leu Trp Ile Gly Lys Ser Leu Gly His
               180                 185                 190
   gcg gga acg ccg gtc gcg ctc ttc ctg ttc ttc ggc gca tgg tgg aat
   624
   Ala Gly Thr Pro Val Ala Leu Phe Leu Phe Phe Gly Ala Trp Trp Asn
           195                 200                 205
   gcg ctg gcg atc att tcg ttc agc ggc ctg cag gcg agc gga cgg ccg
   672
   Ala Leu Ala Ile Ile Ser Phe Ser Gly Leu Gln Ala Ser Gly Arg Pro
       210                 215                 220
   aaa gcg agc gcg atc gtc cag ggg gca gag ctg cta ccc gtg ttg atc
   720
   Lys Ala Ser Ala Ile Val Gln Gly Ala Glu Leu Leu Pro Val Leu Ile
   225                 230                 235                 240
   gcg ctg tat gca ggg atc cga tgg ggc ggc gtg acc ggc gcc gca gcg
   768
   Ala Leu Tyr Ala Gly Ile Arg Trp Gly Gly Val Thr Gly Ala Ala Ala
                   245                 250                 255
   gtc ttt ctg gga cgc tcc gcc ctg gat ttc gtc ctg ttg acc tgg cag
   816
   Val Phe Leu Gly Arg Ser Ala Leu Asp Phe Val Leu Leu Thr Trp Gln
               260                 265                 270
   gca ggc ctg ctc cgc cag acg gtg aag caa gta tcc gta tgc ggc gcc
   864
   Ala Gly Leu Leu Arg Gln Thr Val Lys Gln Val Ser Val Cys Gly Ala
           275                 280                 285
   gtt ctc acc gtc gcg atg ctc gtg ggc gcg acc tat cgc tat tcg gtg
   912
   Val Leu Thr Val Ala Met Leu Val Gly Ala Thr Tyr Arg Tyr Ser Val
       290                 295                 300
   ccg ctc tgg tgc gta ctc agc gcc tgc tgc ctg gtc gcg ctg gca gcc
   960
   Pro Leu Trp Cys Val Leu Ser Ala Cys Cys Leu Val Ala Leu Ala Ala
   305                 310                 315                 320
   tgc tcc tgg tgg aca ttg gcg cgc cag gac aag gca ctg ctg att gga
   1008
   Cys Ser Trp Trp Thr Leu Ala Arg Gln Asp Lys Ala Leu Leu Ile Gly
                   325                 330                 335
   cga ttg agc cga att cta cca aag cag cgg caa ctc gac cta tag
   1053
   Arg Leu Ser Arg Ile Leu Pro Lys Gln Arg Gln Leu Asp Leu  *
               340                 345                 350
<210> 3
   <211> 350
   <212> PRT
   <213> Sphingomonas sp. ATCC53159
<220>
   <221> PEPTIDE
   <222> (0) ... (0)
   <223> homologous to gels
<400> 3
   Ile Asn Gly Ala Leu Leu Gly Arg His Lys Phe Val Leu Val Asn Val
    1               5                  10                  15
   Ser Thr Val Ala Ser Ser Ile Leu Phe Gln Leu Phe Pro Leu Val Val
               20                  25                  30
   Ala Trp Met Ile Gly Pro Asp Leu Arg Thr Leu Leu Ile Ala Ala Leu
           35                  40                  45
   Val Gly Arg Ala Val Pro Met Ile Gly Met Leu Pro Ala Leu Tyr Arg
       50                  55                  60
   Asn Leu Leu Arg Gly Asn Thr Pro Arg Phe His Ala Ser Glu Ala Arg
   65                  70                  75                  80
   Phe Leu Ile Gly Tyr Gly Gly Trp Ala Ser Leu Thr Thr Val Val Ala
                   85                  90                  95
   Thr Val Leu Met Met Ala Asp Arg Phe Leu Ile Gly Ala Leu Leu Gly
               100                 105                 110
   Pro Val Ala Val Thr Ile Tyr Thr Ala Pro Leu Gln Leu Ala Gln Arg
           115                 120                 125
   Val Ser Leu Leu Pro Ser Ala Leu Ser Ala Ala Leu Phe Pro Arg Leu
       130                 135                 140
   Pro Ser Ala Thr Pro Ala Glu Arg Met Ala Leu Gln Ile Arg Ser Leu
   145                 150                 155                 160
   Ser Leu Ile Met Gly Gly Leu Thr Gly Met Ile Gly Gly Gly Leu Leu
                   165                 170                 175
   Leu Ala Ala Pro Phe Leu Asp Leu Trp Ile Gly Lys Ser Leu Gly His
               180                 185                 190
   Ala Gly Thr Pro Val Ala Leu Phe Leu Phe Phe Gly Ala Trp Trp Asn
           195                 200                 205
   Ala Leu Ala Ile Ile Ser Phe Ser Gly Leu Gln Ala Ser Gly Arg Pro
       210                 215                 220
   Lys Ala Ser Ala Ile Val Gln Gly Ala Glu Leu Leu Pro Val Leu Ile
   225                 230                 235                 240
   Ala Leu Tyr Ala Gly Ile Arg Trp Gly Gly Val Thr Gly Ala Ala Ala
                   245                 250                 255
   Val Phe Leu Gly Arg Ser Ala Leu Asp Phe Val Leu Leu Thr Trp Gln
               260                 265                 270
   Ala Gly Leu Leu Arg Gln Thr Val Lys Gln Val Ser Val Cys Gly Ala
           275                 280                 285
   Val Leu Thr Val Ala Met Leu Val Gly Ala Thr Tyr Arg Tyr Ser Val
       290                 295                 300
   Pro Leu Trp Cys Val Leu Ser Ala Cys Cys Leu Val Ala Leu Ala Ala
   305                 310                 315                 320
   Cys Ser Trp Trp Thr Leu Ala Arg Gln Asp Lys Ala Leu Leu Ile Gly
                   325                 330                 335
   Arg Leu Ser Arg Ile Leu Pro Lys Gln Arg Gln Leu Asp Leu
               340                 345                 350
<210> 4
   <211> 1626
   <212> DNA
   <213> Sphingomonas sp. ATCC53159
<220>
   <221> CDS
   <222> (1) ... (1626)
   <223> dpsG
<400> 4
   atg acg act acc tcg gcg ttt cgt cgc ccg gcc ttc cac gga gcg atg
   48
   Met Thr Thr Thr Ser Ala Phe Arg Arg Pro Ala Phe His Gly Ala Met
    1               5                  10                  15
   cag cgg ctt cgc agg ttg cga ctg gtt cgg ttt ctg aca aag cca gcg
   96
   Gln Arg Leu Arg Arg Leu Arg Leu Val Arg Phe Leu Thr Lys Pro Ala
               20                  25                  30
   atc ccg gta ctg ccc gtc tac aaa gcc gag cga tca ggc gtg acg atc
   144
   Ile Pro Val Leu Pro Val Tyr Lys Ala Glu Arg Ser Gly Val Thr Ile
           35                  40                  45
   gcg gcg cgg cgt acc gtt ctg ctg gtc agc gtg atg ttt ctt gcc gca
   192
   Ala Ala Arg Arg Thr Val Leu Leu Val Ser Val Met Phe Leu Ala Ala
       50                  55                  60
   gtc tac ggc ctg ctc gcc gca gtt ctg ccg ctc cag atg ctg gcg atc
   240
   Val Tyr Gly Leu Leu Ala Ala Val Leu Pro Leu Gln Met Leu Ala Ile
    65                  70                  75                  80
   ccg gcc gtg ccc ctc gtt ctg atg gcg ctc gta gtg atc tgg gcg cta
   288
   Pro Ala Val Pro Leu Val Leu Met Ala Leu Val Val Ile Trp Ala Leu
                   85                  90                  95
   ccc gag gcg cgg cag gcg cct act cgc ctg ctg gca aaa cta tac ctc
   336
   Pro Glu Ala Arg Gln Ala Pro Thr Arg Leu Leu Ala Lys Leu Tyr Leu
               100                 105                 110
   gcc tat atg gtg gcg gcg ctc gtg tgg ccg aac tat ctt gcg ctc agc
   384
   Ala Tyr Met Val Ala Ala Leu Val Trp Pro Asn Tyr Leu Ala Leu Ser
           115                 120                 125
   gtt ggt ggt ttg cct tgg atc tcg atc agg cgg atg atc ggt tcg atc
   432
   Val Gly Gly Leu Pro Trp Ile Ser Ile Arg Arg Met Ile Gly Ser Ile
       130                 135                 140
   gca ttg ctc acg ctc atg atc tcg ctt tcg gtc tcg aaa aag ttt cga
   480
   Ala Leu Leu Thr Leu Met Ile Ser Leu Ser Val Ser Lys Lys Phe Arg
   145                 150                 155                 160
   tcc gag atg gcg gcg att atg cgg gcg gcg ccg atc cca tcg cga cta
   528
   Ser Glu Met Ala Ala Ile Met Arg Ala Ala Pro Ile Pro Ser Arg Leu
                   165                 170                 175
   ttg ctt gcc ttc att atg gtc cag atc gtc gcc tcc att gcg acc cca
   576
   Leu Leu Ala Phe Ile Met Val Gln Ile Val Ala Ser Ile Ala Thr Pro
               180                 185                 190
   gcg gcc tcg caa aca att ccg cgg ttg att ggc atc gtt ctg acg gtg
   624
   Ala Ala Ser Gln Thr Ile Pro Arg Leu Ile Gly Ile Val Leu Thr Val
           195                 200                 205
   acg ccg atg gca ttc atc tcg cta tgg ctg atc ggg acc gac acg cgc
   672
   Thr Pro Met Ala Phe Ile Ser Leu Trp Leu Ile Gly Thr Asp Thr Arg
       210                 215                 220
   acc ccg gag tgg tgg gtt acg cgc ttg ttt tgg tgc gtg ggc gtg ctg
   720
   Thr Pro Glu Trp Trp Val Thr Arg Leu Phe Trp Cys Val Gly Val Leu
   225                 230                 235                 240
   atg gcg att ggc gtg ctg gag ttc cgg gtg aag cac gtc ctc tgg gcc
   768
   Met Ala Ile Gly Val Leu Glu Phe Arg Val Lys His Val Leu Trp Ala
                   245                 250                 255
   tac tcg att ccg agc ttc ctg cgc gtt gat gag cag ttt ctt acc gta
   816
   Tyr Ser Ile Pro Ser Phe Leu Arg Val Asp Glu Gln Phe Leu Thr Val
               260                 265                 270
   gtg ctt acg ccc ggt ttc cgg ggt act tac cgc gtt ttg acg act ttc
   864
   Val Leu Thr Pro Gly Phe Arg Gly Thr Tyr Arg Val Leu Thr Thr Phe
           275                 280                 285
   agc agt ccg ctt gtc tgg gga gag ttg acg gct ttg acg att cct ttc
   912
   Ser Ser Pro Leu Val Trp Gly Glu Leu Thr Ala Leu Thr Ile Pro Phe
       290                 295                 300
   gtg ctg cac cgt ata gcg aat tcc cgt ggc gta ggg aga ttg gca ttt
   960
   Val Leu His Arg Ile Ala Asn Ser Arg Gly Val Gly Arg Leu Ala Phe
   305                 310                 315                 320
   tgg att ttc ttc gat ttt ctt gta gtt gcg tcc gga ttt ctt agc ggt
   1008
   Trp Ile Phe Phe Asp Phe Leu Val Val Ala Ser Gly Phe Leu Ser Gly
                   325                 330                 335
   tct cga ctg gca atg gtg ggg gga ttg gtg gcg cat acc gtg tat ttg
   1056
   Ser Arg Leu Ala Met Val Gly Gly Leu Val Ala His Thr Val Tyr Leu
               340                 345                 350
   ctt att tgg gca att cgg cgg tgg cgg acc aca aag ggc ggg ctt gtc
   1104
   Leu Ile Trp Ala Ile Arg Arg Trp Arg Thr Thr Lys Gly Gly Leu Val
           355                 360                 365
   ggc att tcg ctg acg ttg acg tat ccc gcc ttg atg gtg gcg ctg tcg
   1152
   Gly Ile Ser Leu Thr Leu Thr Tyr Pro Ala Leu Met Val Ala Leu Ser
       370                 375                 380
   ctg gcg gtc atg ttc gtg cct gcc gtc cac aac cgt gtt ctc ggt gga
   1200
   Leu Ala Val Met Phe Val Pro Ala Val His Asn Arg Val Leu Gly Gly
   385                 390                 395                 400
   ggc gct tct caa ctt agc aat caa ggc agg cag gag cag ttt cgc ttg
   1248
   Gly Ala Ser Gln Leu Ser Asn Gln Gly Arg Gln Glu Gln Phe Arg Leu
                   405                 410                 415
   ggc gtg cct gcc atc gct cgc cgc cct ttc ttc ggc tat ggc ccg gga
   1296
   Gly Val Pro Ala Ile Ala Arg Arg Pro Phe Phe Gly Tyr Gly Pro Gly
               420                 425                 430
   gag ggt gct ggc gct gtc ggg tgg cgt aat cag caa ggc ttc cta tcg
   1344
   Glu Gly Ala Gly Ala Val Gly Trp Arg Asn Gln Gln Gly Phe Leu Ser
           435                 440                 445
   atc gac agt ggt ttc ctg tcc gta gcg gct gat tac ggc ttg ctg ggt
   1392
   Ile Asp Ser Gly Phe Leu Ser Val Ala Ala Asp Tyr Gly Leu Leu Gly
       450                 455                 460
   ttt gtt tcg cta tac ggc acg atg att acg tta atg att ttg ctc gcg
   1440
   Phe Val Ser Leu Tyr Gly Thr Met Ile Thr Leu Met Ile Leu Leu Ala
   465                 470                 475                 480
   ttt cgt ggt ctt aag atg agc gga gat ggc tac ccg ctt gag ctc gcc
   1488
   Phe Arg Gly Leu Lys Met Ser Gly Asp Gly Tyr Pro Leu Glu Leu Ala
                   485                 490                 495
   gta gca act ttc ctg gct gtt ctt tta aat acg cgg tca gtc ctg tcg
   1536
   Val Ala Thr Phe Leu Ala Val Leu Leu Asn Thr Arg Ser Val Leu Ser
               500                 505                 510
   caa ggt gac aat gac ccg ttc atc ttt atg acg ctg ggg cta ggc ata
   1584
   Gln Gly Asp Asn Asp Pro Phe Ile Phe Met Thr Leu Gly Leu Gly Ile
           515                 520                 525
   gcg ctt cta tat cgc tct cgg cct gtg tct ttg tcg gtg tag
   1626
   Ala Leu Leu Tyr Arg Ser Arg Pro Val Ser Leu Ser Val  *
       530                 535                 540
<210> 5
   <211> 541
   <212> PRT
   <213> Sphingomonas sp. ATCC53159
<220>
   <221> PEPTIDE
   <222> (0)...(0)
   <223> putative polymerase
<400> 5
   Met Thr Thr Thr Ser Ala Phe Arg Arg Pro Ala Phe His Gly Ala Met
    1               5                  10                  15
   Gln Arg Leu Arg Arg Leu Arg Leu Val Arg Phe Leu Thr Lys Pro Ala
               20                  25                  30
   Ile Pro Val Leu Pro Val Tyr Lys Ala Glu Arg Ser Gly Val Thr Ile
           35                  40                  45
   Ala Ala Arg Arg Thr Val Leu Leu Val Ser Val Met Phe Leu Ala Ala
       50                  55                  60
   Val Tyr Gly Leu Leu Ala Ala Val Leu Pro Leu Gln Met Leu Ala Ile
   65                  70                  75                  80
   Pro Ala Val Pro Leu Val Leu Met Ala Leu Val Val Ile Trp Ala Leu
                   85                  90                  95
   Pro Glu Ala Arg Gln Ala Pro Thr Arg Leu Leu Ala Lys Leu Tyr Leu
               100                 105                 110
   Ala Tyr Met Val Ala Ala Leu Val Trp Pro Asn Tyr Leu Ala Leu Ser
           115                 120                 125
   Val Gly Gly Leu Pro Trp Ile Ser Ile Arg Arg Met Ile Gly Ser Ile
       130                 135                 140
   Ala Leu Leu Thr Leu Met Ile Ser Leu Ser Val Ser Lys Lys Phe Arg
   145                 150                 155                 160
   Ser Glu Met Ala Ala Ile Met Arg Ala Ala Pro Ile Pro Ser Arg Leu
                   165                 170                 175
   Leu Leu Ala Phe Ile Met Val Gln Ile Val Ala Ser Ile Ala Thr Pro
               180                 185                 190
   Ala Ala Ser Gln Thr Ile Pro Arg Leu Ile Gly Ile Val Leu Thr Val
           195                 200                 205
   Thr Pro Met Ala Phe Ile Ser Leu Trp Leu Ile Gly Thr Asp Thr Arg
       210                 215                 220
   Thr Pro Glu Trp Trp Val Thr Arg Leu Phe Trp Cys Val Gly Val Leu
   225                 230                 235                 240
   Met Ala Ile Gly Val Leu Glu Phe Arg Val Lys His Val Leu Trp Ala
                   245                 250                 255
   Tyr Ser Ile Pro Ser Phe Leu Arg Val Asp Glu Gln Phe Leu Thr Val
               260                 265                 270
   Val Leu Thr Pro Gly Phe Arg Gly Thr Tyr Arg Val Leu Thr Thr Phe
           275                 280                 285
   Ser Ser Pro Leu Val Trp Gly Glu Leu Thr Ala Leu Thr Ile Pro Phe
       290                 295                 300
   Val Leu His Arg Ile Ala Asn Ser Arg Gly Val Gly Arg Leu Ala Phe
   305                 310                 315                 320
   Trp Ile Phe Phe Asp Phe Leu Val Val Ala Ser Gly Phe Leu Ser Gly
                   325                 330                 335
   Ser Arg Leu Ala Met Val Gly Gly Leu Val Ala His Thr Val Tyr Leu
               340                 345                 350
   Leu Ile Trp Ala Ile Arg Arg Trp Arg Thr Thr Lys Gly Gly Leu Val
           355                 360                 365
   Gly Ile Ser Leu Thr Leu Thr Tyr Pro Ala Leu Met Val Ala Leu Ser
       370                 375                 380
   Leu Ala Val Met Phe Val Pro Ala Val His Asn Arg Val Leu Gly Gly
   385                 390                 395                 400
   Gly Ala Ser Gln Leu Ser Asn Gln Gly Arg Gln Glu Gln Phe Arg Leu
                   405                 410                 415
   Gly Val Pro Ala Ile Ala Arg Arg Pro Phe Phe Gly Tyr Gly Pro Gly
               420                 425                 430
   Glu Gly Ala Gly Ala Val Gly Trp Arg Asn Gln Gln Gly Phe Leu Ser
           435                 440                 445
   Ile Asp Ser Gly Phe Leu Ser Val Ala Ala Asp Tyr Gly Leu Leu Gly
       450                 455                 460
   Phe Val Ser Leu Tyr Gly Thr Met Ile Thr Leu Met Ile Leu Leu Ala
   465                 470                 475                 480
   Phe Arg Gly Leu Lys Met Ser Gly Asp Gly Tyr Pro Leu Glu Leu Ala
                   485                 490                 495
   Val Ala Thr Phe Leu Ala Val Leu Leu Asn Thr Arg Ser Val Leu Ser
               500                 505                 510
   Gln Gly Asp Asn Asp Pro Phe Ile Phe Met Thr Leu Gly Leu Gly Ile
           515                 520                 525
   Ala Leu Leu Tyr Arg Ser Arg Pro Val Ser Leu Ser Val
       530                 535                 540
<210> 6
   <211> 1998
   <212> DNA
   <213> Sphingomonas sp. ATCC53159
<220>
   <221> CDS
   <222> (1)...(1998)
   <223> dpsR
<400> 6
   atg ccg gat atc att gtc aag aat cag acg gag ttg aat gct gca atc
   48
   Met Pro Asp Ile Ile Val Lys Asn Gln Thr Glu Leu Asn Ala Ala Ile
    1               5                  10                  15
   gcg gcg gcg aag ggt ggc gaa acc atc aag ctt gcc gcc ggg gtc tac
   96
   Ala Ala Ala Lys Gly Gly Glu Thr Ile Lys Leu Ala Ala Gly Val Tyr
               20                  25                  30
   aca gat ctc act gta atg acc aag acg ttt acc agc atg gtg aca att
   144
   Thr Asp Leu Thr Val Met Thr Lys Thr Phe Thr Ser Met Val Thr Ile
           35                  40                  45
   gag tcg ctc gac tcg tcg aac ccg gtc aat atc caa aag ctg gtg atc
   192
   Glu Ser Leu Asp Ser Ser Asn Pro Val Asn Ile Gln Lys Leu Val Ile
       50                  55                  60
   ggg aac agt agc aac gtt acc gtc aaa aac atg gtc gct gcg acc gat
   240
   Gly Asn Ser Ser Asn Val Thr Val Lys Asn Met Val Ala Ala Thr Asp
    65                  70                  75                  80
   tac aag ccc gcc gat gac tgg aat cga ctg aat acg atc cag ggt tcg
   288
   Tyr Lys Pro Ala Asp Asp Trp Asn Arg Leu Asn Thr Ile Gln Gly Ser
                   85                  90                  95
   gcc aac atc gtt ttg gac ggc gtg cgg ttc agc ggc ggc act ggt gac
   336
   Ala Asn Ile Val Leu Asp Gly Val Arg Phe Ser Gly Gly Thr Gly Asp
               100                 105                 110
   cct tcg ctc tcg aag ggg gcg ggc ttg ttc gtg cgc aac agc acg tcg
   384
   Pro Ser Leu Ser Lys Gly Ala Gly Leu Phe Val Arg Asn Ser Thr Ser
           115                 120                 125
   gtg acg atg cag aat tcg tct atc gac cac ttc ggt ctg ggc ctt gag
   432
   Val Thr Met Gln Asn Ser Ser Ile Asp His Phe Gly Leu Gly Leu Glu
       130                 135                 140
   gcc tac aac gtc gat ggc atg gtg gtc cag aac agc agc ttc cac gac
   480
   Ala Tyr Asn Val Asp Gly Met Val Val Gln Asn Ser Ser Phe His Asp
   145                 150                 155                 160
   aac cgg cgc gat cat acg aac ttc act gag atg aac aat ctt gtc atc
   528
   Asn Arg Arg Asp His Thr Asn Phe Thr Glu Met Asn Asn Leu Val Ile
   165 170 175
   gac gga aat tcg ttc acg aac ctg ttt ccc gtg ggc acc gaa cat ccc
   576
   Asp Gly Asn Ser Phe Thr Asn Leu Phe Pro Val Gly Thr Glu His Pro
   180 185 190
   gac gcc att cag ttc ttc acg gcg ggc aag gtc aag ggc aat acc aac
   624
   Asp Ala Ile Gln Phe Phe Thr Ala Gly Lys Val Lys Gly Asn Thr Asn
   195 200 205
   atc acc atc tcc aat aac gtc atc atg cag ggc gcg ggc tct ggc gcg
   672
   Ile Thr Ile Ser Asn Asn Val Ile Met Gln Gly Ala Gly Ser Gly Ala
   210 215 220
   caa ggg att ttc atg aat gac gag gcc ggt aat ctt ccc tat gtc aat
   720
   Gln Gly Ile Phe Met Asn Asp Glu Ala Gly Asn Leu Pro Tyr Val Asn
   225 230 235 240
   gta aac atc aaa aac aat ctt atc tat ctg aat ggt tat tac cac ggt
   768
   Val Asn Ile Lys Asn Asn Leu Ile Tyr Leu Asn Gly Tyr Tyr His Gly
   245 250 255
   atc aac gtt gtt aac ggc gtt aat gtc aat atc gaa tcc aat agc gtg
   816
   Ile Asn Val Val Asn Gly Val Asn Val Asn Ile Glu Ser Asn Ser Val
   260 265 270
   ata tcg caa gtg gat ggc aca tca ttt tgg att cgc ctc gac aaa acc
   864
   Ile Ser Gln Val Asp Gly Thr Ser Phe Trp Ile Arg Leu Asp Lys Thr
   275 280 285
   aat ggc gcg acg atc aag aac aat gtt gcg gac ctg atc acc gtc aca
   912
   Asn Gly Ala Thr Ile Lys Asn Asn Val Ala Asp Leu Ile Thr Val Thr
   290 295 300
   agc tcc tcg agc aat atc gtg cag aca ggc aat cgt acg ctg acg agt
   960
   Ser Ser Ser Ser Asn Ile Val Gln Thr Gly Asn Arg Thr Leu Thr Ser
   305 310 315 320
   gac tcg gca acg atc cgc aag atc tat ggc ctc aac gat ggg gct acg
   1008
   Asp Ser Ala Thr Ile Arg Lys Ile Tyr Gly Leu Asn Asp Gly Ala Thr
   325 330 335
   gcg cgg ctc agc gat ttg atc gtt ccc ggc gtc ggg tac cag ccg ccc
   1056
   Ala Arg Leu Ser Asp Leu Ile Val Pro Gly Val Gly Tyr Gln Pro Pro
   340 345 350
   gtg tcg agc gct gct gcc gct cag gtg act acc gaa ctg tcg act gcg
   1104
   Val Ser Ser Ala Ala Ala Ala Gln Val Thr Thr Glu Leu Ser Thr Ala
           355                 360                 365
   aag gcg gca aat ccg tcg ctg ctg ctc gat ctg tcg ttc agc aac agc
   1152
   Lys Ala Ala Asn Pro Ser Leu Leu Leu Asp Leu Ser Phe Ser Asn Ser
       370                 375                 380
   ggc gtc gtg gac ctt tcg cac tgg aat acc ggc cag acg aca aag gcg
   1200
   Gly Val Val Asp Leu Ser His Trp Asn Thr Gly Gln Thr Thr Lys Ala
   385                 390                 395                 400
   gtg gac gtg tcg gcg gtc gtg ggc agc gcc ttc cac gtc tcg acg ggc
   1248
   Val Asp Val Ser Ala Val Val Gly Ser Ala Phe His Val Ser Thr Gly
                   405                 410                 415
   acg ggg gtg gaa cta aac cgg agc tat tcg cgg cag att tac gca ttg
   1296
   Thr Gly Val Glu Leu Asn Arg Ser Tyr Ser Arg Gln Ile Tyr Ala Leu
               420                 425                 430
   tcg gcg ttc acg ctc agc ttc gac ctc aag cgg gac tcg gct acg gcc
   1344
   Ser Ala Phe Thr Leu Ser Phe Asp Leu Lys Arg Asp Ser Ala Thr Ala
           435                 440                 445
   acg gcc ggg caa att ctt ggc atc ttc cag agc tgg tcg gtt tcg ctg
   1392
   Thr Ala Gly Gln Ile Leu Gly Ile Phe Gln Ser Trp Ser Val Ser Leu
       450                 455                 460
   cag gcc aat ggg gaa ctg agc ttc acc atg cgc aac gcc gcg ggc gtc
   1440
   Gln Ala Asn Gly Glu Leu Ser Phe Thr Met Arg Asn Ala Ala Gly Val
   465                 470                 475                 480
   agc cag aca atg gtg acg agc ggc gcc aag ctg ctt gat gct gcc aca
   1488
   Ser Gln Thr Met Val Thr Ser Gly Ala Lys Leu Leu Asp Ala Ala Thr
                   485                 490                 495
   cac aag atc gcc ctg acc tac gac agc acg cgg aaa acc gcg att ctg
   1536
   His Lys Ile Ala Leu Thr Tyr Asp Ser Thr Arg Lys Thr Ala Ile Leu
               500                 505                 510
   tac gta gac ggc atg caa cgc ggc aca gcg acg atg acc ggc acg acc
   1584
   Tyr Val Asp Gly Met Gln Arg Gly Thr Ala Thr Met Thr Gly Thr Thr
           515                 520                 525
   cgg ccc gcc gaa tcc tgg ggg ctg tat gtc ggc agc ccg ttc tcg acc
   1632
   Arg Pro Ala Glu Ser Trp Gly Leu Tyr Val Gly Ser Pro Phe Ser Thr
       530                 535                 540
   gca ttc agc gga acg gtc ggc gac atc gag atc cgc gat ggc gcg atc
   1680
   Ala Phe Ser Gly Thr Val Gly Asp Ile Glu Ile Arg Asp Gly Ala Ile
   545                 550                 555                 560
   agc gcc gcc cag gtg cag gcg ctg gtg acc gcg tcg agc gcc agc gcg
   1728
   Ser Ala Ala Gln Val Gln Ala Leu Val Thr Ala Ser Ser Ala Ser Ala
                   565                 570                 575
   gcg gcg acg gtc aag gac agc ctc gtc acc ggc gcg gcc gcg cag gcc
   1776
   Ala Ala Thr Val Lys Asp Ser Leu Val Thr Gly Ala Ala Ala Gln Ala
               580                 585                 590
   gct gcg ctg ctg gcg ggt gcc ggc gcc gct agc acg gca acg ccg ctt
   1824
   Ala Ala Leu Leu Ala Gly Ala Gly Ala Ala Ser Thr Ala Thr Pro Leu
           595                 600                 605
   gcg acg gtg gcc acg gtg ggc agt acg ctg tct ata ggt act gcc gcg
   1872
   Ala Thr Val Ala Thr Val Gly Ser Thr Leu Ser Ile Gly Thr Ala Ala
       610                 615                 620
   tcc tcg cag atc gcg ctc gtc agc aag atc ggt gtc gac atg atg acc
   1920
   Ser Ser Gln Ile Ala Leu Val Ser Lys Ile Gly Val Asp Met Met Thr
   625                 630                 635                 640
   gcg ggg gcg atg ggc gca atc cgc agc gcg gcg aca ctg agc gct acg
   1968
   Ala Gly Ala Met Gly Ala Ile Arg Ser Ala Ala Thr Leu Ser Ala Thr
                   645                 650                 655
   gcg gat cag tac aac ctg tac cgc gcc tga
   1998
   Ala Asp Gln Tyr Asn Leu Tyr Arg Ala  *
               660                 665
<210> 7
   <211> 665
   <212> PRT
   <213> Sphingomonas sp. ATCC53159
<220>
   <221> PEPTIDE
   <222> (0)...(0)
   <223> putative lyase
<400> 7
   Met Pro Asp Ile Ile Val Lys Asn Gln Thr Glu Leu Asn Ala Ala Ile
    1               5                  10                  15
   Ala Ala Ala Lys Gly Gly Glu Thr Ile Lys Leu Ala Ala Gly Val Tyr
               20                  25                  30
   Thr Asp Leu Thr Val Met Thr Lys Thr Phe Thr Ser Met Val Thr Ile
           35                  40                  45
   Glu Ser Leu Asp Ser Ser Asn Pro Val Asn Ile Gln Lys Leu Val Ile
       50                  55                  60
   Gly Asn Ser Ser Asn Val Thr Val Lys Asn Met Val Ala Ala Thr Asp
   65                  70                  75                  80
   Tyr Lys Pro Ala Asp Asp Trp Asn Arg Leu Asn Thr Ile Gln Gly Ser
                   85                  90                  95
   Ala Asn Ile Val Leu Asp Gly Val Arg Phe Ser Gly Gly Thr Gly Asp
               100                 105                 110
   Pro Ser Leu Ser Lys Gly Ala Gly Leu Phe Val Arg Asn Ser Thr Ser
           115                 120                 125
   Val Thr Met Gln Asn Ser Ser Ile Asp His Phe Gly Leu Gly Leu Glu
       130                 135                 140
   Ala Tyr Asn Val Asp Gly Met Val Val Gln Asn Ser Ser Phe His Asp
   145                 150                 155                 160
   Asn Arg Arg Asp His Thr Asn Phe Thr Glu Met Asn Asn Leu Val Ile
                   165                 170                 175
   Asp Gly Asn Ser Phe Thr Asn Leu Phe Pro Val Gly Thr Glu His Pro
               180                 185                 190
   Asp Ala Ile Gln Phe Phe Thr Ala Gly Lys Val Lys Gly Asn Thr Asn
           195                 200                 205
   Ile Thr Ile Ser Asn Asn Val Ile Met Gln Gly Ala Gly Ser Gly Ala
       210                 215                 220
   Gln Gly Ile Phe Met Asn Asp Glu Ala Gly Asn Leu Pro Tyr Val Asn
   225                 230                 235                 240
   Val Asn Ile Lys Asn Asn Leu Ile Tyr Leu Asn Gly Tyr Tyr His Gly
                   245                 250                 255
   Ile Asn Val Val Asn Gly Val Asn Val Asn Ile Glu Ser Asn Ser Val
               260                 265                 270
   Ile Ser Gln Val Asp Gly Thr Ser Phe Trp Ile Arg Leu Asp Lys Thr
           275                 280                 285
   Asn Gly Ala Thr Ile Lys Asn Asn Val Ala Asp Leu Ile Thr Val Thr
       290                 295                 300
   Ser Ser Ser Ser Asn Ile Val Gln Thr Gly Asn Arg Thr Leu Thr Ser
   305                 310                 315                 320
   Asp Ser Ala Thr Ile Arg Lys Ile Tyr Gly Leu Asn Asp Gly Ala Thr
                   325                 330                 335
   Ala Arg Leu Ser Asp Leu Ile Val Pro Gly Val Gly Tyr Gln Pro Pro
               340                 345                 350
   Val Ser Ser Ala Ala Ala Ala Gln Val Thr Thr Glu Leu Ser Thr Ala
           355                 360                 365
   Lys Ala Ala Asn Pro Ser Leu Leu Leu Asp Leu Ser Phe Ser Asn Ser
       370                 375                 380
   Gly Val Val Asp Leu Ser His Trp Asn Thr Gly Gln Thr Thr Lys Ala
   385                 390                 395                 400
   Val Asp Val Ser Ala Val Val Gly Ser Ala Phe His Val Ser Thr Gly
                   405                 410                 415
   Thr Gly Val Glu Leu Asn Arg Ser Tyr Ser Arg Gln Ile Tyr Ala Leu
               420                 425                 430
   Ser Ala Phe Thr Leu Ser Phe Asp Leu Lys Arg Asp Ser Ala Thr Ala
           435                 440                 445
   Thr Ala Gly Gln Ile Leu Gly Ile Phe Gln Ser Trp Ser Val Ser Leu
       450                 455                 460
   Gln Ala Asn Gly Glu Leu Ser Phe Thr Met Arg Asn Ala Ala Gly Val
   465                 470                 475                 480
   Ser Gln Thr Met Val Thr Ser Gly Ala Lys Leu Leu Asp Ala Ala Thr
                   485                 490                 495
   His Lys Ile Ala Leu Thr Tyr Asp Ser Thr Arg Lys Thr Ala Ile Leu
               500                 505                 510
   Tyr Val Asp Gly Met Gln Arg Gly Thr Ala Thr Met Thr Gly Thr Thr
           515                 520                 525
   Arg Pro Ala Glu Ser Trp Gly Leu Tyr Val Gly Ser Pro Phe Ser Thr
       530                 535                 540
   Ala Phe Ser Gly Thr Val Gly Asp Ile Glu Ile Arg Asp Gly Ala Ile
   545                 550                 555                 560
   Ser Ala Ala Gln Val Gln Ala Leu Val Thr Ala Ser Ser Ala Ser Ala
                   565                 570                 575
   Ala Ala Thr Val Lys Asp Ser Leu Val Thr Gly Ala Ala Ala Gln Ala
               580                 585                 590
   Ala Ala Leu Leu Ala Gly Ala Gly Ala Ala Ser Thr Ala Thr Pro Leu
           595                 600                 605
   Ala Thr Val Ala Thr Val Gly Ser Thr Leu Ser Ile Gly Thr Ala Ala
       610                 615                 620
   Ser Ser Gln Ile Ala Leu Val Ser Lys Ile Gly Val Asp Met Met Thr
   625                 630                 635                 640
   Ala Gly Ala Met Gly Ala Ile Arg Ser Ala Ala Thr Leu Ser Ala Thr
                   645                 650                 655
   Ala Asp Gln Tyr Asn Leu Tyr Arg Ala
               660                 665
<210> 8
   <211> 939
   <212> DNA
   <213> Sphingomonas sp. ATCC53159
<220>
   <221> CDS
   <222> (1)...(939)
   <223> dpsQ
<400> 8
   atg cag atg ctc cca acg ccc gat gtc agc ata ctc gtg gtc gct ttc
   48
   Met Gln Met Leu Pro Thr Pro Asp Val Ser Ile Leu Val Val Ala Phe
    1               5                  10                  15
   aac tcg acc gag tat atc gaa gac tgc ctg cgc ggc atc gcc gaa gga
   96
   Asn Ser Thr Glu Tyr Ile Glu Asp Cys Leu Arg Gly Ile Ala Glu Gly
               20                  25                  30
   gcg ggc aag acc ccc cac gaa gtt ctg ctg atc gac aat ggc gac ggg
   144
   Ala Gly Lys Thr Pro His Glu Val Leu Leu Ile Asp Asn Gly Asp Gly
           35                  40                  45
   cga acc gaa gcg ctg gtc cgg cag cgg ttc cac cac gtc cgc atc gtt
   192
   Arg Thr Glu Ala Leu Val Arg Gln Arg Phe His His Val Arg Ile Val
       50                  55                  60
   ccc agt gag ggc aat att ggt ttc ggg gcc ggc aat aat cgc ctg gca
   240
   Pro Ser Glu Gly Asn Ile Gly Phe Gly Ala Gly Asn Asn Arg Leu Ala
    65                  70                  75                  80
   gcg cag gct gcc ggc ccg ctc ctg ctg ctc gtc aac ccc gat gcc att
   288
   Ala Gln Ala Ala Gly Pro Leu Leu Leu Leu Val Asn Pro Asp Ala Ile
                    85                  90                  95
   ccc cag ccc ggc gca atc gat cag ttg gtc acc ttt gcc aaa cag cat
   336
   Pro Gln Pro Gly Ala Ile Asp Gln Leu Val Thr Phe Ala Lys Gln His
               100                 105                 110
   ccc gag gcg gcg gca tgg ggc ggc cgt tcc tac tcg ccc agc ggc gat
   384
   Pro Glu Ala Ala Ala Trp Gly Gly Arg Ser Tyr Ser Pro Ser Gly Asp
           115                 120                 125
   cta gaa ccc gca aat ttc atg tcc ctg ccg acg ccc gcc gac ttt ctg
   432
   Leu Glu Pro Ala Asn Phe Met Ser Leu Pro Thr Pro Ala Asp Phe Leu
       130                 135                 140
   acg gcg att ttc aac gcg cgt gcg cta cgc agc ggc ggg ctg caa gaa
   480
   Thr Ala Ile Phe Asn Ala Arg Ala Leu Arg Ser Gly Gly Leu Gln Glu
   145                 150                 155                 160
   ggc gcg acc acc ccc gga gcg gtc gag gtg ttg aat ggc ggc ttc atg
   528
   Gly Ala Thr Thr Pro Gly Ala Val Glu Val Leu Asn Gly Gly Phe Met
                   165                 170                 175
   atg gta cgc acc gat gtc tgg cag gcg atc ggc ggt ttt gac gag agc
   576
   Met Val Arg Thr Asp Val Trp Gln Ala Ile Gly Gly Phe Asp Glu Ser
               180                 185                 190
   ttt ttt ctt tat tcg gaa gag atc gat ctc ttc cag cga atc cgc acg
   624
   Phe Phe Leu Tyr Ser Glu Glu Ile Asp Leu Phe Gln Arg Ile Arg Thr
           195                 200                 205
   ttg ggg cac aag gtg ctc gtc gac ccc tcg gtc aaa gtg gta cac aat
   672
   Leu Gly His Lys Val Leu Val Asp Pro Ser Val Lys Val Val His Asn
       210                 215                 220
   acg ggg agt ggt cag tcg atg tcc cag aac cgc ctg atg tat ctc acg
   720
   Thr Gly Ser Gly Gln Ser Met Ser Gln Asn Arg Leu Met Tyr Leu Thr
   225                 230                 235                 240
   acc ggg cgc atg cac tat gcg cga aag cat ttt ggc gca ctc ggc acc
   768
   Thr Gly Arg Met His Tyr Ala Arg Lys His Phe Gly Ala Leu Gly Thr
                   245                 250                 255
   ctt gcc acc ggg tgc gcg ctt tgg ctg atc gcc gcc aaa tac acg ttg
   816
   Leu Ala Thr Gly Cys Ala Leu Trp Leu Ile Ala Ala Lys Tyr Thr Leu
               260                 265                 270
   gtc ggg gcg gca ctc tgg cgc ctg tcg ccg cgg acg ggc acg cga tac
   864
   Val Gly Ala Ala Leu Trp Arg Leu Ser Pro Arg Thr Gly Thr Arg Tyr
           275                 280                 285
   aaa gag ctg agc aac ggg tgg cgt gcc gta ttt agc aat cct ggc cga
   912
   Lys Glu Leu Ser Asn Gly Trp Arg Ala Val Phe Ser Asn Pro Gly Arg
       290                 295                 300
   tgg tgg agc ggc tat ccg cgt cgc taa
   939
   Trp Trp Ser Gly Tyr Pro Arg Arg  *
   305                 310
<210> 9
   <211> 312
   <212> PRT
   <213> Sphingomonas sp. ATCC53159
<220>
   <221> PEPTIDE
   <222> (0)...(0)
   <223> putative rhamnosyl transferase IV
<400> 9
   Met Gln Met Leu Pro Thr Pro Asp Val Ser Ile Leu Val Val Ala Phe
    1               5                  10                  15
   Asn Ser Thr Glu Tyr Ile Glu Asp Cys Leu Arg Gly Ile Ala Glu Gly
               20                  25                  30
   Ala Gly Lys Thr Pro His Glu Val Leu Leu Ile Asp Asn Gly Asp Gly
           35                  40                  45
   Arg Thr Glu Ala Leu Val Arg Gln Arg Phe His His Val Arg Ile Val
       50                  55                  60
   Pro Ser Glu Gly Asn Ile Gly Phe Gly Ala Gly Asn Asn Arg Leu Ala
   65                  70                  75                  80
   Ala Gln Ala Ala Gly Pro Leu Leu Leu Leu Val Asn Pro Asp Ala Ile
                   85                  90                  95
   Pro Gln Pro Gly Ala Ile Asp Gln Leu Val Thr Phe Ala Lys Gln His
               100                 105                 110
   Pro Glu Ala Ala Ala Trp Gly Gly Arg Ser Tyr Ser Pro Ser Gly Asp
           115                 120                 125
   Leu Glu Pro Ala Asn Phe Met Ser Leu Pro Thr Pro Ala Asp Phe Leu
       130                 135                 140
   Thr Ala Ile Phe Asn Ala Arg Ala Leu Arg Ser Gly Gly Leu Gln Glu
   145                 150                 155                 160
   Gly Ala Thr Thr Pro Gly Ala Val Glu Val Leu Asn Gly Gly Phe Met
                   165                 170                 175
   Met Val Arg Thr Asp Val Trp Gln Ala Ile Gly Gly Phe Asp Glu Ser
               180                 185                 190
   Phe Phe Leu Tyr Ser Glu Glu Ile Asp Leu Phe Gln Arg Ile Arg Thr
           195                 200                 205
   Leu Gly His Lys Val Leu Val Asp Pro Ser Val Lys Val Val His Asn
       210                 215                 220
   Thr Gly Ser Gly Gln Ser Met Ser Gln Asn Arg Leu Met Tyr Leu Thr
   225                 230                 235                 240
   Thr Gly Arg Met His Tyr Ala Arg Lys His Phe Gly Ala Leu Gly Thr
                   245                 250                 255
   Leu Ala Thr Gly Cys Ala Leu Trp Leu Ile Ala Ala Lys Tyr Thr Leu
               260                 265                 270
   Val Gly Ala Ala Leu Trp Arg Leu Ser Pro Arg Thr Gly Thr Arg Tyr
           275                 280                 285
   Lys Glu Leu Ser Asn Gly Trp Arg Ala Val Phe Ser Asn Pro Gly Arg
       290                 295                 300
   Trp Trp Ser Gly Tyr Pro Arg Arg
   305                 310
<210> 10
   <211> 972
   <212> DNA
   <213> Sphingomonas sp. ATCC53159
<220>
   <221> CDS
   <222> (1)...(972)
   <223> dpsI
<400> 10
   atg ctg cac tgc caa cgc tat tgc gga tgc ccg ccc gtc cga ata ggt
   48
   Met Leu His Cys Gln Arg Tyr Cys Gly Cys Pro Pro Val Arg Ile Gly
    1               5                  10                  15
   tca agt aga agt ttg tgc cgt gcg caa ttc cgt gcc ggc ggg gag gtc
   96
   Ser Ser Arg Ser Leu Cys Arg Ala Gln Phe Arg Ala Gly Gly Glu Val
                20                  25                  30
   ttc atg aag aaa ttg tac ctg gtt acg gcg gtg gct gcg gcc gcg ctg
   144
   Phe Met Lys Lys Leu Tyr Leu Val Thr Ala Val Ala Ala Ala Ala Leu
            35                  40                  45
   gcc gtc tcc gga tgt ggc ggc aag ggc ggc aag ctc gac aag ggg cag
   192
   Ala Val Ser Gly Cys Gly Gly Lys Gly Gly Lys Leu Asp Lys Gly Gln
        50                  55                  60
   gtg gtc gcc agc gtc gat ggc gaa gaa atc acc gtc ttc gag ctg aat
   240
   Val Val Ala Ser Val Asp Gly Glu Glu Ile Thr Val Phe Glu Leu Asn
    65                  70                  75                  80
   gcc gaa ctg cag gcc tcc cag gta ccc ccg ggg acc gat cgc aag ctg
   288
   Ala Glu Leu Gln Ala Ser Gln Val Pro Pro Gly Thr Asp Arg Lys Leu
   85 90 95
   gcc gag cag ctg gcg ctg cag cgc atc atc gag cgc aag atc ctc gcc
   336
   Ala Glu Gln Leu Ala Leu Gln Arg Ile Ile Glu Arg Lys Ile Leu Ala
   100 105 110
   aag gtc gcc cgc gag cag aag ctg gac aag acg cct gcc ttc ctg atc
   384
   Lys Val Ala Arg Glu Gln Lys Leu Asp Lys Thr Pro Ala Phe Leu Ile
   115 120 125
   cag gag cgc cgg gcc gac gag ctg atc ctc acc gcc atg ctg cgc gac
   432
   Gln Glu Arg Arg Ala Asp Glu Leu Ile Leu Thr Ala Met Leu Arg Asp
   130 135 140
   aag atc gcc ggc ggc atc gcc cag ccg acc gat gcc gag atc gag aaa
   480
   Lys Ile Ala Gly Gly Ile Ala Gln Pro Thr Asp Ala Glu Ile Glu Lys
   145 150 155 160
   tat cag gcc gcg cat ccg gag cgg ttc gcg cag cgc aag atc tac gcg
   528
   Tyr Gln Ala Ala His Pro Glu Arg Phe Ala Gln Arg Lys Ile Tyr Ala
   165 170 175
   atc gat cag gtc gtc ttc gct ccg ccg agc tcg gcc gca aag ctc aag
   576
   Ile Asp Gln Val Val Phe Ala Pro Pro Ser Ser Ala Ala Lys Leu Lys
   180 185 190
   caa ttc gcg ccg ctg aag acg ctg gac cag cta acc gcc aag ctc tcg
   624
   Gln Phe Ala Pro Leu Lys Thr Leu Asp Gln Leu Thr Ala Lys Leu Ser
   195 200 205
   gcg gac aat gtc cag ttc cgt cgc gcg ccg tcg cag atc gac acc gct
   672
   Ala Asp Asn Val Gln Phe Arg Arg Ala Pro Ser Gln Ile Asp Thr Ala
   210 215 220
   gcg ctg ccg ccg gaa atc gct gcc aag atc gcg tcg ctg ccg gca cag
   720
   Ala Leu Pro Pro Glu Ile Ala Ala Lys Ile Ala Ser Leu Pro Ala Gln
   225 230 235 240
   gag atg ttc atc ctg ccg acc cag cag gga ctg acc gcg aat atc atc
   768
   Glu Met Phe Ile Leu Pro Thr Gln Gln Gly Leu Thr Ala Asn Ile Ile
   245 250 255
   acg tcg acc acg gtg ctg ccg gtg ccg gcc gac cag gcg cgc gag atc
   816
   Thr Ser Thr Thr Val Leu Pro Val Pro Ala Asp Gln Ala Arg Glu Ile
   260 265 270
   gcg ctc agc ggg ctg cgt acc gag cgc ttc ggc aag gcg gct gac gca
   864
   Ala Leu Ser Gly Leu Arg Thr Glu Arg Phe Gly Lys Ala Ala Asp Ala
           275                 280                 285
   cag ctc aac gac cgc ctg aag aag gcg cgg gaa acc gtg aaa tat cag
   912
   Gln Leu Asn Asp Arg Leu Lys Lys Ala Arg Glu Thr Val Lys Tyr Gln
       290                 295                 300
   gcc ggc tac agc gca ccg ccg cag ctt cgc ggc agc ggc gca acg ccg
   960
   Ala Gly Tyr Ser Ala Pro Pro Gln Leu Arg Gly Ser Gly Ala Thr Pro
   305                 310                 315                 320
   gcg ggg aac tga
   972
   Ala Gly Asn  *
<210> 11
   <211> 323
   <212> PRT
   <213> Sphingomonas sp. ATCC53159
<220>
   <221> PEPTIDE
   <222> (0) ... (0)
   <223> unknown
<400> 11
   Met Leu His Cys Gln Arg Tyr Cys Gly Cys Pro Pro Val Arg Ile Gly
    1               5                  10                  15
   Ser Ser Arg Ser Leu Cys Arg Ala Gln Phe Arg Ala Gly Gly Glu Val
               20                  25                  30
   Phe Met Lys Lys Leu Tyr Leu Val Thr Ala Val Ala Ala Ala Ala Leu
           35                  40                  45
   Ala Val Ser Gly Cys Gly Gly Lys Gly Gly Lys Leu Asp Lys Gly Gln
       50                  55                  60
   Val Val Ala Ser Val Asp Gly Glu Glu Ile Thr Val Phe Glu Leu Asn
   65                  70                  75                  80
   Ala Glu Leu Gln Ala Ser Gln Val Pro Pro Gly Thr Asp Arg Lys Leu
                   85                  90                  95
   Ala Glu Gln Leu Ala Leu Gln Arg Ile Ile Glu Arg Lys Ile Leu Ala
               100                 105                 110
   Lys Val Ala Arg Glu Gln Lys Leu Asp Lys Thr Pro Ala Phe Leu Ile
           115                 120                 125
   Gln Glu Arg Arg Ala Asp Glu Leu Ile Leu Thr Ala Met Leu Arg Asp
       130                 135                 140
   Lys Ile Ala Gly Gly Ile Ala Gln Pro Thr Asp Ala Glu Ile Glu Lys
   145                 150                 155                 160
   Tyr Gln Ala Ala His Pro Glu Arg Phe Ala Gln Arg Lys Ile Tyr Ala
                   165                 170                 175
   Ile Asp Gln Val Val Phe Ala Pro Pro Ser Ser Ala Ala Lys Leu Lys
               180                 185                 190
   Gln Phe Ala Pro Leu Lys Thr Leu Asp Gln Leu Thr Ala Lys Leu Ser
           195                 200                 205
   Ala Asp Asn Val Gln Phe Arg Arg Ala Pro Ser Gln Ile Asp Thr Ala
       210                 215                 220
   Ala Leu Pro Pro Glu Ile Ala Ala Lys Ile Ala Ser Leu Pro Ala Gln
   225                 230                 235                 240
   Glu Met Phe Ile Leu Pro Thr Gln Gln Gly Leu Thr Ala Asn Ile Ile
                   245                 250                 255
   Thr Ser Thr Thr Val Leu Pro Val Pro Ala Asp Gln Ala Arg Glu Ile
               260                 265                 270
   Ala Leu Ser Gly Leu Arg Thr Glu Arg Phe Gly Lys Ala Ala Asp Ala
           275                 280                 285
   Gln Leu Asn Asp Arg Leu Lys Lys Ala Arg Glu Thr Val Lys Tyr Gln
       290                 295                 300
   Ala Gly Tyr Ser Ala Pro Pro Gln Leu Arg Gly Ser Gly Ala Thr Pro
   305                 310                 315                 320
   Ala Gly Asn
<210> 12
   <211> 1047
   <212> DNA
   <213> Sphingomonas sp. ATCC53159
<220>
   <221> CDS
   <222> (1)...(1047)
   <223> dpsK
<400> 12
   atg gca gaa gcg aac gcg gta gat gga aag gcc tcc aag ccg ctg aaa
   48
   Met Ala Glu Ala Asn Ala Val Asp Gly Lys Ala Ser Lys Pro Leu Lys
    1               5                  10                  15
   atg tgc ctt gca gcg tcg ggc ggc ggc cat ctc cgg caa atc ctc gat
   96
   Met Cys Leu Ala Ala Ser Gly Gly Gly His Leu Arg Gln Ile Leu Asp
               20                  25                  30
   ctg gaa tcg gtg tgg cgc gaa cac gat tat ttc ttc gtt act gaa gat
   144
   Leu Glu Ser Val Trp Arg Glu His Asp Tyr Phe Phe Val Thr Glu Asp
           35                  40                  45
   acc gcg ctc ggc cgg agc ctt gcc gaa aaa cat ccc gtc gaa ctg gtg
   192
   Thr Ala Leu Gly Arg Ser Leu Ala Glu Lys His Pro Val Glu Leu Val
       50                  55                  60
   gag cac tat gcg ctc ggc cag gcc aag ctg ggc cat ccc ttg cgc atg
   240
   Glu His Tyr Ala Leu Gly Gln Ala Lys Leu Gly His Pro Leu Arg Met
    65                  70                  75                  80
   ctg ggc ggc gca tgg cgc aac ctg cgc cag agc ctt tcg atc ctg cgc
   288
   Leu Gly Gly Ala Trp Arg Asn Leu Arg Gln Ser Leu Ser Ile Leu Arg
                   85                  90                  95
   cgg cac aag ccg gat gtg gtg att tcc acc ggc gcg ggc gca gtc tat
   336
   Arg His Lys Pro Asp Val Val Ile Ser Thr Gly Ala Gly Ala Val Tyr
               100                 105                 110
   ttc acc gcg ctg ctc gcc aaa ctg tcg ggc gcc aag ttc gtc cat atc
   384
   Phe Thr Ala Leu Leu Ala Lys Leu Ser Gly Ala Lys Phe Val His Ile
           115                 120                 125
   gaa agc ttc gcg cgc ttc gac cac ccg tct gcc ttc ggc aag atg gtg
   432
   Glu Ser Phe Ala Arg Phe Asp His Pro Ser Ala Phe Gly Lys Met Val
       130                 135                 140
   aag ggc atc gcg acg gtg acg atc gtc cag tcg gcg gcg ctg aaa gaa
   480
   Lys Gly Ile Ala Thr Val Thr Ile Val Gln Ser Ala Ala Leu Lys Glu
   145                 150                 155                 160
   acc tgg cct gat gcc gag ctg ttc gat ccg ttc cgc ctg ctc gat aca
   528
   Thr Trp Pro Asp Ala Glu Leu Phe Asp Pro Phe Arg Leu Leu Asp Thr
                   165                 170                 175
   ccg cgc ccg ccc aag cag gcg cta atc ttc gcg acg gtc ggc gcc acc
   576
   Pro Arg Pro Pro Lys Gln Ala Leu Ile Phe Ala Thr Val Gly Ala Thr
               180                 185                 190
   ctg ccc ttc ccg cgg ctg gtg cag gca gtg ctc gac ctg aag cgc gcc
   624
   Leu Pro Phe Pro Arg Leu Val Gln Ala Val Leu Asp Leu Lys Arg Ala
           195                 200                 205
   ggc ggg ctg ccg ggc aag ctg atc ctg caa tat ggc gac cag gac ctg
   672
   Gly Gly Leu Pro Gly Lys Leu Ile Leu Gln Tyr Gly Asp Gln Asp Leu
       210                 215                 220
   ccc gat ccc ggc atc ccc gac gtc gag atc cgc cgt acc atc ccg ttc
   720
   Pro Asp Pro Gly Ile Pro Asp Val Glu Ile Arg Arg Thr Ile Pro Phe
   225                 230                 235                 240
   gac gat ctg cag ctg ctg ctg cgc gat gcg gat atg gtg ata tgc cac
   768
   Asp Asp Leu Gln Leu Leu Leu Arg Asp Ala Asp Met Val Ile Cys His
                   245                 250                 255
   ggc ggc acc gga tcg ctg gtc acg gcg ctg cgc gcc ggc tgc cgg gtc
   816
   Gly Gly Thr Gly Ser Leu Val Thr Ala Leu Arg Ala Gly Cys Arg Val
               260                 265                 270
   gtc gcc ttt ccg cgc cgc cac gat ctg ggc gag cat tat gac gat cac
   864
   Val Ala Phe Pro Arg Arg His Asp Leu Gly Glu His Tyr Asp Asp His
           275                 280                 285
   cag gaa gag atc gcc cag acc ttc gcc gac cgg ggc ctg ctc cag gcg
   912
   Gln Glu Glu Ile Ala Gln Thr Phe Ala Asp Arg Gly Leu Leu Gln Ala
       290                 295                 300
   gtg cgc gac gag cgc cag ctc ggc gcc gct gtg gaa gcg gcc aag gca
   960
   Val Arg Asp Glu Arg Gln Leu Gly Ala Ala Val Glu Ala Ala Lys Ala
   305                 310                 315                 320
   acc gag ccg cag ctg gcg acc acc gac cac acg gcc ctc gcg gcg cgg
   1008
   Thr Glu Pro Gln Leu Ala Thr Thr Asp His Thr Ala Leu Ala Ala Arg
                   325                 330                 335
   ctg cgc cag ctg ctg gcg cag tgg agt gcc aag cga tga
   1047
   Leu Arg Gln Leu Leu Ala Gln Trp Ser Ala Lys Arg  *
               340                 345
<210> 13
   <211> 348
   <212> PRT
   <213> Sphingomonas sp. ATCC53159
<220>
   <221> PEPTIDE
   <222> (0) ... (0)
   <223> beta-1,4-glucuronosyl transferase II
<400> 13
   Met Ala Glu Ala Asn Ala Val Asp Gly Lys Ala Ser Lys Pro Leu Lys
    1               5                  10                  15
   Met Cys Leu Ala Ala Ser Gly Gly Gly His Leu Arg Gln Ile Leu Asp
               20                  25                  30
   Leu Glu Ser Val Trp Arg Glu His Asp Tyr Phe Phe Val Thr Glu Asp
           35                  40                  45
   Thr Ala Leu Gly Arg Ser Leu Ala Glu Lys His Pro Val Glu Leu Val
       50                  55                  60
   Glu His Tyr Ala Leu Gly Gln Ala Lys Leu Gly His Pro Leu Arg Met
   65                  70                  75                  80
   Leu Gly Gly Ala Trp Arg Asn Leu Arg Gln Ser Leu Ser Ile Leu Arg
                   85                  90                  95
   Arg His Lys Pro Asp Val Val Ile Ser Thr Gly Ala Gly Ala Val Tyr
               100                 105                 110
   Phe Thr Ala Leu Leu Ala Lys Leu Ser Gly Ala Lys Phe Val His Ile
           115                 120                 125
   Glu Ser Phe Ala Arg Phe Asp His Pro Ser Ala Phe Gly Lys Met Val
       130                 135                 140
   Lys Gly Ile Ala Thr Val Thr Ile Val Gln Ser Ala Ala Leu Lys Glu
   145                 150                 155                 160
   Thr Trp Pro Asp Ala Glu Leu Phe Asp Pro Phe Arg Leu Leu Asp Thr
                   165                 170                 175
   Pro Arg Pro Pro Lys Gln Ala Leu Ile Phe Ala Thr Val Gly Ala Thr
               180                 185                 190
   Leu Pro Phe Pro Arg Leu Val Gln Ala Val Leu Asp Leu Lys Arg Ala
           195                 200                 205
   Gly Gly Leu Pro Gly Lys Leu Ile Leu Gln Tyr Gly Asp Gln Asp Leu
       210                 215                 220
   Pro Asp Pro Gly Ile Pro Asp Val Glu Ile Arg Arg Thr Ile Pro Phe
   225                 230                 235                 240
   Asp Asp Leu Gln Leu Leu Leu Arg Asp Ala Asp Met Val Ile Cys His
                   245                 250                 255
   Gly Gly Thr Gly Ser Leu Val Thr Ala Leu Arg Ala Gly Cys Arg Val
               260                 265                 270
   Val Ala Phe Pro Arg Arg His Asp Leu Gly Glu His Tyr Asp Asp His
           275                 280                 285
   Gln Glu Glu Ile Ala Gln Thr Phe Ala Asp Arg Gly Leu Leu Gln Ala
       290                 295                 300
   Val Arg Asp Glu Arg Gln Leu Gly Ala Ala Val Glu Ala Ala Lys Ala
   305                 310                 315                 320
   Thr Glu Pro Gln Leu Ala Thr Thr Asp His Thr Ala Leu Ala Ala Arg
                   325                 330                 335
   Leu Arg Gln Leu Leu Ala Gln Trp Ser Ala Lys Arg
               340                 345
<210> 14
   <211> 867
   <212> DNA
   <213> Sphingomonas sp. ATCC53159
<220>
   <221> CDS
   <222> (1)...(867)
   <223> dpsL
<400> 14
   atg agc acg ccc cgg atc agc gtc gtc atc ccg cac tat aac gat ccg
   48
   Met Ser Thr Pro Arg Ile Ser Val Val Ile Pro His Tyr Asn Asp Pro
    1               5                  10                  15
   caa tcc ttg cgg ctc tgc ctg gat gcg ctg gag cgg cag acg atc ggt
   96
   Gln Ser Leu Arg Leu Cys Leu Asp Ala Leu Glu Arg Gln Thr Ile Gly
               20                  25                  30
   cgc gac gcg ttc gag atc atc gtc ggc gac aac aat tcg ccc tgt ggg
   144
   Arg Asp Ala Phe Glu Ile Ile Val Gly Asp Asn Asn Ser Pro Cys Gly
           35                  40                  45
   ctc gcg gcg gtg gag gcg gcg gtc gcc gga cgt gcg cgg atc gtg acc
   192
   Leu Ala Ala Val Glu Ala Ala Val Ala Gly Arg Ala Arg Ile Val Thr
       50                  55                  60
   att ctg gaa aag ggg gcg ggc ccc gcg cgc aac ggg gcg gca gcc gca
   240
   Ile Leu Glu Lys Gly Ala Gly Pro Ala Arg Asn Gly Ala Ala Ala Ala
    65                  70                  75                  80
   gcg cgt ggc gag atc ctc gcc ttt acc gac agt gac tgc gtg gtg gag
   288
   Ala Arg Gly Glu Ile Leu Ala Phe Thr Asp Ser Asp Cys Val Val Glu
                   85                  90                  95
   ccc ggc tgg ctg gcg ggc ggc acg acc agg gtc gcg cct ggc cgt ttc
   336
   Pro Gly Trp Leu Ala Gly Gly Thr Thr Arg Val Ala Pro Gly Arg Phe
               100                 105                 110
   atc ggc ggg cac atg tat gtg cgc aag ccc gaa ggg ccg ccg aac ggc
   384
   Ile Gly Gly His Met Tyr Val Arg Lys Pro Glu Gly Pro Pro Asn Gly
           115                 120                 125
   gcc gag gcg ctg gag atg gcg ctg gcg ttc gac aat gaa ggc tat gtg
   432
   Ala Glu Ala Leu Glu Met Ala Leu Ala Phe Asp Asn Glu Gly Tyr Val
       130                 135                 140
   cgg cgc acc cag ttc acg gtc acc gca aac ctg ttc gtg atg cgc gcc
   480
   Arg Arg Thr Gln Phe Thr Val Thr Ala Asn Leu Phe Val Met Arg Ala
   145                 150                 155                 160
   gat ttc gaa cgg gtc ggc ggc ttc cgc gtt ggc gtg tcc gag gat ctg
   528
   Asp Phe Glu Arg Val Gly Gly Phe Arg Val Gly Val Ser Glu Asp Leu
                   165                 170                 175
   gaa tgg tgc cac cgg gcg atc gcc agc ggc ctc acc atc aac tat gca
   576
   Glu Trp Cys His Arg Ala Ile Ala Ser Gly Leu Thr Ile Asn Tyr Ala
               180                 185                 190
   ccg gat gca tcg gtg ggc cac ccg ccc cgg ccc gac tgg tcg gcc ctg
   624
   Pro Asp Ala Ser Val Gly His Pro Pro Arg Pro Asp Trp Ser Ala Leu
           195                 200                 205
   ctg gtg aag acg cgg cgc atc cag cgc gaa ctc tat ctg ttc aac atc
   672
   Leu Val Lys Thr Arg Arg Ile Gln Arg Glu Leu Tyr Leu Phe Asn Ile
       210                 215                 220
   gag cgg ccg aag ggc agg ctg cgc tgg ctg gtc cgt tcc gtg gcg caa
   720
   Glu Arg Pro Lys Gly Arg Leu Arg Trp Leu Val Arg Ser Val Ala Gln
   225                 230                 235                 240
   ccg gcg atg atc cca cag gac gtg gcc aag atc ctg cgc aca ccg ggt
   768
   Pro Ala Met Ile Pro Gln Asp Val Ala Lys Ile Leu Arg Thr Pro Gly
                   245                 250                 255
   acc aag ggc gcg cgc ctc gct gcg gtc acc acg ctg gtc cgg ctg cgg
   816
   Thr Lys Gly Ala Arg Leu Ala Ala Val Thr Thr Leu Val Arg Leu Arg
               260                 265                 270
   ctg tgg cgc ggc ggc gcc ggc ttg ttg cag ttg ctc ggc cgc gac atc
   864
   Leu Trp Arg Gly Gly Ala Gly Leu Leu Gln Leu Leu Gly Arg Asp Ile
           275                 280                 285
   tga
   867
    *
<210> 15
   <211> 288
   <212> PRT
   <213> Sphingomonas sp. ATCC53159
<220>
   <221> PEPTIDE
   <222> (0) ... (0)
   <223> glucosyl transferase III
<400> 15
   Met Ser Thr Pro Arg Ile Ser Val Val Ile Pro His Tyr Asn Asp Pro
    1               5                  10                  15
   Gln Ser Leu Arg Leu Cys Leu Asp Ala Leu Glu Arg Gln Thr Ile Gly
               20                  25                  30
   Arg Asp Ala Phe Glu Ile Ile Val Gly Asp Asn Asn Ser Pro Cys Gly
           35                  40                  45
   Leu Ala Ala Val Glu Ala Ala Val Ala Gly Arg Ala Arg Ile Val Thr
       50                  55                  60
   Ile Leu Glu Lys Gly Ala Gly Pro Ala Arg Asn Gly Ala Ala Ala Ala
   65                  70                  75                  80
   Ala Arg Gly Glu Ile Leu Ala Phe Thr Asp Ser Asp Cys Val Val Glu
                   85                  90                  95
   Pro Gly Trp Leu Ala Gly Gly Thr Thr Arg Val Ala Pro Gly Arg Phe
               100                 105                 110
   Ile Gly Gly His Met Tyr Val Arg Lys Pro Glu Gly Pro Pro Asn Gly
           115                 120                 125
   Ala Glu Ala Leu Glu Met Ala Leu Ala Phe Asp Asn Glu Gly Tyr Val
       130                 135                 140
   Arg Arg Thr Gln Phe Thr Val Thr Ala Asn Leu Phe Val Met Arg Ala
   145                 150                 155                 160
   Asp Phe Glu Arg Val Gly Gly Phe Arg Val Gly Val Ser Glu Asp Leu
                   165                 170                 175
   Glu Trp Cys His Arg Ala Ile Ala Ser Gly Leu Thr Ile Asn Tyr Ala
               180                 185                 190
   Pro Asp Ala Ser Val Gly His Pro Pro Arg Pro Asp Trp Ser Ala Leu
           195                 200                 205
   Leu Val Lys Thr Arg Arg Ile Gln Arg Glu Leu Tyr Leu Phe Asn Ile
       210                     215                     220
   Glu Arg Pro Lys Gly Arg Leu Arg Trp Leu Val Arg Ser Val Ala Gln
   225                 230                     235                 240
   Pro Ala Met Ile Pro Gln Asp Val Ala Lys Ile Leu Arg Thr Pro Gly
                   245                     250                 255
   Thr Lys Gly Ala Arg Leu Ala Ala Val Thr Thr Leu Val Arg Leu Arg
               260                     265                 270
   Leu Trp Arg Gly Gly Ala Gly Leu Leu Gln Leu Leu Gly Arg Asp Ile
           275                     280                 285
<210> 16
   <211> 1389
   <212> DNA
   <213> Sphingomonas sp. ATCC53159
<220>
   <221> CDS
   <222> (1)...(1389)
   <223> dpsJ
<400> 16
   gtg gct gta ggt tcc gcg ctg aga ttc ttg tgg ccg ttc ggc cgt cga
   48
   Val Ala Val Gly Ser Ala Leu Arg Phe Leu Trp Pro Phe Gly Arg Arg
    1              5                   10                  15
   gaa gaa cct gaa gaa gag ggc tat ttc ccg ctg act gcg aca gtg gtg
   96
   Glu Glu Pro Glu Glu Glu Gly Tyr Phe Pro Leu Thr Ala Thr Val Val
               20                  25                  30
   ccg cat cgc gat gcc cat agc ggc cgt ggt cgc ccg gat ttc cca acc
   144
   Pro His Arg Asp Ala His Ser Gly Arg Gly Arg Pro Asp Phe Pro Thr
           35                  40                  45
   ttc cgt gcc tcc gcg ctg gac cgc ccg ctg gat cgc cgc cgc gac gag
   192
   Phe Arg Ala Ser Ala Leu Asp Arg Pro Leu Asp Arg Arg Arg Asp Glu
       50                  55                  60
   cgc cgc gag atc acg cgc gcc cgg ttc gcg ctg gcg acc ttc ttc aca
   240
   Arg Arg Glu Ile Thr Arg Ala Arg Phe Ala Leu Ala Thr Phe Phe Thr
    65                 70                  75                  80
   ccc acc cag ccg gtg gcc gat cgg tcg agc ttc gcc ggg cgc ctc ggc
   288
   Pro Thr Gln Pro Val Ala Asp Arg Ser Ser Phe Ala Gly Arg Leu Gly
                   85                  90                  95
   gtg ctg gcg cgc cta atc tcc tcg atc gag agc cag cgc agc cat gtc
   336
   Val Leu Ala Arg Leu Ile Ser Ser Ile Glu Ser Gln Arg Ser His Val
               100                 105                 110
   gtg ctc tat ggc gag cgc ggc atc ggc aag acc tcg ctg ctc cac gtg
   384
   Val Leu Tyr Gly Glu Arg Gly Ile Gly Lys Thr Ser Leu Leu His Val
           115                 120                 125
   ctg acc gat gtc gcc cgc gaa tcc agc tat atc gtc agc tat gcg acc
   432
   Leu Thr Asp Val Ala Arg Glu Ser Ser Tyr Ile Val Ser Tyr Ala Thr
       130                 135                 140
   tgc ggt gcg aac gcg aat ttc agc gat gtc ttc cgc gcc gtg ctg gaa
   480
   Cys Gly Ala Asn Ala Asn Phe Ser Asp Val Phe Arg Ala Val Leu Glu
   145                 150                 155                 160
   gac gtg ccg ctg ctg ttc cat cgc ggc gtg gcg ccc aac gcc ggc gag
   528
   Asp Val Pro Leu Leu Phe His Arg Gly Val Ala Pro Asn Ala Gly Glu
                   165                 170                 175
   gcg gag agc ggc ggc aac ttg gcc gac cgc ctg ccg acg ggc agc ttc
   576
   Ala Glu Ser Gly Gly Asn Leu Ala Asp Arg Leu Pro Thr Gly Ser Phe
               180                 185                 190
   ggg ccc ggc gaa ctg gcc gac ctg tgc gcc gac atc acg ggc aca cgc
   624
   Gly Pro Gly Glu Leu Ala Asp Leu Cys Ala Asp Ile Thr Gly Thr Arg
           195                 200                 205
   gtg ctg atc atc ctc gac gaa tat gat cgc gtc agc gat tcc gcc ttc
   672
   Val Leu Ile Ile Leu Asp Glu Tyr Asp Arg Val Ser Asp Ser Ala Phe
       210                 215                 220
   cgt cag cag gtc gcc gag ctg atc aag aac ctg tcg gac cgt tcg gcg
   720
   Arg Gln Gln Val Ala Glu Leu Ile Lys Asn Leu Ser Asp Arg Ser Ala
   225                 230                 235                 240
   cgc gtc cag ctg gtg atc gcg ggc gtc gcc tcg aac ctg cag gag ctg
   768
   Arg Val Gln Leu Val Ile Ala Gly Val Ala Ser Asn Leu Gln Glu Leu
                   245                 250                 255
   atc ggt tat gcg ccg tcg atc cgc cgc aac gtc atc ggc ctg ccg atg
   816
   Ile Gly Tyr Ala Pro Ser Ile Arg Arg Asn Val Ile Gly Leu Pro Met
               260                 265                 270
   ccc cgg ctg gag gaa tcg gag gtg cag gag atg atc gcg ctc ggc gaa
   864
   Pro Arg Leu Glu Glu Ser Glu Val Gln Glu Met Ile Ala Leu Gly Glu
           275                 280                 285
   acc gcc tcg ggc gtt cgc ttc gat ccg gac ctg act cac atg atc cac
   912
   Thr Ala Ser Gly Val Arg Phe Asp Pro Asp Leu Thr His Met Ile His
       290                 295                 300
   ctg ctc gcg ctg ggg tcg ccc tat ttc gcg cgg ctg ctg tgc cac cat
   960
   Leu Leu Ala Leu Gly Ser Pro Tyr Phe Ala Arg Leu Leu Cys His His
   305                 310                 315                 320
   tcc gcg ctg gaa gcc ctg gac cag ggc cgc ctc acg gtc gac gcc ggg
   1008
   Ser Ala Leu Glu Ala Leu Asp Gln Gly Arg Leu Thr Val Asp Ala Gly
                   325                 330                 335
   cat ctg cgt cgt gcg ctc gac cag gcg atc ctt gag atc gag ggc cgc
   1056
   His Leu Arg Arg Ala Leu Asp Gln Ala Ile Leu Glu Ile Glu Gly Arg
               340                 345                 350
   atg ccg ccg cgc gcg gtg atc gag atg cgc aag ttc gtc ggc ggc cgc
   1104
   Met Pro Pro Arg Ala Val Ile Glu Met Arg Lys Phe Val Gly Gly Arg
           355                 360                 365
   tac gat cca ctc gtc gcg gcg ctg ggc gag gcc tcg cgc tcg gcg gat
   1152
   Tyr Asp Pro Leu Val Ala Ala Leu Gly Glu Ala Ser Arg Ser Ala Asp
       370                 375                 380
   ggc tgg ttc agc ggc caa gcc gtg gtg gat ctg ctg ccg ggc gcg cac
   1200
   Gly Trp Phe Ser Gly Gln Ala Val Val Asp Leu Leu Pro Gly Ala His
   385                 390                 395                 400
   atc acg gcg gcg cag gtc gag cag gag ctg ggc gag ctc acc ggt caa
   1248
   Ile Thr Ala Ala Gln Val Glu Gln Glu Leu Gly Glu Leu Thr Gly Gln
                   405                 410                 415
   ctc ggc ctc gaa tcc gaa acg cag gac ggc gat tgc cgc ttc cgc ttc
   1296
   Leu Gly Leu Glu Ser Glu Thr Gln Asp Gly Asp Cys Arg Phe Arg Phe
               420                 425                 430
   acc gac gat acg ctg ccg gtc tat ctg tgg ctg atg atc ggc cgc ctg
   1344
   Thr Asp Asp Thr Leu Pro Val Tyr Leu Trp Leu Met Ile Gly Arg Leu
           435                 440                 445
   cgg ctc gac agc ggc acg ctg gaa gac gcg ctg gcc acc gtc tga
   1389
   Arg Leu Asp Ser Gly Thr Leu Glu Asp Ala Leu Ala Thr Val  *
       450                 455                 460
<210> 17
   <211> 462
   <212> PRT
   <213> Sphingomonas sp. ATCC53159
<220>
   <221> PEPTIDE
   <222> (0)...(0)
   <223> unknown
<400> 17
   Val Ala Val Gly Ser Ala Leu Arg Phe Leu Trp Pro Phe Gly Arg Arg
    1               5                   10                  15
   Glu Glu Pro Glu Glu Glu Gly Tyr Phe Pro Leu Thr Ala Thr Val Val
               20                  25                  30
   Pro His Arg Asp Ala His Ser Gly Arg Gly Arg Pro Asp Phe Pro Thr
           35                  40                  45
   Phe Arg Ala Ser Ala Leu Asp Arg Pro Leu Asp Arg Arg Arg Asp Glu
       50                  55                  60
   Arg Arg Glu Ile Thr Arg Ala Arg Phe Ala Leu Ala Thr Phe Phe Thr
   65                  70                  75                  80
   Pro Thr Gln Pro Val Ala Asp Arg Ser Ser Phe Ala Gly Arg Leu Gly
                   85                  90                  95
   Val Leu Ala Arg Leu Ile Ser Ser Ile Glu Ser Gln Arg Ser His Val
               100                 105                 110
   Val Leu Tyr Gly Glu Arg Gly Ile Gly Lys Thr Ser Leu Leu His Val
           115                 120                 125
   Leu Thr Asp Val Ala Arg Glu Ser Ser Tyr Ile Val Ser Tyr Ala Thr
       130                 135                 140
   Cys Gly Ala Asn Ala Asn Phe Ser Asp Val Phe Arg Ala Val Leu Glu
   145                 150                 155                 160
   Asp Val Pro Leu Leu Phe His Arg Gly Val Ala Pro Asn Ala Gly Glu
                   165                 170                 175
   Ala Glu Ser Gly Gly Asn Leu Ala Asp Arg Leu Pro Thr Gly Ser Phe
               180                 185                 190
   Gly Pro Gly Glu Leu Ala Asp Leu Cys Ala Asp Ile Thr Gly Thr Arg
           195                 200                 205
   Val Leu Ile Ile Leu Asp Glu Tyr Asp Arg Val Ser Asp Ser Ala Phe
       210                 215                 220
   Arg Gln Gln Val Ala Glu Leu Ile Lys Asn Leu Ser Asp Arg Ser Ala
   225                 230                 235                 240
   Arg Val Gln Leu Val Ile Ala Gly Val Ala Ser Asn Leu Gln Glu Leu
                   245                 250                 255
   Ile Gly Tyr Ala Pro Ser Ile Arg Arg Asn Val Ile Gly Leu Pro Met
               260                 265                 270
   Pro Arg Leu Glu Glu Ser Glu Val Gln Glu Met Ile Ala Leu Gly Glu
           275                 280                 285
   Thr Ala Ser Gly Val Arg Phe Asp Pro Asp Leu Thr His Met Ile His
       290                 295                 300
   Leu Leu Ala Leu Gly Ser Pro Tyr Phe Ala Arg Leu Leu Cys His His
   305                 310                 315                 320
   Ser Ala Leu Glu Ala Leu Asp Gln Gly Arg Leu Thr Val Asp Ala Gly
                   325                 330                 335
   His Leu Arg Arg Ala Leu Asp Gln Ala Ile Leu Glu Ile Glu Gly Arg
               340                 345                 350
   Met Pro Pro Arg Ala Val Ile Glu Met Arg Lys Phe Val Gly Gly Arg
           355                 360                 365
   Tyr Asp Pro Leu Val Ala Ala Leu Gly Glu Ala Ser Arg Ser Ala Asp
       370                 375                 380
   Gly Trp Phe Ser Gly Gln Ala Val Val Asp Leu Leu Pro Gly Ala His
   385                 390                 395                 400
   Ile Thr Ala Ala Gln Val Glu Gln Glu Leu Gly Glu Leu Thr Gly Gln
                   405                 410                 415
   Leu Gly Leu Glu Ser Glu Thr Gln Asp Gly Asp Cys Arg Phe Arg Phe
               420                 425                 430
   Thr Asp Asp Thr Leu Pro Val Tyr Leu Trp Leu Met Ile Gly Arg Leu
           435                 440                 445
   Arg Leu Asp Ser Gly Thr Leu Glu Asp Ala Leu Ala Thr Val
       450                 455                 460
<210> 18
   <211> 1299
   <212> DNA
   <213> Sphingomonas sp. ATCC53159
<220>
   <221> CDS
   <222> (1)...(1299)
   <223> dpsF
<400> 18
   atg aag ccg aga ccc ggg gga acc ttt atg caa gta aat ttc aat cga
   48
   Met Lys Pro Arg Pro Gly Gly Thr Phe Met Gln Val Asn Phe Asn Arg
    1               5                  10                  15
   cag gct cgc aag ctc ggt gcc ggc aat gcg ctc gcg cgg ggg ggg ccc
   96
   Gln Ala Arg Lys Leu Gly Ala Gly Asn Ala Leu Ala Arg Gly Gly Pro
               20                  25                  30
   gtg ctt gcg ctg ctt gcg acc gcg gca tgg aca caa cct gcg ctg gcg
   144
   Val Leu Ala Leu Leu Ala Thr Ala Ala Trp Thr Gln Pro Ala Leu Ala
           35                  40                  45
   cag cga cag gca ttt gag tcc cgc ccc tcc ggt agc gag cga cag gtc
   192
   Gln Arg Gln Ala Phe Glu Ser Arg Pro Ser Gly Ser Glu Arg Gln Val
       50                  55                  60
   gat att cgc gcg acg ggg tcg ctg gaa tat gac gac aac gtc gtg ctg
   240
   Asp Ile Arg Ala Thr Gly Ser Leu Glu Tyr Asp Asp Asn Val Val Leu
    65                  70                  75                  80
   aac gac cag cgg atc acg gac ggc gcg cgt ggc gat gtg atc gca tcg
   288
   Asn Asp Gln Arg Ile Thr Asp Gly Ala Arg Gly Asp Val Ile Ala Ser
                   85                  90                  95
   ccc ggg ctg gac gtg acc cta gtt ctg ccc cgc gcc acc ggg cag ctc
   336
   Pro Gly Leu Asp Val Thr Leu Val Leu Pro Arg Ala Thr Gly Gln Leu
               100                 105                 110
   tac ctc acc ggc aat gtc gga tat cgc ttt tac aag cga tat acc aac
   384
   Tyr Leu Thr Gly Asn Val Gly Tyr Arg Phe Tyr Lys Arg Tyr Thr Asn
           115                 120                 125
   ttt aac cgc gag cag atc tcg ctc acc ggc ggc gca gat cag cgg ttc
   432
   Phe Asn Arg Glu Gln Ile Ser Leu Thr Gly Gly Ala Asp Gln Arg Phe
       130                 135                 140
   gcc tcc tgc gtc gtg cac ggg gaa gtc ggc tat cag cgc cac ctc acc
   480
   Ala Ser Cys Val Val His Gly Glu Val Gly Tyr Gln Arg His Leu Thr
   145                 150                 155                 160
   gac ctg tcc agc atc ttg atc cag gac acc acg cct gcg ctc aac aac
   528
   Asp Leu Ser Ser Ile Leu Ile Gln Asp Thr Thr Pro Ala Leu Asn Asn
                   165                 170                 175
   acc gaa gag gcc cgg cag tac acc gcg gat atc ggc tgc ggc gcg acc
   576
   Thr Glu Glu Ala Arg Gln Tyr Thr Ala Asp Ile Gly Cys Gly Ala Thr
               180                 185                 190
   tac ggc ctg cgg cct gcc gtt tcc tac acc cgc aac gaa gtg cgc aac
   624
   Tyr Gly Leu Arg Pro Ala Val Ser Tyr Thr Arg Asn Glu Val Arg Asn
           195                 200                 205
   agc ctt gcc gag cgc cga tac gcg gac tcg aat acc aac acc ttt acc
   672
   Ser Leu Ala Glu Arg Arg Tyr Ala Asp Ser Asn Thr Asn Thr Phe Thr
       210                 215                 220
   gca cag ctt ggc ctg act tcg cct gcc ctg ggg acc gtg gcg gta ttt
   720
   Ala Gln Leu Gly Leu Thr Ser Pro Ala Leu Gly Thr Val Ala Val Phe
   225                 230                 235                 240
   ggg cgt atg tcc gac agc agc tat gtc cat cgc gtc ctt ccc ggc att
   768
   Gly Arg Met Ser Asp Ser Ser Tyr Val His Arg Val Leu Pro Gly Ile
                   245                 250                 255
   acc ggc cag gac ggg atg aag agc tac gcg gcc ggc gtc cag ctc gag
   816
   Thr Gly Gln Asp Gly Met Lys Ser Tyr Ala Ala Gly Val Gln Leu Glu
               260                 265                 270
   cgc tcg gtg gcc aac cga ctc cat ttc aac ggc tcg gtg aat tac acc
   864
   Arg Ser Val Ala Asn Arg Leu His Phe Asn Gly Ser Val Asn Tyr Thr
           275                 280                 285
   gag gtt gac cca aag ctc gca tcc acc aaa gga ttc aag ggc gta gga
   912
   Glu Val Asp Pro Lys Leu Ala Ser Thr Lys Gly Phe Lys Gly Val Gly
       290                 295                 300
   ttt aac gtt tcc ggc gat tat gct ggt gat cag tac agc ctc caa ttg
   960
   Phe Asn Val Ser Gly Asp Tyr Ala Gly Asp Gln Tyr Ser Leu Gln Leu
   305                 310                 315                 320
   ctg gct tca cga tcg ccc cag cct tca ctt ctt ctg ttc gtg ggt tac
   1008
   Leu Ala Ser Arg Ser Pro Gln Pro Ser Leu Leu Leu Phe Val Gly Tyr
                   325                 330                 335
   gag att gtg aca gcg gtt tcg gcg aat gcg acg cgc cgg ctg agc gat
   1056
   Glu Ile Val Thr Ala Val Ser Ala Asn Ala Thr Arg Arg Leu Ser Asp
               340                 345                 350
   cgc att cag ata tcg ctg caa ggc agc cga acc tgg cgc gag ctc gcg
   1104
   Arg Ile Gln Ile Ser Leu Gln Gly Ser Arg Thr Trp Arg Glu Leu Ala
           355                 360                 365
   tct tcg cgg ctg ctc acc aac gtg ccg att tcc ggc aac gac aac acc
   1152
   Ser Ser Arg Leu Leu Thr Asn Val Pro Ile Ser Gly Asn Asp Asn Thr
       370                 375                 380
   tcg acg ttg ttc gcc tcc gct acc ttc cgg ccg aat cgc cgg ctg agc
   1200
   Ser Thr Leu Phe Ala Ser Ala Thr Phe Arg Pro Asn Arg Arg Leu Ser
   385                 390                 395                 400
   ttt gtg ctg ggt gcc ggc ctt cag cgg cgc acc agc aac acg cag cta
   1248
   Phe Val Leu Gly Ala Gly Leu Gln Arg Arg Thr Ser Asn Thr Gln Leu
                   405                 410                 415
   tac agt tac agc tcc aaa cgc atc aat ctc tcg acg tcg ctt tcg ctc
   1296
   Tyr Ser Tyr Ser Ser Lys Arg Ile Asn Leu Ser Thr Ser Leu Ser Leu
               420                 425                 430
   tga
   1299
    *
<210> 19
   <211> 432
   <212> PRT
   <213> Sphingomonas sp. ATCC53159
<220>
   <221> PEPTIDE
   <222> (0) ... (0)
   <223> unknown
<400> 19
   Met Lys Pro Arg Pro Gly Gly Thr Phe Met Gln Val Asn Phe Asn Arg
    1               5                  10                  15
   Gln Ala Arg Lys Leu Gly Ala Gly Asn Ala Leu Ala Arg Gly Gly Pro
               20                  25                  30
   Val Leu Ala Leu Leu Ala Thr Ala Ala Trp Thr Gln Pro Ala Leu Ala
           35                  40                  45
   Gln Arg Gln Ala Phe Glu Ser Arg Pro Ser Gly Ser Glu Arg Gln Val
       50                  55                  60
   Asp Ile Arg Ala Thr Gly Ser Leu Glu Tyr Asp Asp Asn Val Val Leu
   65                  70                  75                  80
   Asn Asp Gln Arg Ile Thr Asp Gly Ala Arg Gly Asp Val Ile Ala Ser
                   85                  90                  95
   Pro Gly Leu Asp Val Thr Leu Val Leu Pro Arg Ala Thr Gly Gln Leu
               100                 105                 110
   Tyr Leu Thr Gly Asn Val Gly Tyr Arg Phe Tyr Lys Arg Tyr Thr Asn
           115                 120                 125
   Phe Asn Arg Glu Gln Ile Ser Leu Thr Gly Gly Ala Asp Gln Arg Phe
       130                 135                 140
   Ala Ser Cys Val Val His Gly Glu Val Gly Tyr Gln Arg His Leu Thr
   145                 150                 155                 160
   Asp Leu Ser Ser Ile Leu Ile Gln Asp Thr Thr Pro Ala Leu Asn Asn
                   165                 170                 175
   Thr Glu Glu Ala Arg Gln Tyr Thr Ala Asp Ile Gly Cys Gly Ala Thr
               180                 185                 190
   Tyr Gly Leu Arg Pro Ala Val Ser Tyr Thr Arg Asn Glu Val Arg Asn
           195                 200                 205
   Ser Leu Ala Glu Arg Arg Tyr Ala Asp Ser Asn Thr Asn Thr Phe Thr
       210                 215                 220
   Ala Gln Leu Gly Leu Thr Ser Pro Ala Leu Gly Thr Val Ala Val Phe
   225                 230                 235                 240
   Gly Arg Met Ser Asp Ser Ser Tyr Val His Arg Val Leu Pro Gly Ile
                   245                 250                 255
   Thr Gly Gln Asp Gly Met Lys Ser Tyr Ala Ala Gly Val Gln Leu Glu
               260                 265                 270
   Arg Ser Val Ala Asn Arg Leu His Phe Asn Gly Ser Val Asn Tyr Thr
           275                 280                 285
   Glu Val Asp Pro Lys Leu Ala Ser Thr Lys Gly Phe Lys Gly Val Gly
       290                 295                 300
   Phe Asn Val Ser Gly Asp Tyr Ala Gly Asp Gln Tyr Ser Leu Gln Leu
   305                 310                 315                 320
   Leu Ala Ser Arg Ser Pro Gln Pro Ser Leu Leu Leu Phe Val Gly Tyr
                   325                 330                 335
   Glu Ile Val Thr Ala Val Ser Ala Asn Ala Thr Arg Arg Leu Ser Asp
               340                 345                 350
   Arg Ile Gln Ile Ser Leu Gln Gly Ser Arg Thr Trp Arg Glu Leu Ala
           355                 360                 365
   Ser Ser Arg Leu Leu Thr Asn Val Pro Ile Ser Gly Asn Asp Asn Thr
       370                 375                 380
   Ser Thr Leu Phe Ala Ser Ala Thr Phe Arg Pro Asn Arg Arg Leu Ser
   385                 390                 395                 400
   Phe Val Leu Gly Ala Gly Leu Gln Arg Arg Thr Ser Asn Thr Gln Leu
                   405                 410                 415
   Tyr Ser Tyr Ser Ser Lys Arg Ile Asn Leu Ser Thr Ser Leu Ser Leu
               420                 425                 430
<210> 20
   <211> 918
   <212> DNA
   <213> Sphingomonas sp. ATCC53159
<220>
   <221> CDS
   <222> (1)...(918)
   <223> dpsD
<400> 20
   atg cat atc aag aat cgc ttc gtg aat atc tcg acg ttg gcc atc gcc
   48
   Met His Ile Lys Asn Arg Phe Val Asn Ile Ser Thr Leu Ala Ile Ala
    1               5                  10                  15
   gcc gcg ctg gcc acg ccg gcg gcg gcg cag atc ccc acg cgg tcc gtg
   96
   Ala Ala Leu Ala Thr Pro Ala Ala Ala Gln Ile Pro Thr Arg Ser Val
               20                  25                  30
   ccc gcg ccg gcc cgc ccg cgg cct gca acg ccg ccg gcg caa cag cag
   144
   Pro Ala Pro Ala Arg Pro Arg Pro Ala Thr Pro Pro Ala Gln Gln Gln
           35                  40                  45
   aac cag gcg ccg tcg acg ccc gca gcg gca acc ccg gcg cag acc gcc
   192
   Asn Gln Ala Pro Ser Thr Pro Ala Ala Ala Thr Pro Ala Gln Thr Ala
       50                  55                  60
   gca acc gtt gcc cct gca gca acc gca ccc gca ggt tac aaa atc ggc
   240
   Ala Thr Val Ala Pro Ala Ala Thr Ala Pro Ala Gly Tyr Lys Ile Gly
    65                  70                  75                  80
   gtg gac gac gtg atc gag gcc gac gtg ctc ggc cag acc gac ttc aag
   288
   Val Asp Asp Val Ile Glu Ala Asp Val Leu Gly Gln Thr Asp Phe Lys
                    85                  90                  95
   acg cgc gcc cgt gtg cag gcg gac ggc acg gtg acc ctg ccc tat ctg
   336
   Thr Arg Ala Arg Val Gln Ala Asp Gly Thr Val Thr Leu Pro Tyr Leu
               100                 105                 110
   ggc gcc gtg cag gtc aag ggc gag acc gcg acc tcg ctc gcc gaa aag
   384
   Gly Ala Val Gln Val Lys Gly Glu Thr Ala Thr Ser Leu Ala Glu Lys
           115                 120                 125
   ctg gcc ggg ctg ctg cgc gcc ggc ggc tat tat gcc aag ccg atc gtc
   432
   Leu Ala Gly Leu Leu Arg Ala Gly Gly Tyr Tyr Ala Lys Pro Ile Val
       130                 135                 140
   agc gtc gaa atc gtc ggt ttc gtc agc aac tat gtg acg gtg ctg ggc
   480
   Ser Val Glu Ile Val Gly Phe Val Ser Asn Tyr Val Thr Val Leu Gly
   145                 150                 155                 160
   cag gtg aac agt tcc ggc ctg cag ccg gtc gac cgc ggc tat cac gtt
   528
   Gln Val Asn Ser Ser Gly Leu Gln Pro Val Asp Arg Gly Tyr His Val
                   165                 170                 175
   tcc gag atc atc gcc cgt gcc ggc ggc ctg cgc ccc gaa gcg gcc gat
   576
   Ser Glu Ile Ile Ala Arg Ala Gly Gly Leu Arg Pro Glu Ala Ala Asp
               180                 185                 190
   ttc gtc gtt ctc acc cgc gcc gat ggc tcc agc gcc aag ctg gac tac
   624
   Phe Val Val Leu Thr Arg Ala Asp Gly Ser Ser Ala Lys Leu Asp Tyr
           195                 200                 205
   aag aag ctc gcc caa ggt ggc ccc aat gac gat ccg atg gtg acg ccc
   672
   Lys Lys Leu Ala Gln Gly Gly Pro Asn Asp Asp Pro Met Val Thr Pro
       210                 215                 220
   ggg gac aag gtc ttt gtc ccg gaa gtc gag cat ttc tac att tat ggt
   720
   Gly Asp Lys Val Phe Val Pro Glu Val Glu His Phe Tyr Ile Tyr Gly
   225                 230                 235                 240
   caa att aac gcg cct ggc gta tac gcg att cga tcg gac atg acg ctc
   768
   Gln Ile Asn Ala Pro Gly Val Tyr Ala Ile Arg Ser Asp Met Thr Leu
                   245                 250                 255
   cgt cgc gcg ctg gcc cag ggc ggt ggg ctt gcc ccc gca ggc tcc gtc
   816
   Arg Arg Ala Leu Ala Gln Gly Gly Gly Leu Ala Pro Ala Gly Ser Val
               260                 265                 270
   aag cgt gtg aag gtc acg cgg gat ggc aat gaa ctc aag ttg aag ctg
   864
   Lys Arg Val Lys Val Thr Arg Asp Gly Asn Glu Leu Lys Leu Lys Leu
           275                 280                 285
   gac gat ccg att ctc cca ggc gac acg atc gtc atc ggc gaa cga ttg
   912
   Asp Asp Pro Ile Leu Pro Gly Asp Thr Ile Val Ile Gly Glu Arg Leu
       290                 295                 300
   ttc tga
   918
   Phe  *
   305
<210> 21
   <211> 305
   <212> PRT
   <213> Sphingomonas sp. ATCC53159
<220>
   <221> PEPTIDE
   <222> (0) ... (0)
   <223> putative polysaccharide export protein
<400> 21
   Met His Ile Lys Asn Arg Phe Val Asn Ile Ser Thr Leu Ala Ile Ala
    1               5                  10                  15
   Ala Ala Leu Ala Thr Pro Ala Ala Ala Gln Ile Pro Thr Arg Ser Val
               20                  25                  30
   Pro Ala Pro Ala Arg Pro Arg Pro Ala Thr Pro Pro Ala Gln Gln Gln
           35                  40                  45
   Asn Gln Ala Pro Ser Thr Pro Ala Ala Ala Thr Pro Ala Gln Thr Ala
       50                  55                  60
   Ala Thr Val Ala Pro Ala Ala Thr Ala Pro Ala Gly Tyr Lys Ile Gly
   65                  70                  75                  80
   Val Asp Asp Val Ile Glu Ala Asp Val Leu Gly Gln Thr Asp Phe Lys
                   85                  90                  95
   Thr Arg Ala Arg Val Gln Ala Asp Gly Thr Val Thr Leu Pro Tyr Leu
               100                 105                 110
   Gly Ala Val Gln Val Lys Gly Glu Thr Ala Thr Ser Leu Ala Glu Lys
           115                 120                 125
   Leu Ala Gly Leu Leu Arg Ala Gly Gly Tyr Tyr Ala Lys Pro Ile Val
       130                 135                 140
   Ser Val Glu Ile Val Gly Phe Val Ser Asn Tyr Val Thr Val Leu Gly
   145                 150                 155                 160
   Gln Val Asn Ser Ser Gly Leu Gln Pro Val Asp Arg Gly Tyr His Val
                   165                 170                 175
   Ser Glu Ile Ile Ala Arg Ala Gly Gly Leu Arg Pro Glu Ala Ala Asp
               180                 185                 190
   Phe Val Val Leu Thr Arg Ala Asp Gly Ser Ser Ala Lys Leu Asp Tyr
           195                 200                 205
   Lys Lys Leu Ala Gln Gly Gly Pro Asn Asp Asp Pro Met Val Thr Pro
       210                 215                 220
   Gly Asp Lys Val Phe Val Pro Glu Val Glu His Phe Tyr Ile Tyr Gly
   225                 230                 235                 240
   Gln Ile Asn Ala Pro Gly Val Tyr Ala Ile Arg Ser Asp Met Thr Leu
                   245                 250                 255
   Arg Arg Ala Leu Ala Gln Gly Gly Gly Leu Ala Pro Ala Gly Ser Val
               260                 265                 270
   Lys Arg Val Lys Val Thr Arg Asp Gly Asn Glu Leu Lys Leu Lys Leu
           275                 280                 285
   Asp Asp Pro Ile Leu Pro Gly Asp Thr Ile Val Ile Gly Glu Arg Leu
       290                 295                 300
   Phe
   305
<210> 22
   <211> 1347
   <212> DNA
   <213> Sphingomonas sp. ATCC53159
<220>
   <221> CDS
   <222> (1)...(1347)
   <223> dpsC
<400> 22
   gtg aat atc att cag ttc ttc cgc att ctg tgg gtg cgc cga tgg atc
   48
   Val Asn Ile Ile Gln Phe Phe Arg Ile Leu Trp Val Arg Arg Trp Ile
    1               5                  10                  15
   atc ctc ccg gcg ttt ctc gtt tgc gtt acc act gcc acc att gtg gtc
   96
   Ile Leu Pro Ala Phe Leu Val Cys Val Thr Thr Ala Thr Ile Val Val
               20                  25                  30
   cag ttt ctg ccc gaa cgc tac aag gcc act acg cgg gtg gtg ctc gac
   144
   Gln Phe Leu Pro Glu Arg Tyr Lys Ala Thr Thr Arg Val Val Leu Asp
           35                  40                  45
   acg ttt aag ccc gat ccc gtc acc gga cag gtg atg agc tcg cag ttc
   192
   Thr Phe Lys Pro Asp Pro Val Thr Gly Gln Val Met Ser Ser Gln Phe
       50                  55                  60
   atg cgc gcc tat gtc gag act cag acc cag ctg atc gag gac tat gcg
   240
   Met Arg Ala Tyr Val Glu Thr Gln Thr Gln Leu Ile Glu Asp Tyr Ala
    65                 70                  75                  80
   acc gcc ggt cgc gtg gtc gac gaa ctg ggc tgg gtg aat gat ccg gcg
   288
   Thr Ala Gly Arg Val Val Asp Glu Leu Gly Trp Val Asn Asp Pro Ala
                   85                  90                  95
   aac atc tcc gcg ttc aac aac tcg tcc gcg gct gcc acc ggc gac atc
   336
   Asn Ile Ser Ala Phe Asn Asn Ser Ser Ala Ala Ala Thr Gly Asp Ile
               100                 105                 110
   cgc cgc tgg ctc gcc aag cag atc atc gac aat acc aag gcc gat gtg
   384
   Arg Arg Trp Leu Ala Lys Gln Ile Ile Asp Asn Thr Lys Ala Asp Val
           115                 120                 125
   atg gag ggg agc aac atc ctc gaa atc acc tat tcg gac agc tcg ccc
   432
   Met Glu Gly Ser Asn Ile Leu Glu Ile Thr Tyr Ser Asp Ser Ser Pro
       130                 135                 140
   gag cgc gcc gaa cgc atc gcc aac ctg atc cgc acc tcg ttc ctc gcc
   480
   Glu Arg Ala Glu Arg Ile Ala Asn Leu Ile Arg Thr Ser Phe Leu Ala
   145                 150                 155                 160
   cag tcg ctc gcc gcc aag cgc cag gcc gcg acc aag tcg gcc gac tgg
   528
   Gln Ser Leu Ala Ala Lys Arg Gln Ala Ala Thr Lys Ser Ala Asp Trp
                   165                 170                 175
   tac gcc cag cag gcc gaa gct gcc cgc gat tcg ctc gct gcg gcg gtc
   576
   Tyr Ala Gln Gln Ala Glu Ala Ala Arg Asp Ser Leu Ala Ala Ala Val
               180                 185                 190
   cag gcc cgc acc gat ttc gtg aag aag acc ggc atc gtg ctg acc gaa
   624
   Gln Ala Arg Thr Asp Phe Val Lys Lys Thr Gly Ile Val Leu Thr Glu
           195                 200                 205
   acc ggc gcc gac ctg gaa acc cag aag ctc cag cag atc gag ggg cag
   672
   Thr Gly Ala Asp Leu Glu Thr Gln Lys Leu Gln Gln Ile Glu Gly Gln
       210                 215                 220
   acg acg acc gcc acc gcc ccg gtt gcc atg gcc ccc agc ggc atg ggc
   720
   Thr Thr Thr Ala Thr Ala Pro Val Ala Met Ala Pro Ser Gly Met Gly
   225                 230                 235                 240
   ccg gcg cag atg cag ctc gcc cag atc gac cag cag atc cag cag gca
   768
   Pro Ala Gln Met Gln Leu Ala Gln Ile Asp Gln Gln Ile Gln Gln Ala
                   245                 250                 255
   gcg acc agc cta ggt ccg aac cac cca act ttc cag gcc ttg cag cgg
   816
   Ala Thr Ser Leu Gly Pro Asn His Pro Thr Phe Gln Ala Leu Gln Arg
               260                 265                 270
   cag cgc gaa gtg ttc gcc aag gca gcg gcg gcg gaa cgc gcg cag gcg
   864
   Gln Arg Glu Val Phe Ala Lys Ala Ala Ala Ala Glu Arg Ala Gln Ala
           275                 280                 285
   aac ggc gta tcc ggt ccg gca cgc ggg gcc atc gaa agc gca gcc aac
   912
   Asn Gly Val Ser Gly Pro Ala Arg Gly Ala Ile Glu Ser Ala Ala Asn
       290                 295                 300
   gcc cag cgc gcg cgg gtt ctc ggc aat cgt cag gat gtc gac aag ctt
   960
   Ala Gln Arg Ala Arg Val Leu Gly Asn Arg Gln Asp Val Asp Lys Leu
   305                 310                 315                 320
   acg cag ctg cag cgt gac gtc tcg ctg aag cag gat cag tac atg aag
   1008
   Thr Gln Leu Gln Arg Asp Val Ser Leu Lys Gln Asp Gln Tyr Met Lys
                   325                 330                 335
   gcg gca cag cgc gtc gcc gat ctg cgg ctg gaa gca agc agc aac gat
   1056
   Ala Ala Gln Arg Val Ala Asp Leu Arg Leu Glu Ala Ser Ser Asn Asp
               340                 345                 350
   gtc ggc atg tcg acg ctc agc gaa gca tcg gcg ccg gaa acg ccc tat
   1104
   Val Gly Met Ser Thr Leu Ser Glu Ala Ser Ala Pro Glu Thr Pro Tyr
           355                 360                 365
   tac ccc aag gtg ccg ctc atc atc ggt ggt gca gcc ggc ttc ggc ctc
   1152
   Tyr Pro Lys Val Pro Leu Ile Ile Gly Gly Ala Ala Gly Phe Gly Leu
       370                 375                 380
   ggg ctc ggt ctg ctg gtc gcg ctg ctc gtc gag ctg ctc ggc cgc cgc
   1200
   Gly Leu Gly Leu Leu Val Ala Leu Leu Val Glu Leu Leu Gly Arg Arg
   385                 390                 395                 400
   gtc cgc agc ccc gag gat ctg gaa gtt gcg atc gat gca ccg gtg ctg
   1248
   Val Arg Ser Pro Glu Asp Leu Glu Val Ala Ile Asp Ala Pro Val Leu
                   405                 410                 415
   ggc gtg atc cag agc cgc gcc tcg ctt gcc gcc cgc ctt cgc cgc gcc
   1296
   Gly Val Ile Gln Ser Arg Ala Ser Leu Ala Ala Arg Leu Arg Arg Ala
               420                 425                 430
   caa gaa acc ctc ggc gaa ggt gcc gac acg cac gga gct tca gta aac
   1344
   Gln Glu Thr Leu Gly Glu Gly Ala Asp Thr His Gly Ala Ser Val Asn
           435                 440                 445
   tga
   1347
    *
<210> 23
   <211> 448
   <212> PRT
   <213> Sphingomonas sp. ATCC53159
<220>
   <221> PEPTIDE
   <222> (0)...(0)
   <223> putative polysaccharide export protein
<400> 23
   Val Asn Ile Ile Gln Phe Phe Arg Ile Leu Trp Val Arg Arg Trp Ile
    1               5                  10                  15
   Ile Leu Pro Ala Phe Leu Val Cys Val Thr Thr Ala Thr Ile Val Val
               20                  25                  30
   Gln Phe Leu Pro Glu Arg Tyr Lys Ala Thr Thr Arg Val Val Leu Asp
           35                  40                  45
   Thr Phe Lys Pro Asp Pro Val Thr Gly Gln Val Met Ser Ser Gln Phe
       50                  55                  60
   Met Arg Ala Tyr Val Glu Thr Gln Thr Gln Leu Ile Glu Asp Tyr Ala
   65                  70                  75                  80
   Thr Ala Gly Arg Val Val Asp Glu Leu Gly Trp Val Asn Asp Pro Ala
                   85                  90                  95
   Asn Ile Ser Ala Phe Asn Asn Ser Ser Ala Ala Ala Thr Gly Asp Ile
               100                 105                 110
   Arg Arg Trp Leu Ala Lys Gln Ile Ile Asp Asn Thr Lys Ala Asp Val
           115                 120                 125
   Met Glu Gly Ser Asn Ile Leu Glu Ile Thr Tyr Ser Asp Ser Ser Pro
       130                 135                 140
   Glu Arg Ala Glu Arg Ile Ala Asn Leu Ile Arg Thr Ser Phe Leu Ala
   145                 150                 155                 160
   Gln Ser Leu Ala Ala Lys Arg Gln Ala Ala Thr Lys Ser Ala Asp Trp
                   165                 170                 175
   Tyr Ala Gln Gln Ala Glu Ala Ala Arg Asp Ser Leu Ala Ala Ala Val
               180                 185                 190
   Gln Ala Arg Thr Asp Phe Val Lys Lys Thr Gly Ile Val Leu Thr Glu
           195                 200                 205
   Thr Gly Ala Asp Leu Glu Thr Gln Lys Leu Gln Gln Ile Glu Gly Gln
       210                 215                 220
   Thr Thr Thr Ala Thr Ala Pro Val Ala Met Ala Pro Ser Gly Met Gly
   225                 230                 235                 240
   Pro Ala Gln Met Gln Leu Ala Gln Ile Asp Gln Gln Ile Gln Gln Ala
                   245                 250                 255
   Ala Thr Ser Leu Gly Pro Asn His Pro Thr Phe Gln Ala Leu Gln Arg
               260                 265                 270
   Gln Arg Glu Val Phe Ala Lys Ala Ala Ala Ala Glu Arg Ala Gln Ala
           275                 280                 285
   Asn Gly Val Ser Gly Pro Ala Arg Gly Ala Ile Glu Ser Ala Ala Asn
       290                 295                 300
   Ala Gln Arg Ala Arg Val Leu Gly Asn Arg Gln Asp Val Asp Lys Leu
   305                 310                 315                 320
   Thr Gln Leu Gln Arg Asp Val Ser Leu Lys Gln Asp Gln Tyr Met Lys
                   325                 330                 335
   Ala Ala Gln Arg Val Ala Asp Leu Arg Leu Glu Ala Ser Ser Asn Asp
               340                 345                 350
   Val Gly Met Ser Thr Leu Ser Glu Ala Ser Ala Pro Glu Thr Pro Tyr
           355                 360                 365
   Tyr Pro Lys Val Pro Leu Ile Ile Gly Gly Ala Ala Gly Phe Gly Leu
       370                 375                 380
   Gly Leu Gly Leu Leu Val Ala Leu Leu Val Glu Leu Leu Gly Arg Arg
   385                 390                 395                 400
   Val Arg Ser Pro Glu Asp Leu Glu Val Ala Ile Asp Ala Pro Val Leu
                   405                 410                 415
   Gly Val Ile Gln Ser Arg Ala Ser Leu Ala Ala Arg Leu Arg Arg Ala
               420                 425                 430
   Gln Glu Thr Leu Gly Glu Gly Ala Asp Thr His Gly Ala Ser Val Asn
           435                 440                 445
<210> 24
   <211> 708
   <212> DNA
   <213> Sphingomonas sp. ATCC53159
<220>
   <221> CDS
   <222> (1)...(708)
   <223> dpsE
<400> 24
   atg gac gcg atg acc agc gaa ccg ctg ccc gaa ggc gat cgt ccg agc
   48
   Met Asp Ala Met Thr Ser Glu Pro Leu Pro Glu Gly Asp Arg Pro Ser
    1               5                  10                  15
   gcc gtg ccg acc acg ccg gat acg atc ggc atg ctc gaa tac cag ctc
   96
   Ala Val Pro Thr Thr Pro Asp Thr Ile Gly Met Leu Glu Tyr Gln Leu
               20                  25                  30
   gtc ctc tcc gat ccg acc ggg atc gag gcg gaa gcg atc cgc gcg cta
   144
   Val Leu Ser Asp Pro Thr Gly Ile Glu Ala Glu Ala Ile Arg Ala Leu
           35                  40                  45
   cgc acg cgc atc atg acc cag cac ctc cgc gag ggc cgg cgc gcg ctc
   192
   Arg Thr Arg Ile Met Thr Gln His Leu Arg Glu Gly Arg Arg Ala Leu
       50                  55                  60
   gcg atc tgc gcc gcc tcg gcg gga tcc ggc tgc agc ttc acc gcc gtc
   240
   Ala Ile Cys Ala Ala Ser Ala Gly Ser Gly Cys Ser Phe Thr Ala Val
    65                  70                  75                  80
   aat ctg gcg acg gcg ctg gcg cag atc ggc gtt aag act gcg ctg gtc
   288
   Asn Leu Ala Thr Ala Leu Ala Gln Ile Gly Val Lys Thr Ala Leu Val
                   85                  90                  95
   gat gcc aat ctg cgc gat ccc agc atc ggc gca gcc ttc ggc ctc gcc
   336
   Asp Ala Asn Leu Arg Asp Pro Ser Ile Gly Ala Ala Phe Gly Leu Ala
               100                 105                 110
   gcc gac aag ccc ggc ctg gcc gat tat ctc gcc tcg ggc gat gtc gac
   384
   Ala Asp Lys Pro Gly Leu Ala Asp Tyr Leu Ala Ser Gly Asp Val Asp
           115                 120                 125
   ctc gcc tcg atc atc cat gcg acc cgc ctc gac cag ctc tcg atc atc
   432
   Leu Ala Ser Ile Ile His Ala Thr Arg Leu Asp Gln Leu Ser Ile Ile
       130                 135                 140
   ccg gcc ggg cat gtc gag cac agc ccg cag gaa ctg ctc gcg tcc gaa
   480
   Pro Ala Gly His Val Glu His Ser Pro Gln Glu Leu Leu Ala Ser Glu
   145                 150                 155                 160
   cag ttc cat gat ctg gcg acg cag ctg ctg cgc gag ttc gac atc acg
   528
   Gln Phe His Asp Leu Ala Thr Gln Leu Leu Arg Glu Phe Asp Ile Thr
                   165                 170                 175
   atc ttc gac acc acg gcg tcc aac acc tgc gcc gac gcg cag cgt gtc
   576
   Ile Phe Asp Thr Thr Ala Ser Asn Thr Cys Ala Asp Ala Gln Arg Val
               180                 185                     190
   gcg cat atc gcc ggc tat gcg atc atc gtg gcg cgc aag gat gcg agc
   624
   Ala His Ile Ala Gly Tyr Ala Ile Ile Val Ala Arg Lys Asp Ala Ser
           195                 200                     205
   tac atc cgc gac gtg aac acg ctc agc cgc acg ctg cgt gca gac cgc
   672
   Tyr Ile Arg Asp Val Asn Thr Leu Ser Arg Thr Leu Arg Ala Asp Arg
       210                 215                 220
   acc aac gtc atc ggc tgc gta ctg aac ggc tat tga
   708
   Thr Asn Val Ile Gly Cys Val Leu Asn Gly Tyr  *
   225                 230                 235
<210> 25
   <211> 235
   <212> PRT
   <213> Sphingomonas sp. ATCC53159
<220>
   <221> PEPTIDE
   <222> (0) ... (0)
   <223> putative polysaccharide export protein
<400> 25
   Met Asp Ala Met Thr Ser Glu Pro Leu Pro Glu Gly Asp Arg Pro Ser
    1               5                  10                  15
   Ala Val Pro Thr Thr Pro Asp Thr Ile Gly Met Leu Glu Tyr Gln Leu
               20                  25                  30
   Val Leu Ser Asp Pro Thr Gly Ile Glu Ala Glu Ala Ile Arg Ala Leu
           35                  40                  45
   Arg Thr Arg Ile Met Thr Gln His Leu Arg Glu Gly Arg Arg Ala Leu
       50                  55                  60
   Ala Ile Cys Ala Ala Ser Ala Gly Ser Gly Cys Ser Phe Thr Ala Val
   65                  70                  75                  80
   Asn Leu Ala Thr Ala Leu Ala Gln Ile Gly Val Lys Thr Ala Leu Val
                   85                  90                  95
   Asp Ala Asn Leu Arg Asp Pro Ser Ile Gly Ala Ala Phe Gly Leu Ala
               100                 105                 110
   Ala Asp Lys Pro Gly Leu Ala Asp Tyr Leu Ala Ser Gly Asp Val Asp
           115                 120                 125
   Leu Ala Ser Ile Ile His Ala Thr Arg Leu Asp Gln Leu Ser Ile Ile
       130                 135                 140
   Pro Ala Gly His Val Glu His Ser Pro Gln Glu Leu Leu Ala Ser Glu
   145                 150                 155                 160
   Gln Phe His Asp Leu Ala Thr Gln Leu Leu Arg Glu Phe Asp Ile Thr
                   165                 170                 175
   Ile Phe Asp Thr Thr Ala Ser Asn Thr Cys Ala Asp Ala Gln Arg Val
               180                 185                 190
   Ala His Ile Ala Gly Tyr Ala Ile Ile Val Ala Arg Lys Asp Ala Ser
           195                 200                 205
   Tyr Ile Arg Asp Val Asn Thr Leu Ser Arg Thr Leu Arg Ala Asp Arg
       210                 215                 220
   Thr Asn Val Ile Gly Cys Val Leu Asn Gly Tyr
   225                 230                 235
<210> 26
   <211> 882
   <212> DNA
   <213> Sphingomonas sp. ATCC53159
<220>
   <221> CDS
   <222> (1)...(882)
   <223> dpsM
<400> 26
   atg gca gcg acc gcg atg acg cgg cag cag gag agg aag ggc ggt ggc
   48
   Met Ala Ala Thr Ala Met Thr Arg Gln Gln Glu Arg Lys Gly Gly Gly
    1               5                  10                  15
   tat tgg ctg gcc gtt gcc ggt ctt gcc gcg cta acc atc ccg acc ttc
   96
   Tyr Trp Leu Ala Val Ala Gly Leu Ala Ala Leu Thr Ile Pro Thr Phe
               20                  25                  30
   atc acc ctg ggt cgc gag gtt tgg agt gcg gaa ggc ggc gtg cag ggt
   144
   Ile Thr Leu Gly Arg Glu Val Trp Ser Ala Glu Gly Gly Val Gln Gly
           35                  40                  45
   ccg atc gtg ctc gcc acg ggc gcc tgg atg ctg gcc cgc cag tgc tcg
   192
   Pro Ile Val Leu Ala Thr Gly Ala Trp Met Leu Ala Arg Gln Cys Ser
       50                  55                  60
   acg atc gag gcg cta cgc cgc ccc ggc agc gtg ctg ctc ggc gcg ctg
   240
   Thr Ile Glu Ala Leu Arg Arg Pro Gly Ser Val Leu Leu Gly Ala Leu
    65                  70                  75                  80
   ttc ctg ctg gcg acg ctt gcc ttc tac acc gtt gga cgg gtg ttc gac
   288
   Phe Leu Leu Ala Thr Leu Ala Phe Tyr Thr Val Gly Arg Val Phe Asp
                    85                  90                  95
   ttc atc agt gtc gaa acc ttc gga ctg gtc gcg acc tat ctg gtc gtc
   336
   Phe Ile Ser Val Glu Thr Phe Gly Leu Val Ala Thr Tyr Leu Val Val
               100                 105                 110
   gcc tat ctc tat ttc ggt gcc agg gtg ctc cgt gcc gcc tgg ttc ccg
   384
   Ala Tyr Leu Tyr Phe Gly Ala Arg Val Leu Arg Ala Ala Trp Phe Pro
           115                 120                 125
   gtg ctg tgg ctg ttc ttc ctg gtg ccg ccg ccc ggc tgg gcc gtc gac
   432
   Val Leu Trp Leu Phe Phe Leu Val Pro Pro Pro Gly Trp Ala Val Asp
       130                 135                 140
   cgc atc acc gca ccg ctc aag gag ttc gtc tcc tat gcg gca acg ggc
   480
   Arg Ile Thr Ala Pro Leu Lys Glu Phe Val Ser Tyr Ala Ala Thr Gly
   145                 150                 155                 160
   ctg ctt tcc tgg gtg gat tat ccg atc ctg cgc cag ggc gtg aca ctg
   528
   Leu Leu Ser Trp Val Asp Tyr Pro Ile Leu Arg Gln Gly Val Thr Leu
                   165                 170                 175
   ttc gtc ggc ccc tat cag ctg ctc gtc gaa gat gcc tgt tcg ggt ctg
   576
   Phe Val Gly Pro Tyr Gln Leu Leu Val Glu Asp Ala Cys Ser Gly Leu
               180                 185                 190
   cgc tcg ctg tcc agc ctg gtc gtc gtg acg ctg ctc tac atc tac atc
   624
   Arg Ser Leu Ser Ser Leu Val Val Val Thr Leu Leu Tyr Ile Tyr Ile
           195                 200                 205
   aag aac aag ccg tcc tgg cgc tac gcg gcg ttc atc gca gcg ctg gtg
   672
   Lys Asn Lys Pro Ser Trp Arg Tyr Ala Ala Phe Ile Ala Ala Leu Val
       210                 215                 220
   atc ccg gtg gca gtg gtg acc aac gtc ctg cgg atc atc atc ctg gta
   720
   Ile Pro Val Ala Val Val Thr Asn Val Leu Arg Ile Ile Ile Leu Val
   225                 230                 235                 240
   ctg atc acc tat cat ctg ggc gac gag gcg gcg cag agc ttc ctc cac
   768
   Leu Ile Thr Tyr His Leu Gly Asp Glu Ala Ala Gln Ser Phe Leu His
                   245                 250                 255
   gtc tcc acc ggc atg gtg atg ttc gtg gtc gcc ctg ctt tgc atc ttc
   816
   Val Ser Thr Gly Met Val Met Phe Val Val Ala Leu Leu Cys Ile Phe
               260                 265                 270
   gcg atc gac tgg gtg gtc gag caa ctt ctt ctc ctg cgt cgg agg cat
   864
   Ala Ile Asp Trp Val Val Glu Gln Leu Leu Leu Leu Arg Arg Arg His
           275                 280                 285
   cat gtt caa ccg gcg tga
   882
   His Val Gln Pro Ala  *
       290
<210> 27
   <211> 293
   <212> PRT
   <213> Sphingomonas sp. ATCC53159
<220>
   <221> PEPTIDE
   <222> (0)...(0)
   <223> putative polysaccharide export protein
<400> 27
   Met Ala Ala Thr Ala Met Thr Arg Gln Gln Glu Arg Lys Gly Gly Gly
    1               5                  10                  15
   Tyr Trp Leu Ala Val Ala Gly Leu Ala Ala Leu Thr Ile Pro Thr Phe
               20                  25                  30
   Ile Thr Leu Gly Arg Glu Val Trp Ser Ala Glu Gly Gly Val Gln Gly
           35                  40                  45
   Pro Ile Val Leu Ala Thr Gly Ala Trp Met Leu Ala Arg Gln Cys Ser
       50                  55                  60
   Thr Ile Glu Ala Leu Arg Arg Pro Gly Ser Val Leu Leu Gly Ala Leu
   65                  70                  75                  80
   Phe Leu Leu Ala Thr Leu Ala Phe Tyr Thr Val Gly Arg Val Phe Asp
                   85                  90                  95
   Phe Ile Ser Val Glu Thr Phe Gly Leu Val Ala Thr Tyr Leu Val Val
               100                 105                 110
   Ala Tyr Leu Tyr Phe Gly Ala Arg Val Leu Arg Ala Ala Trp Phe Pro
           115                 120                 125
   Val Leu Trp Leu Phe Phe Leu Val Pro Pro Pro Gly Trp Ala Val Asp
       130                 135                 140
   Arg Ile Thr Ala Pro Leu Lys Glu Phe Val Ser Tyr Ala Ala Thr Gly
   145                 150                 155                 160
   Leu Leu Ser Trp Val Asp Tyr Pro Ile Leu Arg Gln Gly Val Thr Leu
                   165                 170                 175
   Phe Val Gly Pro Tyr Gln Leu Leu Val Glu Asp Ala Cys Ser Gly Leu
               180                 185                 190
   Arg Ser Leu Ser Ser Leu Val Val Val Thr Leu Leu Tyr Ile Tyr Ile
           195                 200                 205
   Lys Asn Lys Pro Ser Trp Arg Tyr Ala Ala Phe Ile Ala Ala Leu Val
       210                 215                 220
   Ile Pro Val Ala Val Val Thr Asn Val Leu Arg Ile Ile Ile Leu Val
   225                 230                 235                 240
   Leu Ile Thr Tyr His Leu Gly Asp Glu Ala Ala Gln Ser Phe Leu His
                   245                 250                 255
   Val Ser Thr Gly Met Val Met Phe Val Val Ala Leu Leu Cys Ile Phe
               260                 265                 270
   Ala Ile Asp Trp Val Val Glu Gln Leu Leu Leu Leu Arg Arg Arg His
           275                 280                 285
   His Val Gln Pro Ala
       290
<210> 28
   <211> 699
   <212> DNA
   <213> Sphingomonas sp. ATCC53159
<220>
   <221> CDS
   <222> (1) ... (699)
   <223> dpsN
<400> 28
   atg ttc aac cgg cgt gac ctg ctg atc ggc gca ggc tgc ttc gcc gcc
   48
   Met Phe Asn Arg Arg Asp Leu Leu Ile Gly Ala Gly Cys Phe Ala Ala
    1               5                  10                  15
   gct ggc gcc tcg ctc ggc ctg aag ccg cac cgg cgg atg gac ctg ctg
   96
   Ala Gly Ala Ser Leu Gly Leu Lys Pro His Arg Arg Met Asp Leu Leu
               20                  25                  30
   ggc ggc acc aag ctc gac acg ctg atg ccc aag gca ttc ggc gca tgg
   144
   Gly Gly Thr Lys Leu Asp Thr Leu Met Pro Lys Ala Phe Gly Ala Trp
           35                  40                  45
   aag gca gag gat acc ggt tcg ctg atc gcg ccg gcg cgc gaa ggc agc
   192
   Lys Ala Glu Asp Thr Gly Ser Leu Ile Ala Pro Ala Arg Glu Gly Ser
       50                  55                  60
   ctg gag gac aag ctc tac aac cag gtg gtc acc cgc gcc ttc tcc cgc
   240
   Leu Glu Asp Lys Leu Tyr Asn Gln Val Val Thr Arg Ala Phe Ser Arg
    65                  70                  75                  80
   gcg gac ggt gcc caa gtg atg ctg ctg atc gcc tat ggc aac gcc cag
   288
   Ala Asp Gly Ala Gln Val Met Leu Leu Ile Ala Tyr Gly Asn Ala Gln
                   85                  90                  95
   acc gat cta ctg cag ctg cac cgg ccg gaa ata tgc tac ccg ttc ttc
   336
   Thr Asp Leu Leu Gln Leu His Arg Pro Glu Ile Cys Tyr Pro Phe Phe
               100                 105                 110
   ggc ttc acc gtg gtg gaa agc cat gag cag acc atc ccg gtg acg ccg
   384
   Gly Phe Thr Val Val Glu Ser His Glu Gln Thr Ile Pro Val Thr Pro
           115                 120                 125
   cag gtg acg atc ccc ggt cgc gcg ctg acc gcc acc aac ttc aac cgc
   432
   Gln Val Thr Ile Pro Gly Arg Ala Leu Thr Ala Thr Asn Phe Asn Arg
       130                 135                 140
   acc gag cag atc ctc tac tgg acc cgc gtc ggc gaa tat ctg ccg cag
   480
   Thr Glu Gln Ile Leu Tyr Trp Thr Arg Val Gly Glu Tyr Leu Pro Gln
   145                 150                 155                 160
   aac ggc aat cag cag atg ctc gcg cgg ctg aag agc cag gtc cag ggc
   528
   Asn Gly Asn Gln Gln Met Leu Ala Arg Leu Lys Ser Gln Val Gln Gly
                   165                 170                 175
   tgg atc gtc gac ggt gtg ctg gtg cgc atc tcg acg gtg acg ccc gag
   576
   Trp Ile Val Asp Gly Val Leu Val Arg Ile Ser Thr Val Thr Pro Glu
               180                 185                 190
   gcg gaa gat ggc ctg agc gcc aat ctc gat ttc gcg cgc gag ctg gtg
   624
   Ala Glu Asp Gly Leu Ser Ala Asn Leu Asp Phe Ala Arg Glu Leu Val
           195                 200                 205
   aag acg ctc gac ccg cgc gtg ctg cgc ccg ctg ctc ggg aac ggg ctc
   672
   Lys Thr Leu Asp Pro Arg Val Leu Arg Pro Leu Leu Gly Asn Gly Leu
       210                 215                 220
   aca cgg cag ctc ggt cac cag gtc tga
   699
   Thr Arg Gln Leu Gly His Gln Val  *
   225                 230
<210> 29
   <211> 232
   <212> PRT
   <213> Sphingomonas sp. ATCC53159
<220>
   <221> PEPTIDE
   <222> (0)...(0)
   <223> putative polysaccharide export protein
<400> 29
   Met Phe Asn Arg Arg Asp Leu Leu Ile Gly Ala Gly Cys Phe Ala Ala
    1               5                  10                  15
   Ala Gly Ala Ser Leu Gly Leu Lys Pro His Arg Arg Met Asp Leu Leu
               20                  25                  30
   Gly Gly Thr Lys Leu Asp Thr Leu Met Pro Lys Ala Phe Gly Ala Trp
           35                  40                  45
   Lys Ala Glu Asp Thr Gly Ser Leu Ile Ala Pro Ala Arg Glu Gly Ser
       50                  55                  60
   Leu Glu Asp Lys Leu Tyr Asn Gln Val Val Thr Arg Ala Phe Ser Arg
   65                  70                  75                  80
   Ala Asp Gly Ala Gln Val Met Leu Leu Ile Ala Tyr Gly Asn Ala Gln
                   85                  90                  95
   Thr Asp Leu Leu Gln Leu His Arg Pro Glu Ile Cys Tyr Pro Phe Phe
               100                 105                 110
   Gly Phe Thr Val Val Glu Ser His Glu Gln Thr Ile Pro Val Thr Pro
           115                 120                 125
   Gln Val Thr Ile Pro Gly Arg Ala Leu Thr Ala Thr Asn Phe Asn Arg
       130                 135                 140
   Thr Glu Gln Ile Leu Tyr Trp Thr Arg Val Gly Glu Tyr Leu Pro Gln
   145                 150                 155                 160
   Asn Gly Asn Gln Gln Met Leu Ala Arg Leu Lys Ser Gln Val Gln Gly
                   165                 170                 175
   Trp Ile Val Asp Gly Val Leu Val Arg Ile Ser Thr Val Thr Pro Glu
               180                 185                 190
   Ala Glu Asp Gly Leu Ser Ala Asn Leu Asp Phe Ala Arg Glu Leu Val
           195                 200                 205
   Lys Thr Leu Asp Pro Arg Val Leu Arg Pro Leu Leu Gly Asn Gly Leu
       210                 215                 220
   Thr Arg Gln Leu Gly His Gln Val
   225                 230
<210> 30
   <211> 1395
   <212> DNA
   <213> Sphingomonas sp. ATCC53159
<220>
   <221> CDS
   <222> (1)...(1395)
   <223> atrD
<400> 30
   atg aac gcc gtt gtt ccg atg cgc cgc ggc ggc ccg ctc gcc cgc atg
   48
   Met Asn Ala Val Val Pro Met Arg Arg Gly Gly Pro Leu Ala Arg Met
    1               5                  10                  15
   cgc gat acc gtg ctg cct gcc cgc gtc gac gct tat gac acc gcc ttc
   96
   Arg Asp Thr Val Leu Pro Ala Arg Val Asp Ala Tyr Asp Thr Ala Phe
               20                  25                  30
   ctg cct gcc gcg ctg gag atc atc gag cgg ccg gtt tcg ccc acc gcg
   144
   Leu Pro Ala Ala Leu Glu Ile Ile Glu Arg Pro Val Ser Pro Thr Ala
           35                  40                  45
   cgg ctt acc gcc aag gtg atg ctg gcc ggg ctg gcg atc acc gcc gcc
   192
   Arg Leu Thr Ala Lys Val Met Leu Ala Gly Leu Ala Ile Thr Ala Ala
       50                  55                  60
   tgg ctg gcg atc ggc aag gtc gaa gtc gtc gcg ccg acg cag ggg cgg
   240
   Trp Leu Ala Ile Gly Lys Val Glu Val Val Ala Pro Thr Gln Gly Arg
    65                  70                  75                  80
   atc gcg ccg atc ggc gag acc aag atc gtc cag tcg ccc gaa tcg ggg
   288
   Ile Ala Pro Ile Gly Glu Thr Lys Ile Val Gln Ser Pro Glu Ser Gly
                   85                  90                  95
   atc gtc cgc cgc atc ctg gtg ggc gag ggg cag aag gtc gcc aag ggc
   336
   Ile Val Arg Arg Ile Leu Val Gly Glu Gly Gln Lys Val Ala Lys Gly
               100                 105                 110
   cag gtg ctg atc acg ctc gac ccg acc gtg tcg tcg gcg gag gcg gca
   384
   Gln Val Leu Ile Thr Leu Asp Pro Thr Val Ser Ser Ala Glu Ala Ala
           115                 120                 125
   cag gcg aag gtg gcg ctg ctc agc gcc cag ctc gac gcc gca cgc aac
   432
   Gln Ala Lys Val Ala Leu Leu Ser Ala Gln Leu Asp Ala Ala Arg Asn
       130                 135                 140
   cag gcg atc atc gac gcg ctg gac ggc agg ggc ttc cgc ttc gtc gcg
   480
   Gln Ala Ile Ile Asp Ala Leu Asp Gly Arg Gly Phe Arg Phe Val Ala
   145                 150                 155                 160
   cct gcc gcc gcc agc ccg ggc gaa gtg gcg acg cat cgc ggc ctc gcc
   528
   Pro Ala Ala Ala Ser Pro Gly Glu Val Ala Thr His Arg Gly Leu Ala
                   165                 170                 175
   cgc gcc cgg ctg ggc cag atc gag gcg gcg ctg gcc ggc ggc cgc tcc
   576
   Arg Ala Arg Leu Gly Gln Ile Glu Ala Ala Leu Ala Gly Gly Arg Ser
               180                 185                 190
   gat cgc ggt gcc gcc gtc tcg gcc gcg gcc gag gcg cag gca cag gtg
   624
   Asp Arg Gly Ala Ala Val Ser Ala Ala Ala Glu Ala Gln Ala Gln Val
           195                 200                 205
   cgg aag ctc gaa cag tcg ctg ccg ctg ctc gaa cag cag atc gcc gcg
   672
   Arg Lys Leu Glu Gln Ser Leu Pro Leu Leu Glu Gln Gln Ile Ala Ala
       210                 215                 220
   aac gag acg atg gcc gcc aag ggc tat gtc tcg aag ctg cgc gtc gtg
   720
   Asn Glu Thr Met Ala Ala Lys Gly Tyr Val Ser Lys Leu Arg Val Val
   225                 230                 235                 240
   gag atg cgt cgc cag ctg atc gcc gag cgg cag gac ctg acg gcg gcg
   768
   Glu Met Arg Arg Gln Leu Ile Ala Glu Arg Gln Asp Leu Thr Ala Ala
                   245                 250                 255
   cgc gct acg ctc gcc aaa ctc ggc cag cag tcg ctg agc gtc tcc agc
   816
   Arg Ala Thr Leu Ala Lys Leu Gly Gln Gln Ser Leu Ser Val Ser Ser
               260                 265                 270
   ctg tcg gcc aag acg cgc gag gag gcg cgg gcg cag gtg ctg cag gat
   864
   Leu Ser Ala Lys Thr Arg Glu Glu Ala Arg Ala Gln Val Leu Gln Asp
           275                 280                 285
   ctg gtc aag gcg cag gac gag gtg cgt gcc cgc ggc gag gac gtc gcc
   912
   Leu Val Lys Ala Gln Asp Glu Val Arg Ala Arg Gly Glu Asp Val Ala
       290                 295                 300
   aag gcg aat ctg cgc agc tcg ttc cgc gaa ctg cgc gcg ccg gtg agc
   960
   Lys Ala Asn Leu Arg Ser Ser Phe Arg Glu Leu Arg Ala Pro Val Ser
   305                 310                 315                 320
   ggt acc gtc tcg cag ctg cag gtc cac acc gaa ggc ggc gtg gtg gaa
   1008
   Gly Thr Val Ser Gln Leu Gln Val His Thr Glu Gly Gly Val Val Glu
                   325                 330                 335
   ggg gcc aag ccg ctc ctc agc ctg gtt ccc gac aat gcc cgg ctc gag
   1056
   Gly Ala Lys Pro Leu Leu Ser Leu Val Pro Asp Asn Ala Arg Leu Glu
               340                 345                 350
   gcc gag gtg atg gtc gac aac agc gac atc ggc ttc gtc cac atc ggc
   1104
   Ala Glu Val Met Val Asp Asn Ser Asp Ile Gly Phe Val His Ile Gly
           355                 360                 365
   atg ccg gta aag gtg aag ctg cag gcc ttt ccc tat acc cgc tac ggc
   1152
   Met Pro Val Lys Val Lys Leu Gln Ala Phe Pro Tyr Thr Arg Tyr Gly
       370                 375                 380
   atg att ccc ggc acg gtg gcg ggc atc agc ccc gag gcg gtg cag atg
   1200
   Met Ile Pro Gly Thr Val Ala Gly Ile Ser Pro Glu Ala Val Gln Met
   385                 390                 395                 400
   aag gag aac cag ccg ccg gtc tac aag gcg cgg atc gcg ctg gcg cgc
   1248
   Lys Glu Asn Gln Pro Pro Val Tyr Lys Ala Arg Ile Ala Leu Ala Arg
                   405                 410                 415
   ggg tat gtg ctg gcc cat ggc gca cag gtg ccg ctg cgg ccg ggg atg
   1296
   Gly Tyr Val Leu Ala His Gly Ala Gln Val Pro Leu Arg Pro Gly Met
               420                 425                 430
   ctc gcg agc gcg gac atc gtc acc ggc aag cga acc ctg ttc agc tat
   1344
   Leu Ala Ser Ala Asp Ile Val Thr Gly Lys Arg Thr Leu Phe Ser Tyr
           435                 440                 445
   ctg gtg ggg ccc gtg ctc gag acg ggg agt gac gcg ctg cac gag cgg
   1392
   Leu Val Gly Pro Val Leu Glu Thr Gly Ser Asp Ala Leu His Glu Arg
       450                 455                 460
   tga
   1395
    *
<210> 31
   <211> 464
   <212> PRT
   <213> Sphingomonas sp. ATCC53159
<220>
   <221> PEPTIDE
   <222> (0) ... (0)
   <223> putative secretion protein
<400> 31
   Met Asn Ala Val Val Pro Met Arg Arg Gly Gly Pro Leu Ala Arg Met
    1               5                  10                  15
   Arg Asp Thr Val Leu Pro Ala Arg Val Asp Ala Tyr Asp Thr Ala Phe
               20                  25                  30
   Leu Pro Ala Ala Leu Glu Ile Ile Glu Arg Pro Val Ser Pro Thr Ala
           35                  40                  45
   Arg Leu Thr Ala Lys Val Met Leu Ala Gly Leu Ala Ile Thr Ala Ala
       50                  55                  60
   Trp Leu Ala Ile Gly Lys Val Glu Val Val Ala Pro Thr Gln Gly Arg
   65                  70                  75                  80
   Ile Ala Pro Ile Gly Glu Thr Lys Ile Val Gln Ser Pro Glu Ser Gly
                   85                  90                  95
   Ile Val Arg Arg Ile Leu Val Gly Glu Gly Gln Lys Val Ala Lys Gly
               100                 105                 110
   Gln Val Leu Ile Thr Leu Asp Pro Thr Val Ser Ser Ala Glu Ala Ala
           115                 120                 125
   Gln Ala Lys Val Ala Leu Leu Ser Ala Gln Leu Asp Ala Ala Arg Asn
       130                 135                 140
   Gln Ala Ile Ile Asp Ala Leu Asp Gly Arg Gly Phe Arg Phe Val Ala
   145                 150                 155                 160
   Pro Ala Ala Ala Ser Pro Gly Glu Val Ala Thr His Arg Gly Leu Ala
                   165                 170                 175
   Arg Ala Arg Leu Gly Gln Ile Glu Ala Ala Leu Ala Gly Gly Arg Ser
               180                 185                 190
   Asp Arg Gly Ala Ala Val Ser Ala Ala Ala Glu Ala Gln Ala Gln Val
           195                 200                 205
   Arg Lys Leu Glu Gln Ser Leu Pro Leu Leu Glu Gln Gln Ile Ala Ala
       210                 215                 220
   Asn Glu Thr Met Ala Ala Lys Gly Tyr Val Ser Lys Leu Arg Val Val
   225                 230                 235                 240
   Glu Met Arg Arg Gln Leu Ile Ala Glu Arg Gln Asp Leu Thr Ala Ala
                   245                 250                 255
   Arg Ala Thr Leu Ala Lys Leu Gly Gln Gln Ser Leu Ser Val Ser Ser
               260                 265                 270
   Leu Ser Ala Lys Thr Arg Glu Glu Ala Arg Ala Gln Val Leu Gln Asp
           275                 280                 285
   Leu Val Lys Ala Gln Asp Glu Val Arg Ala Arg Gly Glu Asp Val Ala
       290                 295                 300
   Lys Ala Asn Leu Arg Ser Ser Phe Arg Glu Leu Arg Ala Pro Val Ser
   305                 310                 315                 320
   Gly Thr Val Ser Gln Leu Gln Val His Thr Glu Gly Gly Val Val Glu
                   325                 330                 335
   Gly Ala Lys Pro Leu Leu Ser Leu Val Pro Asp Asn Ala Arg Leu Glu
               340                 345                 350
   Ala Glu Val Met Val Asp Asn Ser Asp Ile Gly Phe Val His Ile Gly
           355                 360                 365
   Met Pro Val Lys Val Lys Leu Gln Ala Phe Pro Tyr Thr Arg Tyr Gly
       370                 375                 380
   Met Ile Pro Gly Thr Val Ala Gly Ile Ser Pro Glu Ala Val Gln Met
   385                 390                 395                 400
   Lys Glu Asn Gln Pro Pro Val Tyr Lys Ala Arg Ile Ala Leu Ala Arg
                   405                 410                 415
   Gly Tyr Val Leu Ala His Gly Ala Gln Val Pro Leu Arg Pro Gly Met
               420                 425                 430
   Leu Ala Ser Ala Asp Ile Val Thr Gly Lys Arg Thr Leu Phe Ser Tyr
           435                 440                 445
   Leu Val Gly Pro Val Leu Glu Thr Gly Ser Asp Ala Leu His Glu Arg
       450                 455                 460
<210> 32
   <211> 2187
   <212> DNA
   <213> Sphingomonas sp. ATCC53159
<220>
   <221> CDS
   <222> (1) ... (2187)
   <223> atrB
<400> 32
   atg aca cgc gac gaa atg cag gcc acc ctg cag agc gcg ctc gcg gcc
   48
   Met Thr Arg Asp Glu Met Gln Ala Thr Leu Gln Ser Ala Leu Ala Ala
    1               5                  10                  15
   cat ggg gcg gcg gag cgc gag gcg gag ctg cgc gaa tcc gga ctg gtg
   96
   His Gly Ala Ala Glu Arg Glu Ala Glu Leu Arg Glu Ser Gly Leu Val
               20                  25                  30
   gcg ttg tcg ctg ctg ctc ggc gcg cac aac atc gcc atc acg ccc gaa
   144
   Ala Leu Ser Leu Leu Leu Gly Ala His Asn Ile Ala Ile Thr Pro Glu
           35                  40                  45
   cag ctg cgc cac gcg ctg ggc cat gcc gag gcg gca agc gcc gac gac
   192
   Gln Leu Arg His Ala Leu Gly His Ala Glu Ala Ala Ser Ala Asp Asp
       50                  55                  60
   ctg atc ctc ctg gcc aag cgc cag cag ggc gtg cgc gcc aag gcc gtc
   240
   Leu Ile Leu Leu Ala Lys Arg Gln Gln Gly Val Arg Ala Lys Ala Val
    65                  70                  75                  80
   gag gtg ccg cgc ggc gga ctc gcc cgc cag ccg ctg ccc gcg atc gcc
   288
   Glu Val Pro Arg Gly Gly Leu Ala Arg Gln Pro Leu Pro Ala Ile Ala
                    85                  90                  95
   gac ggg ccc gaa ggc tgg ttc gtg atc ggc ggc ctg acc gaa cat ggc
   336
   Asp Gly Pro Glu Gly Trp Phe Val Ile Gly Gly Leu Thr Glu His Gly
               100                 105                 110
   gtg atc atc cag cgc ccg ggc cat gcc ccg gaa cag gtc gac cgg gac
   384
   Val Ile Ile Gln Arg Pro Gly His Ala Pro Glu Gln Val Asp Arg Asp
           115                 120                 125
   gcg ctg gac gcg atc tgg tcc ggc gcg ctg gtg ctg ctc acc acc cgc
   432
   Ala Leu Asp Ala Ile Trp Ser Gly Ala Leu Val Leu Leu Thr Thr Arg
       130                 135                 140
   gcg gtg gcg gga cgg ccg ctg cgg ttc ggc ctc tcc tgg ttc acc gcg
   480
   Ala Val Ala Gly Arg Pro Leu Arg Phe Gly Leu Ser Trp Phe Thr Ala
   145                 150                 155                 160
   cag ttc cgg cgc tat cgc acg ctg ttc ctc gag gtg ctc ggc atc acc
   528
   Gln Phe Arg Arg Tyr Arg Thr Leu Phe Leu Glu Val Leu Gly Ile Thr
                   165                 170                 175
   ctc gcg ctc aac ctg ctc ggc ctc gcc gcg ccg ctg ttg ttc cag agc
   576
   Leu Ala Leu Asn Leu Leu Gly Leu Ala Ala Pro Leu Leu Phe Gln Ser
               180                 185                 190
   gtg atc gac aag gtg ctg atc cac aac agc atg agc acg ctg agc gtg
   624
   Val Ile Asp Lys Val Leu Ile His Asn Ser Met Ser Thr Leu Ser Val
           195                 200                 205
   ctc gcc ttc gcc ttc ctg gcg gtt tcg gtg tgg gaa gtg gcg ctc ggc
   672
   Leu Ala Phe Ala Phe Leu Ala Val Ser Val Trp Glu Val Ala Leu Gly
       210                 215                 220
   tgg atc cgc acc cgc ctg ttc acc gag acg acg cag aag atc gac gtc
   720
   Trp Ile Arg Thr Arg Leu Phe Thr Glu Thr Thr Gln Lys Ile Asp Val
   225                 230                 235                 240
   gag ctg ggt gcc cgg ctg ttc cac cac ctg ctg gcg ctg ccg ctc gcc
   768
   Glu Leu Gly Ala Arg Leu Phe His His Leu Leu Ala Leu Pro Leu Ala
                   245                 250                 255
   tat ttc gag aag cgc cgc gtg ggc gac acc gtc acc cgc gtc cgc cag
   816
   Tyr Phe Glu Lys Arg Arg Val Gly Asp Thr Val Thr Arg Val Arg Gln
               260                 265                 270
   ctc gag acg atc cgc gaa ttc ctt acc agc gcc tcg ctg acg gtg atg
   864
   Leu Glu Thr Ile Arg Glu Phe Leu Thr Ser Ala Ser Leu Thr Val Met
           275                 280                 285
   gtg gac ccg ctg ttc acc ttc gtg ttc ctc gcc gcg atg ctg ttc tac 912
   Val Asp Pro Leu Phe Thr Phe Val Phe Leu Ala Ala Met Leu Phe Tyr
   290 295 300
   tcg ccg atg ctc tcg ggc atc gtg ctc gtg tcg ctg atc gcc tat gcg 960
   Ser Pro Met Leu Ser Gly Ile Val Leu Val Ser Leu Ile Ala Tyr Ala
   305 310 315 320
   atc gta tcg ttc agc gtc gcc ggg ccg ctc cgc gcg cgg gtg gag gac 1008
   Ile Val Ser Phe Ser Val Ala Gly Pro Leu Arg Ala Arg Val Glu Asp
   325 330 335
   aag ttc gag aag agc tcc gcc agc aac gcg ctg ctc gtc gag agc gtc 1056
   Lys Phe Glu Lys Ser Ser Ala Ser Asn Ala Leu Leu Val Glu Ser Val
   340 345 350
   tcg ggc atc cac acg atc aag gcg acc gcg gtc gag ccg cac tgg cag 1104
   Ser Gly Ile His Thr Ile Lys Ala Thr Ala Val Glu Pro His Trp Gln
   355 360 365
   aat cgc tgg gag cgc cag ctc gcc gcc cat acc gcc gcg tcg cag cgg 1152
   Asn Arg Trp Glu Arg Gln Leu Ala Ala His Thr Ala Ala Ser Gln Arg
   370 375 380
   ctg atc aat acc gcc aac acc ggc agc cag gcg atc gag ctg atc tcg 1200
   Leu Ile Asn Thr Ala Asn Thr Gly Ser Gln Ala Ile Glu Leu Ile Ser
   385 390 395 400
   aag ctg agc ttc gcg gcg atc ctg ttc ttc ggc gcc aag gcg gtg atc 1248
   Lys Leu Ser Phe Ala Ala Ile Leu Phe Phe Gly Ala Lys Ala Val Ile
   405 410 415
   ggc ggc gcg atg agc gta ggc gcg ctg gtg gcg ttc aac atg ttc gcc 1296
   Gly Gly Ala Met Ser Val Gly Ala Leu Val Ala Phe Asn Met Phe Ala
   420 425 430
   cag cgc gtg tcc ggg ccg gtg atc cgc atg gcg cag ctg tgg cag gat 1344
   Gln Arg Val Ser Gly Pro Val Ile Arg Met Ala Gln Leu Trp Gln Asp
   435 440 445
   ttc cag cag gtg cgc atc tcg gtc gag cgg ctg ggc gac gtg ctc aac 1392
   Phe Gln Gln Val Arg Ile Ser Val Glu Arg Leu Gly Asp Val Leu Asn
   450 455 460
   cat ccg gtg gaa ccg cgc ccg gcc tcg gcg gcg acg ctg ccg gtg ctg 1440
   His Pro Val Glu Pro Arg Pro Ala Ser Ala Ala Thr Leu Pro Val Leu
   465 470 475 480
   cgc ggt gcg att cgc ttc gag aat gtc agc ttc cgc tat gcc gag gac
   1488
   Arg Gly Ala Ile Arg Phe Glu Asn Val Ser Phe Arg Tyr Ala Glu Asp
   485 490 495
   cag ccg ccg gtg ctg agc gac atc acg ctc gac att ccg gcg ggc acc
   1536
   Gln Pro Pro Val Leu Ser Asp Ile Thr Leu Asp Ile Pro Ala Gly Thr
   500 505 510
   tcg ctc ggc atc gtc ggt tcg tcg ggc tcg ggc aag tcg acg ctg gcc
   1584
   Ser Leu Gly Ile Val Gly Ser Ser Gly Ser Gly Lys Ser Thr Leu Ala
   515 520 525
   aag ctg ctc cag cgg ctc aac ctg ccg aat ctc ggc cgc gtg ctg gtc
   1632
   Lys Leu Leu Gln Arg Leu Asn Leu Pro Asn Leu Gly Arg Val Leu Val
   530 535 540
   gac gag gtc gac gtg gcg cag ctc gat ccc gcc tgg ctg cgt cgc cag
   1680
   Asp Glu Val Asp Val Ala Gln Leu Asp Pro Ala Trp Leu Arg Arg Gln
   545 550 555 560
   atc ggc gtc gtg ctg cag gag aat ctg ctg ttc agc cgc tcg atc cgc
   1728
   Ile Gly Val Val Leu Gln Glu Asn Leu Leu Phe Ser Arg Ser Ile Arg
   565 570 575
   gag aac atc gcg ctc tcc aac ccc gcc atg ccg ttc gag aat gtc gtc
   1776
   Glu Asn Ile Ala Leu Ser Asn Pro Ala Met Pro Phe Glu Asn Val Val
   580 585 590
   gcg gcg gcg acg ctg gcc ggc gcg cat gat ttc atc ctg cgc cag ccg
   1824
   Ala Ala Ala Thr Leu Ala Gly Ala His Asp Phe Ile Leu Arg Gln Pro
   595 600 605
   cgc ggc tat gac acc gag atc gtc gag cgc ggc gtc aat ctc tcc ggc
   1872
   Arg Gly Tyr Asp Thr Glu Ile Val Glu Arg Gly Val Asn Leu Ser Gly
   610 615 620
   ggc cag cgc cag cgg ctc gcc atc gcc cgc gcg ctc gtc ggc aat ccg
   1920
   Gly Gln Arg Gln Arg Leu Ala Ile Ala Arg Ala Leu Val Gly Asn Pro
   625 630 635 640
   cgc atc ctg gtg ttc gac gaa gcg acc tcg gcg ctc gat gcc gag agc
   1968
   Arg Ile Leu Val Phe Asp Glu Ala Thr Ser Ala Leu Asp Ala Glu Ser
   645 650 655
   gag gag ctg atc cag aac aac ctg cgc gcc atc tcg gcc ggc cgc acg
   2016
   Glu Glu Leu Ile Gln Asn Asn Leu Arg Ala Ile Ser Ala Gly Arg Thr
               660                 665                 670
   ctc gtg gtg atc gcg cat cgc ctg agc gcg gtg cgc agc tgc gac cgg
   2064
   Leu Val Val Ile Ala His Arg Leu Ser Ala Val Arg Ser Cys Asp Arg
           675                 680                 685
   atc atc acg ctc gaa cag ggc cgc atc gtc gag agc ggc cga cac gac
   2112
   Ile Ile Thr Leu Glu Gln Gly Arg Ile Val Glu Ser Gly Arg His Asp
       690                 695                 700
   gaa ttg ttg cgc ctg ggc ggc cgc tat gcc gac ctg cac cgc cgc cag
   2160
   Glu Leu Leu Arg Leu Gly Gly Arg Tyr Ala Asp Leu His Arg Arg Gln
   705                 710                 715                 720
   ggc ggc tat ggg gag att gcc gca tga
   2187
   Gly Gly Tyr Gly Glu Ile Ala Ala  *
                   725
<210> 33
   <211> 728
   <212> PRT
   <213> Sphingomonas sp. ATCC53159
<220>
   <221> PEPTIDE
   <222> (0) ... (0)
   <223> putative secretion protein
<400> 33
   Met Thr Arg Asp Glu Met Gln Ala Thr Leu Gln Ser Ala Leu Ala Ala
    1               5                  10                  15
   His Gly Ala Ala Glu Arg Glu Ala Glu Leu Arg Glu Ser Gly Leu Val
               20                  25                  30
   Ala Leu Ser Leu Leu Leu Gly Ala His Asn Ile Ala Ile Thr Pro Glu
           35                  40                  45
   Gln Leu Arg His Ala Leu Gly His Ala Glu Ala Ala Ser Ala Asp Asp
       50                  55                  60
   Leu Ile Leu Leu Ala Lys Arg Gln Gln Gly Val Arg Ala Lys Ala Val
   65                  70                  75                  80
   Glu Val Pro Arg Gly Gly Leu Ala Arg Gln Pro Leu Pro Ala Ile Ala
                   85                  90                  95
   Asp Gly Pro Glu Gly Trp Phe Val Ile Gly Gly Leu Thr Glu His Gly
               100                 105                 110
   Val Ile Ile Gln Arg Pro Gly His Ala Pro Glu Gln Val Asp Arg Asp
           115                 120                 125
   Ala Leu Asp Ala Ile Trp Ser Gly Ala Leu Val Leu Leu Thr Thr Arg
       130                 135                 140
   Ala Val Ala Gly Arg Pro Leu Arg Phe Gly Leu Ser Trp Phe Thr Ala
   145                 150                 155                 160
   Gln Phe Arg Arg Tyr Arg Thr Leu Phe Leu Glu Val Leu Gly Ile Thr
                   165                 170                 175
   Leu Ala Leu Asn Leu Leu Gly Leu Ala Ala Pro Leu Leu Phe Gln Ser
               180                 185                 190
   Val Ile Asp Lys Val Leu Ile His Asn Ser Met Ser Thr Leu Ser Val
           195                 200                 205
   Leu Ala Phe Ala Phe Leu Ala Val Ser Val Trp Glu Val Ala Leu Gly
       210                 215                 220
   Trp Ile Arg Thr Arg Leu Phe Thr Glu Thr Thr Gln Lys Ile Asp Val
   225                 230                 235                 240
   Glu Leu Gly Ala Arg Leu Phe His His Leu Leu Ala Leu Pro Leu Ala
                   245                 250                 255
   Tyr Phe Glu Lys Arg Arg Val Gly Asp Thr Val Thr Arg Val Arg Gln
               260                 265                 270
   Leu Glu Thr Ile Arg Glu Phe Leu Thr Ser Ala Ser Leu Thr Val Met
           275                 280                 285
   Val Asp Pro Leu Phe Thr Phe Val Phe Leu Ala Ala Met Leu Phe Tyr
       290                 295                 300
   Ser Pro Met Leu Ser Gly Ile Val Leu Val Ser Leu Ile Ala Tyr Ala
   305                 310                 315                 320
   Ile Val Ser Phe Ser Val Ala Gly Pro Leu Arg Ala Arg Val Glu Asp
                   325                 330                 335
   Lys Phe Glu Lys Ser Ser Ala Ser Asn Ala Leu Leu Val Glu Ser Val
               340                 345                 350
   Ser Gly Ile His Thr Ile Lys Ala Thr Ala Val Glu Pro His Trp Gln
           355                 360                 365
   Asn Arg Trp Glu Arg Gln Leu Ala Ala His Thr Ala Ala Ser Gln Arg
       370                 375                 380
   Leu Ile Asn Thr Ala Asn Thr Gly Ser Gln Ala Ile Glu Leu Ile Ser
   385                 390                 395                 400
   Lys Leu Ser Phe Ala Ala Ile Leu Phe Phe Gly Ala Lys Ala Val Ile
                   405                 410                 415
   Gly Gly Ala Met Ser Val Gly Ala Leu Val Ala Phe Asn Met Phe Ala
               420                 425                 430
   Gln Arg Val Ser Gly Pro Val Ile Arg Met Ala Gln Leu Trp Gln Asp
           435                 440                 445
   Phe Gln Gln Val Arg Ile Ser Val Glu Arg Leu Gly Asp Val Leu Asn
       450                 455                 460
   His Pro Val Glu Pro Arg Pro Ala Ser Ala Ala Thr Leu Pro Val Leu
   465                 470                 475                 480
   Arg Gly Ala Ile Arg Phe Glu Asn Val Ser Phe Arg Tyr Ala Glu Asp
                   485                 490                 495
   Gln Pro Pro Val Leu Ser Asp Ile Thr Leu Asp Ile Pro Ala Gly Thr
               500                 505                 510
   Ser Leu Gly Ile Val Gly Ser Ser Gly Ser Gly Lys Ser Thr Leu Ala
           515                 520                 525
   Lys Leu Leu Gln Arg Leu Asn Leu Pro Asn Leu Gly Arg Val Leu Val
       530                 535                 540
   Asp Glu Val Asp Val Ala Gln Leu Asp Pro Ala Trp Leu Arg Arg Gln
   545                 550                 555                 560
   Ile Gly Val Val Leu Gln Glu Asn Leu Leu Phe Ser Arg Ser Ile Arg
                   565                 570                 575
   Glu Asn Ile Ala Leu Ser Asn Pro Ala Met Pro Phe Glu Asn Val Val
               580                 585                 590
   Ala Ala Ala Thr Leu Ala Gly Ala His Asp Phe Ile Leu Arg Gln Pro
           595                 600                 605
   Arg Gly Tyr Asp Thr Glu Ile Val Glu Arg Gly Val Asn Leu Ser Gly
       610                 615                 620
   Gly Gln Arg Gln Arg Leu Ala Ile Ala Arg Ala Leu Val Gly Asn Pro
   625                 630                 635                 640
   Arg Ile Leu Val Phe Asp Glu Ala Thr Ser Ala Leu Asp Ala Glu Ser
                   645                 650                 655
   Glu Glu Leu Ile Gln Asn Asn Leu Arg Ala Ile Ser Ala Gly Arg Thr
               660                 665                 670
   Leu Val Val Ile Ala His Arg Leu Ser Ala Val Arg Ser Cys Asp Arg
           675                 680                 685
   Ile Ile Thr Leu Glu Gln Gly Arg Ile Val Glu Ser Gly Arg His Asp
       690                 695                 700
   Glu Leu Leu Arg Leu Gly Gly Arg Tyr Ala Asp Leu His Arg Arg Gln
   705                 710                 715                 720
   Gly Gly Tyr Gly Glu Ile Ala Ala
                   725
<210> 34
   <211> 1413
   <212> DNA
   <213> Sphingomonas sp. ATCC53159
<220>
   <221> CDS
   <222> (1)...(1413)
   <223> dpsB
<400> 34
   atg aac gct ttc gaa gca cag cgc gcc ttt gag gag caa ctt cgg gcg
   48
   Met Asn Ala Phe Glu Ala Gln Arg Ala Phe Glu Glu Gln Leu Arg Ala
    1               5                  10                  15
   cat tcc cgg gtt acg cca tct gcc gct ccc gtg tgg cgt cgc tcg acg
   96
   His Ser Arg Val Thr Pro Ser Ala Ala Pro Val Trp Arg Arg Ser Thr
                20                  25                  30
   ctg cgg atg gtc ctc tat acc gag ttg ctg ctg ctg gac agt ctc tcg
   144
   Leu Arg Met Val Leu Tyr Thr Glu Leu Leu Leu Leu Asp Ser Leu Ser
            35                  40                  45
   atc ctg gcc gga ttc cac gtc gcg gcg ggc acg cgc gac ggc aac tgg
   192
   Ile Leu Ala Gly Phe His Val Ala Ala Gly Thr Arg Asp Gly Asn Trp
        50                  55                  60
   ctg tcg ctg gcg ggc atc aac gtc ggc gtc ttc ctg ctg ccg atc gct
   240
   Leu Ser Leu Ala Gly Ile Asn Val Gly Val Phe Leu Leu Pro Ile Ala
    65                  70                  75                  80
   ctc ggc acc gcg ctc gca agc ggc acc tac tcg ctg aac tgc ctg cgc
   288
   Leu Gly Thr Ala Leu Ala Ser Gly Thr Tyr Ser Leu Asn Cys Leu Arg
                    85                  90                  95
   tac ccg gtc agc ggc gtg aag agc atc ttc tcg gca ttc ttc ttc tcg
   336
   Tyr Pro Val Ser Gly Val Lys Ser Ile Phe Ser Ala Phe Phe Phe Ser
               100                 105                 110
   atc ttc gtc gtc ctg ctc ggc agc tac ctg ctg acg gcc gag ctg ccg
   384
   Ile Phe Val Val Leu Leu Gly Ser Tyr Leu Leu Thr Ala Glu Leu Pro
           115                 120                 125
   ctg tcc cgc gtg cag ctg gcg gag ggc gcg atc ctc tcg ctg gtc ctc
   432
   Leu Ser Arg Val Gln Leu Ala Glu Gly Ala Ile Leu Ser Leu Val Leu
       130                 135                 140
   ctg atg gtg ggc cgc ctg atg ttc cgc cgc cac gtc cgc gcg gtt acc
   480
   Leu Met Val Gly Arg Leu Met Phe Arg Arg His Val Arg Ala Val Thr
   145                 150                 155                 160
   ggc ggc agg ctg ctc gac gaa ctg gtc atc atc gac ggc gtc tcg ctc
   528
   Gly Gly Arg Leu Leu Asp Glu Leu Val Ile Ile Asp Gly Val Ser Leu
                   165                 170                 175
   gac gtc gcg ggc aat gcg gtc gcg ctc gac gcg cgg atc atc aat ctc
   576
   Asp Val Ala Gly Asn Ala Val Ala Leu Asp Ala Arg Ile Ile Asn Leu
               180                 185                 190
   tcg ccg aac ccg cgc gat ccg caa atg ctg cat cgc ctg ggc acc acc
   624
   Ser Pro Asn Pro Arg Asp Pro Gln Met Leu His Arg Leu Gly Thr Thr
           195                 200                 205
   gtg atc ggg ttc gac cgg gtg atc gtc gcc tgc acc aag gag cat cgc
   672
   Val Ile Gly Phe Asp Arg Val Ile Val Ala Cys Thr Lys Glu His Arg
       210                 215                 220
   gcg gtc tgg gcg ctg ctg ctc aag ggc atg aac atc aag ggc gag atc
   720
   Ala Val Trp Ala Leu Leu Leu Lys Gly Met Asn Ile Lys Gly Glu Ile
   225                 230                 235                 240
   ctc gtc ccc cag ttc aat gcg ctg ggc gcg atc ggc gtg gac gcc ttt
   768
   Leu Val Pro Gln Phe Asn Ala Leu Gly Ala Ile Gly Val Asp Ala Phe
                   245                 250                 255
   gac ggg aag gat acg ctg gtc gtc tcg cag ggc ccg ctc aac atg ccc
   816
   Asp Gly Lys Asp Thr Leu Val Val Ser Gln Gly Pro Leu Asn Met Pro
               260                 265                 270
   aac cgc gcg aag aag cgc gcg ctc gat ctc gcg atc acc gta ccg gcc
   864
   Asn Arg Ala Lys Lys Arg Ala Leu Asp Leu Ala Ile Thr Val Pro Ala
   275 280 285
   gtg ctc gcg ctg gcg ccg ctg atg atc ctg gtg gcg atc ctg atc aag
   912
   Val Leu Ala Leu Ala Pro Leu Met Ile Leu Val Ala Ile Leu Ile Lys
   290 295 300
   ctg gag agc ccg ggc ccg gtg ttg ttc gcg cag gat cgc gtc ggc cgc
   960
   Leu Glu Ser Pro Gly Pro Val Leu Phe Ala Gln Asp Arg Val Gly Arg
   305 310 315 320
   ggc aac cgg ctg ttc aag atc atg aag ttc cgc tcg atg cgc gta acg
   1008
   Gly Asn Arg Leu Phe Lys Ile Met Lys Phe Arg Ser Met Arg Val Thr
   325 330 335
   ctg tgc gac gcg aac ggc aac gtc tcg gcc agc cgc gac gac gat cgc
   1056
   Leu Cys Asp Ala Asn Gly Asn Val Ser Ala Ser Arg Asp Asp Asp Arg
   340 345 350
   atc acc aag gtc ggc cgc ttc atc cgc aag acc agc atc gac gaa ctg
   1104
   Ile Thr Lys Val Gly Arg Phe Ile Arg Lys Thr Ser Ile Asp Glu Leu
   355 360 365
   ccg cag ctg ctg aac gtg ctg cgc ggc gac atg agc gtc gtc ggc ccg
   1152
   Pro Gln Leu Leu Asn Val Leu Arg Gly Asp Met Ser Val Val Gly Pro
   370 375 380
   cgg ccg cat gcg ctg ggc tcg cgc gcc gcc gat cac ctg ttc tgg gaa
   1200
   Arg Pro His Ala Leu Gly Ser Arg Ala Ala Asp His Leu Phe Trp Glu
   385 390 395 400
   atc gac gag cgc tac tgg cac cgc cac acg ctc aag ccg ggc atg acc
   1248
   Ile Asp Glu Arg Tyr Trp His Arg His Thr Leu Lys Pro Gly Met Thr
   405 410 415
   ggt ctg gcc cag gtg cgc ggt ttc cgc ggg gcg acc gat cgc cgc gtc
   1296
   Gly Leu Ala Gln Val Arg Gly Phe Arg Gly Ala Thr Asp Arg Arg Val
   420 425 430
   gat ctg acc aac cgg ctc cag gca gac atg gaa tat atc gac gga tgg
   1344
   Asp Leu Thr Asn Arg Leu Gln Ala Asp Met Glu Tyr Ile Asp Gly Trp
   435 440 445
   gat atc tgg cgc gat atc acg atc ctg ttc aag acg ctg cgg gtg atc
   1392
   Asp Ile Trp Arg Asp Ile Thr Ile Leu Phe Lys Thr Leu Arg Val Ile
       450                 455                     460
   gtg cat tcg aac gca ttc tga
   1413
   Val His Ser Asn Ala Phe  *
   465                 470
<210> 35
   <211> 470
   <212> PRT
   <213> Sphingomonas sp. ATCC53159
<220>
   <221> PEPTIDE
   <222> (0)...(0)
   <223> glucosyl-isoprenylphosphate transferase I
<400> 35
   Met Asn Ala Phe Glu Ala Gln Arg Ala Phe Glu Glu Gln Leu Arg Ala
    1               5                  10                  15
   His Ser Arg Val Thr Pro Ser Ala Ala Pro Val Trp Arg Arg Ser Thr
               20                  25                  30
   Leu Arg Met Val Leu Tyr Thr Glu Leu Leu Leu Leu Asp Ser Leu Ser
           35                  40                  45
   Ile Leu Ala Gly Phe His Val Ala Ala Gly Thr Arg Asp Gly Asn Trp
       50                  55                  60
   Leu Ser Leu Ala Gly Ile Asn Val Gly Val Phe Leu Leu Pro Ile Ala
   65                  70                  75                  80
   Leu Gly Thr Ala Leu Ala Ser Gly Thr Tyr Ser Leu Asn Cys Leu Arg
                   85                  90                  95
   Tyr Pro Val Ser Gly Val Lys Ser Ile Phe Ser Ala Phe Phe Phe Ser
               100                 105                 110
   Ile Phe Val Val Leu Leu Gly Ser Tyr Leu Leu Thr Ala Glu Leu Pro
           115                 120                 125
   Leu Ser Arg Val Gln Leu Ala Glu Gly Ala Ile Leu Ser Leu Val Leu
       130                 135                 140
   Leu Met Val Gly Arg Leu Met Phe Arg Arg His Val Arg Ala Val Thr
   145                 150                 155                 160
   Gly Gly Arg Leu Leu Asp Glu Leu Val Ile Ile Asp Gly Val Ser Leu
                   165                 170                 175
   Asp Val Ala Gly Asn Ala Val Ala Leu Asp Ala Arg Ile Ile Asn Leu
               180                 185                 190
   Ser Pro Asn Pro Arg Asp Pro Gln Met Leu His Arg Leu Gly Thr Thr
           195                 200                 205
   Val Ile Gly Phe Asp Arg Val Ile Val Ala Cys Thr Lys Glu His Arg
       210                 215                 220
   Ala Val Trp Ala Leu Leu Leu Lys Gly Met Asn Ile Lys Gly Glu Ile
   225                 230                 235                 240
   Leu Val Pro Gln Phe Asn Ala Leu Gly Ala Ile Gly Val Asp Ala Phe
                   245                 250                 255
   Asp Gly Lys Asp Thr Leu Val Val Ser Gln Gly Pro Leu Asn Met Pro
               260                 265                 270
   Asn Arg Ala Lys Lys Arg Ala Leu Asp Leu Ala Ile Thr Val Pro Ala
           275                 280                 285
   Val Leu Ala Leu Ala Pro Leu Met Ile Leu Val Ala Ile Leu Ile Lys
       290                 295                 300
   Leu Glu Ser Pro Gly Pro Val Leu Phe Ala Gln Asp Arg Val Gly Arg
   305                 310                 315                 320
   Gly Asn Arg Leu Phe Lys Ile Met Lys Phe Arg Ser Met Arg Val Thr
                   325                 330                 335
   Leu Cys Asp Ala Asn Gly Asn Val Ser Ala Ser Arg Asp Asp Asp Arg
               340                 345                 350
   Ile Thr Lys Val Gly Arg Phe Ile Arg Lys Thr Ser Ile Asp Glu Leu
           355                 360                 365
   Pro Gln Leu Leu Asn Val Leu Arg Gly Asp Met Ser Val Val Gly Pro
       370                 375                 380
   Arg Pro His Ala Leu Gly Ser Arg Ala Ala Asp His Leu Phe Trp Glu
   385                 390                 395                 400
   Ile Asp Glu Arg Tyr Trp His Arg His Thr Leu Lys Pro Gly Met Thr
                   405                 410                 415
   Gly Leu Ala Gln Val Arg Gly Phe Arg Gly Ala Thr Asp Arg Arg Val
               420                 425                 430
   Asp Leu Thr Asn Arg Leu Gln Ala Asp Met Glu Tyr Ile Asp Gly Trp
           435                 440                 445
   Asp Ile Trp Arg Asp Ile Thr Ile Leu Phe Lys Thr Leu Arg Val Ile
       450                 455                 460
   Val His Ser Asn Ala Phe
   465                 470
<210> 36
   <211> 879
   <212> DNA
   <213> Sphingomonas sp. ATCC53159
<220>
   <221> CDS
   <222> (1)...(879)
   <223> rmlA
<400> 36
   atg aag ggc atc atc ctt gcg ggg ggc agc ggg acg cgc ctg tac ccc
   48
   Met Lys Gly Ile Ile Leu Ala Gly Gly Ser Gly Thr Arg Leu Tyr Pro
    1               5                  10                  15
   gca acg cta tcg atc tcg aag cag ctg ctt ccc gtc tat gac aag ccg
   96
   Ala Thr Leu Ser Ile Ser Lys Gln Leu Leu Pro Val Tyr Asp Lys Pro
                20                  25                  30
   atg atc ttc tat ccg ctg tcg gtg ctg atg ctc acc ggc atc cgg gac
   144
   Met Ile Phe Tyr Pro Leu Ser Val Leu Met Leu Thr Gly Ile Arg Asp
            35                  40                  45
   atc ctg att atc tcc acc ccg cgc gac ctg ccg atg ttc cag gcg ctg
   192
   Ile Leu Ile Ile Ser Thr Pro Arg Asp Leu Pro Met Phe Gln Ala Leu
        50                  55                  60
   ctg ggc gac ggc tcg gcc ttc ggc atc aac ctc agc tat gcc gag cag
   240
   Leu Gly Asp Gly Ser Ala Phe Gly Ile Asn Leu Ser Tyr Ala Glu Gln
    65                  70                  75                  80
   ccc tcc ccc aac ggg ctg gcc gaa gcg ttc atc atc ggc gcg gat ttc
   288
   Pro Ser Pro Asn Gly Leu Ala Glu Ala Phe Ile Ile Gly Ala Asp Phe
                    85                  90                  95
   gtc ggc aac gat ccc agc gcg ctg atc ctg ggc gac aac atc tat cac
   336
   Val Gly Asn Asp Pro Ser Ala Leu Ile Leu Gly Asp Asn Ile Tyr His
               100                 105                 110
   ggc gaa aag atg ggc gag cgc tgc cag gca gcc gca gcg cag gca gcg
   384
   Gly Glu Lys Met Gly Glu Arg Cys Gln Ala Ala Ala Ala Gln Ala Ala
           115                 120                 125
   cag ggc ggt gca aac gtc ttc gcc tat cat gtc gac gac ccc gag cgc
   432
   Gln Gly Gly Ala Asn Val Phe Ala Tyr His Val Asp Asp Pro Glu Arg
       130                 135                 140
   tac ggc gtg gtc gcg ttc gac ccg gag acg ggc gtc gcc acc agc gtc
   480
   Tyr Gly Val Val Ala Phe Asp Pro Glu Thr Gly Val Ala Thr Ser Val
   145                 150                 155                 160
   gag gaa aag ccg gcc gag ccc aag tcc aac tgg gcg atc acc ggc ctg
   528
   Glu Glu Lys Pro Ala Glu Pro Lys Ser Asn Trp Ala Ile Thr Gly Leu
                   165                 170                 175
   tat ttc tac gac aag gac gtg gtc gac atc gcc aag tcg atc cag ccc
   576
   Tyr Phe Tyr Asp Lys Asp Val Val Asp Ile Ala Lys Ser Ile Gln Pro
               180                 185                 190
   tcg gcg cgc ggc gaa ctc gag atc acc gac gtc aac cgc gtt tac atg
   624
   Ser Ala Arg Gly Glu Leu Glu Ile Thr Asp Val Asn Arg Val Tyr Met
           195                 200                 205
   gag cgc ggc gac ctg cac atc acg cgc ctc ggc cgc ggc tat gcc tgg
   672
   Glu Arg Gly Asp Leu His Ile Thr Arg Leu Gly Arg Gly Tyr Ala Trp
       210                 215                 220
   ctc gac acc ggc acg cat gac agc ctg cac gaa gcc ggc tcg ttc gtt
   720
   Leu Asp Thr Gly Thr His Asp Ser Leu His Glu Ala Gly Ser Phe Val
   225                 230                 235                 240
   cgc acg ctc gag cat cgg acg ggc gtg aag atc gcc tgc ccg gag gaa
   768
   Arg Thr Leu Glu His Arg Thr Gly Val Lys Ile Ala Cys Pro Glu Glu
                   245                 250                 255
   atc gcc ttc gaa agc ggc tgg ctc ggc gcc gaa gac ctg ctc aag cgc
   816
   Ile Ala Phe Glu Ser Gly Trp Leu Gly Ala Glu Asp Leu Leu Lys Arg
               260                 265                 270
   gcc gcc ggc ctc ggc aag acc ggc tat gcc gcc tat ctc cgc aag gtt
   864
   Ala Ala Gly Leu Gly Lys Thr Gly Tyr Ala Ala Tyr Leu Arg Lys Val
           275                 280                 285
   gcg acc gca gca tga
   879
   Ala Thr Ala Ala  *
       290
<210> 37
   <211> 292
   <212> PRT
   <213> Sphingomonas sp. ATCC53159
<220>
   <221> PEPTIDE
   <222> (0)...(0)
   <223> glucose-1-phosphate thymidylyltransferase
<400> 37
   Met Lys Gly Ile Ile Leu Ala Gly Gly Ser Gly Thr Arg Leu Tyr Pro
    1               5                  10                  15
   Ala Thr Leu Ser Ile Ser Lys Gln Leu Leu Pro Val Tyr Asp Lys Pro
               20                  25                  30
   Met Ile Phe Tyr Pro Leu Ser Val Leu Met Leu Thr Gly Ile Arg Asp
           35                  40                  45
   Ile Leu Ile Ile Ser Thr Pro Arg Asp Leu Pro Met Phe Gln Ala Leu
       50                  55                  60
   Leu Gly Asp Gly Ser Ala Phe Gly Ile Asn Leu Ser Tyr Ala Glu Gln
   65                  70                  75                  80
   Pro Ser Pro Asn Gly Leu Ala Glu Ala Phe Ile Ile Gly Ala Asp Phe
                   85                  90                  95
   Val Gly Asn Asp Pro Ser Ala Leu Ile Leu Gly Asp Asn Ile Tyr His
               100                 105                 110
   Gly Glu Lys Met Gly Glu Arg Cys Gln Ala Ala Ala Ala Gln Ala Ala
           115                 120                 125
   Gln Gly Gly Ala Asn Val Phe Ala Tyr His Val Asp Asp Pro Glu Arg
       130                 135                 140
   Tyr Gly Val Val Ala Phe Asp Pro Glu Thr Gly Val Ala Thr Ser Val
   145                 150                 155                 160
   Glu Glu Lys Pro Ala Glu Pro Lys Ser Asn Trp Ala Ile Thr Gly Leu
                   165                 170                 175
   Tyr Phe Tyr Asp Lys Asp Val Val Asp Ile Ala Lys Ser Ile Gln Pro
               180                 185                 190
   Ser Ala Arg Gly Glu Leu Glu Ile Thr Asp Val Asn Arg Val Tyr Met
           195                 200                 205
   Glu Arg Gly Asp Leu His Ile Thr Arg Leu Gly Arg Gly Tyr Ala Trp
       210                 215                 220
   Leu Asp Thr Gly Thr His Asp Ser Leu His Glu Ala Gly Ser Phe Val
   225                 230                 235                 240
   Arg Thr Leu Glu His Arg Thr Gly Val Lys Ile Ala Cys Pro Glu Glu
                   245                 250                 255
   Ile Ala Phe Glu Ser Gly Trp Leu Gly Ala Glu Asp Leu Leu Lys Arg
               260                 265                 270
   Ala Ala Gly Leu Gly Lys Thr Gly Tyr Ala Ala Tyr Leu Arg Lys Val
           275                 280                 285
   Ala Thr Ala Ala
       290
<210> 38
   <211> 567
   <212> DNA
   <213> Sphingomonas sp. ATCC53159
<220>
   <221> CDS
   <222> (1)...(567)
   <223> rmlC
<400> 38
   atg acc cag gtc cat cat cac gaa ctg tcc ggc gtc atc gag ttc acg
   48
   Met Thr Gln Val His His His Glu Leu Ser Gly Val Ile Glu Phe Thr
    1               5                  10                  15
   ccg ccc aaa tat ggc gac cac cgc ggc ttc ttc tcc gaa gtg ttc aag
   96
   Pro Pro Lys Tyr Gly Asp His Arg Gly Phe Phe Ser Glu Val Phe Lys
               20                  25                  30
   cag tcg gtg ctc gat gcc gaa ggc gtc gag gca cgc tgg gtg cag gac
   144
   Gln Ser Val Leu Asp Ala Glu Gly Val Glu Ala Arg Trp Val Gln Asp
           35                  40                  45
   aat cag agc ttc tcg gcg gcc ccg ggc acg atc cgc ggc ctg cat ctc
   192
   Asn Gln Ser Phe Ser Ala Ala Pro Gly Thr Ile Arg Gly Leu His Leu
       50                  55                  60
   cag gcg ccg ccc ttc gcc cag gcc aag ctg gtc cgc gtg ttg cgc ggc
   240
   Gln Ala Pro Pro Phe Ala Gln Ala Lys Leu Val Arg Val Leu Arg Gly
   65                  70                  75                  80
   gcg atc ttc gac gtc gcg gtc gac atc cgt cgc ggc tcg ccc acc tat
   288
   Ala Ile Phe Asp Val Ala Val Asp Ile Arg Arg Gly Ser Pro Thr Tyr
                   85                  90                  95
   ggc aaa tgg gtc ggc gtc gag ctc tcg gcc gag aag tgg aac cag ctg
   336
   Gly Lys Trp Val Gly Val Glu Leu Ser Ala Glu Lys Trp Asn Gln Leu
               100                 105                 110
   ctg gtc ccc gcc ggc tat gcg cac ggc ttc atg acg ctc gtt ccg gat
   384
   Leu Val Pro Ala Gly Tyr Ala His Gly Phe Met Thr Leu Val Pro Asp
           115                 120                 125
   tgc gag atc ctc tac aag gtc agc gcc aaa tat tcg aag gat tcg gag
   432
   Cys Glu Ile Leu Tyr Lys Val Ser Ala Lys Tyr Ser Lys Asp Ser Glu
       130                 135                 140
   atg gcg atc cgt tgg gac gat ccc gat ctc gcc atc gcc tgg ccg gac
   480
   Met Ala Ile Arg Trp Asp Asp Pro Asp Leu Ala Ile Ala Trp Pro Asp
   145                 150                 155                 160
   atc ggc gtc gag ccg gtc ctc tcc gaa aag gac gcg gtc gcc acg ccc
   528
   Ile Gly Val Glu Pro Val Leu Ser Glu Lys Asp Ala Val Ala Thr Pro
                   165                 170                 175
   ttc gcc gaa ttc aac acc ccc ttc ttc tat cag ggc tga
   567
   Phe Ala Glu Phe Asn Thr Pro Phe Phe Tyr Gln Gly  *
               180                 185
<210> 39
   <211> 188
   <212> PRT
   <213> Sphingomonas sp. ATCC53159
<220>
   <221> PEPTIDE
   <222> (0) ... (0)
   <223> dTDP-6-deoxy-D-glucose-3-5-epimerase
<400> 39
   Met Thr Gln Val His His His Glu Leu Ser Gly Val Ile Glu Phe Thr
    1               5                  10                  15
   Pro Pro Lys Tyr Gly Asp His Arg Gly Phe Phe Ser Glu Val Phe Lys
               20                  25                  30
   Gln Ser Val Leu Asp Ala Glu Gly Val Glu Ala Arg Trp Val Gln Asp
           35                  40                  45
   Asn Gln Ser Phe Ser Ala Ala Pro Gly Thr Ile Arg Gly Leu His Leu
       50                  55                  60
   Gln Ala Pro Pro Phe Ala Gln Ala Lys Leu Val Arg Val Leu Arg Gly
   65                  70                  75                  80
   Ala Ile Phe Asp Val Ala Val Asp Ile Arg Arg Gly Ser Pro Thr Tyr
                   85                  90                  95
   Gly Lys Trp Val Gly Val Glu Leu Ser Ala Glu Lys Trp Asn Gln Leu
               100                 105                 110
   Leu Val Pro Ala Gly Tyr Ala His Gly Phe Met Thr Leu Val Pro Asp
           115                 120                 125
   Cys Glu Ile Leu Tyr Lys Val Ser Ala Lys Tyr Ser Lys Asp Ser Glu
       130                 135                 140
   Met Ala Ile Arg Trp Asp Asp Pro Asp Leu Ala Ile Ala Trp Pro Asp
   145                 150                 155                 160
   Ile Gly Val Glu Pro Val Leu Ser Glu Lys Asp Ala Val Ala Thr Pro
                   165                 170                 175
   Phe Ala Glu Phe Asn Thr Pro Phe Phe Tyr Gln Gly
               180                 185
<210> 40
   <211> 1062
   <212> DNA
   <213> Sphingomonas sp. ATCC53159
<220>
   <221> CDS
   <222> (1)...(1062)
   <223> rmlB
<400> 40
   atg cag cag acc ttc ctc gtc acc ggc ggc gcc ggc ttc atc ggc tcg
   48
   Met Gln Gln Thr Phe Leu Val Thr Gly Gly Ala Gly Phe Ile Gly Ser
    1               5                  10                  15
   gcg gtg gtg cgc cac ctc gtc cgc cag ggc gcg cgc gtc atc aat ctc
   96
   Ala Val Val Arg His Leu Val Arg Gln Gly Ala Arg Val Ile Asn Leu
               20                  25                  30
   gac aag ctc acc tat gcc ggc aac ccg gcc tcg ctg act gcg atc gag
   144
   Asp Lys Leu Thr Tyr Ala Gly Asn Pro Ala Ser Leu Thr Ala Ile Glu
           35                  40                  45
   aac gcg ccc aac tat cgc ttc gtc cat gcc gac atc gcc gac acc gcg
   192
   Asn Ala Pro Asn Tyr Arg Phe Val His Ala Asp Ile Ala Asp Thr Ala
       50                  55                  60
   acg atc cta ccg ctg ctg cgc gag gag cag gtc gat gtg gtg atg cac
   240
   Thr Ile Leu Pro Leu Leu Arg Glu Glu Gln Val Asp Val Val Met His
    65                  70                  75                  80
   ctc gcc gcc gag agc cat gtc gat cgc tcg atc gac ggc cct ggc gag
   288
   Leu Ala Ala Glu Ser His Val Asp Arg Ser Ile Asp Gly Pro Gly Glu
                    85                  90                  95
   ttc atc gag acc aat gtc gtc ggc acc ttc aag ctg ctc cag tcg gcg
   336
   Phe Ile Glu Thr Asn Val Val Gly Thr Phe Lys Leu Leu Gln Ser Ala
               100                 105                 110
   ctg caa tat tgg cgc gag ctg gag ggc gag aaa cgc gac gcg ttc cgc
   384
   Leu Gln Tyr Trp Arg Glu Leu Glu Gly Glu Lys Arg Asp Ala Phe Arg
           115                 120                 125
   ttc cac cac atc tcc acc gac gaa gtg ttc ggc gac ctg ccg ttc gac
   432
   Phe His His Ile Ser Thr Asp Glu Val Phe Gly Asp Leu Pro Phe Asp
   130 135 140
   agc ggc atc ttc acc gaa gag acg ccc tat gat ccc tcc tcg ccc tat
   480
   Ser Gly Ile Phe Thr Glu Glu Thr Pro Tyr Asp Pro Ser Ser Pro Tyr
   145 150 155 160
   tcg gcg tcg aag gcg gcg agc gac cat ctg gtg cgc gcc tgg ggc cac
   528
   Ser Ala Ser Lys Ala Ala Ser Asp His Leu Val Arg Ala Trp Gly His
   165 170 175
   acc tat ggc ctg ccg gtg gtg ctg tcg aac tgc tcg aac aat tac ggg
   576
   Thr Tyr Gly Leu Pro Val Val Leu Ser Asn Cys Ser Asn Asn Tyr Gly
   180 185 190
   ccg ttc cac ttc ccc gag aag ctg atc ccg ttg acc atc ctc aac gcg
   624
   Pro Phe His Phe Pro Glu Lys Leu Ile Pro Leu Thr Ile Leu Asn Ala
   195 200 205
   ctc gag ggc aag ccg ctg ccg gtc tac ggc aag ggc gag aat atc cgc
   672
   Leu Glu Gly Lys Pro Leu Pro Val Tyr Gly Lys Gly Glu Asn Ile Arg
   210 215 220
   gac tgg ctg tat gtc gac gat cac gcc aag gcg ctg gcg acc atc gcc
   720
   Asp Trp Leu Tyr Val Asp Asp His Ala Lys Ala Leu Ala Thr Ile Ala
   225 230 235 240
   acc acc ggc aag gtc ggc cag agc tac aat gtc ggc ggc cgc aac gag
   768
   Thr Thr Gly Lys Val Gly Gln Ser Tyr Asn Val Gly Gly Arg Asn Glu
   245 250 255
   cgg acc aac ctg cag gtg gtc gag acg atc tgc gac ctg ctc gac cag
   816
   Arg Thr Asn Leu Gln Val Val Glu Thr Ile Cys Asp Leu Leu Asp Gln
   260 265 270
   cgc att ccg ctg gcc gac ggt cgc aag cgc cgc gaa ctg atc acc ttc
   864
   Arg Ile Pro Leu Ala Asp Gly Arg Lys Arg Arg Glu Leu Ile Thr Phe
   275 280 285
   gtc acc gat cgc ccc ggc cat gac cgc cgc tac gcg atc gac gcg acc
   912
   Val Thr Asp Arg Pro Gly His Asp Arg Arg Tyr Ala Ile Asp Ala Thr
   290 295 300
   aag ctc gag acc gag ctg ggc tgg aag gct gag gag aat ttc gac acc
   960
   Lys Leu Glu Thr Glu Leu Gly Trp Lys Ala Glu Glu Asn Phe Asp Thr
   305                 310                 315                 320
   ggc atc gcc gcg acg atc gac tgg tat ctg gcg aac gag tgg tgg tgg
   1008
   Gly Ile Ala Ala Thr Ile Asp Trp Tyr Leu Ala Asn Glu Trp Trp Trp
                   325                 330                 335
   ggc ccg atc cgc tcc ggc aaa tat gcc ggc gag cgg ctg ggg cag acc
   1056
   Gly Pro Ile Arg Ser Gly Lys Tyr Ala Gly Glu Arg Leu Gly Gln Thr
               340                 345                 350
   gcc tga
   1062
   Ala  *
<210> 41
   <211> 353
   <212> PRT
   <213> Sphingomonas sp. ATCC53159
<220>
   <221> PEPTIDE
   <222> (0) ... (0)
   <223> dTDP-D-glucose-4,6-dehydratase
<400> 41
   Met Gln Gln Thr Phe Leu Val Thr Gly Gly Ala Gly Phe Ile Gly Ser
    1               5                  10                  15
   Ala Val Val Arg His Leu Val Arg Gln Gly Ala Arg Val Ile Asn Leu
               20                  25                  30
   Asp Lys Leu Thr Tyr Ala Gly Asn Pro Ala Ser Leu Thr Ala Ile Glu
           35                  40                  45
   Asn Ala Pro Asn Tyr Arg Phe Val His Ala Asp Ile Ala Asp Thr Ala
       50                  55                  60
   Thr Ile Leu Pro Leu Leu Arg Glu Glu Gln Val Asp Val Val Met His
   65                  70                  75                  80
   Leu Ala Ala Glu Ser His Val Asp Arg Ser Ile Asp Gly Pro Gly Glu
                   85                  90                  95
   Phe Ile Glu Thr Asn Val Val Gly Thr Phe Lys Leu Leu Gln Ser Ala
               100                 105                 110
   Leu Gln Tyr Trp Arg Glu Leu Glu Gly Glu Lys Arg Asp Ala Phe Arg
           115                 120                 125
   Phe His His Ile Ser Thr Asp Glu Val Phe Gly Asp Leu Pro Phe Asp
       130                 135                 140
   Ser Gly Ile Phe Thr Glu Glu Thr Pro Tyr Asp Pro Ser Ser Pro Tyr
   145                 150                 155                 160
   Ser Ala Ser Lys Ala Ala Ser Asp His Leu Val Arg Ala Trp Gly His
                   165                 170                 175
   Thr Tyr Gly Leu Pro Val Val Leu Ser Asn Cys Ser Asn Asn Tyr Gly
               180                 185                 190
   Pro Phe His Phe Pro Glu Lys Leu Ile Pro Leu Thr Ile Leu Asn Ala
           195                 200                 205
   Leu Glu Gly Lys Pro Leu Pro Val Tyr Gly Lys Gly Glu Asn Ile Arg
       210                 215                 220
   Asp Trp Leu Tyr Val Asp Asp His Ala Lys Ala Leu Ala Thr Ile Ala
   225                 230                 235                 240
   Thr Thr Gly Lys Val Gly Gln Ser Tyr Asn Val Gly Gly Arg Asn Glu
                   245                 250                 255
   Arg Thr Asn Leu Gln Val Val Glu Thr Ile Cys Asp Leu Leu Asp Gln
               260                 265                 270
   Arg Ile Pro Leu Ala Asp Gly Arg Lys Arg Arg Glu Leu Ile Thr Phe
           275                 280                 285
   Val Thr Asp Arg Pro Gly His Asp Arg Arg Tyr Ala Ile Asp Ala Thr
       290                 295                 300
   Lys Leu Glu Thr Glu Leu Gly Trp Lys Ala Glu Glu Asn Phe Asp Thr
   305                 310                 315                 320
   Gly Ile Ala Ala Thr Ile Asp Trp Tyr Leu Ala Asn Glu Trp Trp Trp
                   325                 330                 335
   Gly Pro Ile Arg Ser Gly Lys Tyr Ala Gly Glu Arg Leu Gly Gln Thr
               340                 345                 350
   Ala
<210> 42
   <211> 867
   <212> DNA
   <213> Sphingomonas sp. ATCC53159
<220>
   <221> CDS
   <222> (1)...(867)
   <223> rmlD
<400> 42
   atg cgt atc ctc gtc acc ggg cat gac ggc cag gtc gcc cag tcg ctg
   48
   Met Arg Ile Leu Val Thr Gly His Asp Gly Gln Val Ala Gln Ser Leu
    1               5                  10                  15
   gcc gag cag gcg gtg ggc cac gag ctg gtc ttc acc acc tac ccc gaa
   96
   Ala Glu Gln Ala Val Gly His Glu Leu Val Phe Thr Thr Tyr Pro Glu
               20                  25                  30
   ttc gat ctc tcc aag ccg gag acg atc gag gcc ggt gtg gcg cgg gtg
   144
   Phe Asp Leu Ser Lys Pro Glu Thr Ile Glu Ala Gly Val Ala Arg Val
           35                  40                  45
   cac ccg gac ctg atc gtc tcc gcc gcc gcc tac acg gcg gtc gac aag
   192
   His Pro Asp Leu Ile Val Ser Ala Ala Ala Tyr Thr Ala Val Asp Lys
       50                  55                  60
   gcg gaa agc gaa ccc gag ctg gcg atg gcg atc aac ggc gac ggt ccc
   240
   Ala Glu Ser Glu Pro Glu Leu Ala Met Ala Ile Asn Gly Asp Gly Pro
    65                  70                  75                  80
   ggc gtg ctg gcg cgc gcg ggc gcg aag atc ggc gcg ccg atc atc cac
   288
   Gly Val Leu Ala Arg Ala Gly Ala Lys Ile Gly Ala Pro Ile Ile His
                   85                  90                  95
   ctg tcg acc gat tat gtg ttc gac ggc agt ctc gac cgc cct tgg cgc
   336
   Leu Ser Thr Asp Tyr Val Phe Asp Gly Ser Leu Asp Arg Pro Trp Arg
               100                 105                 110
   gag gac gat ccc acc ggc ccg ctc ggc gtc tat ggc gcg acc aag ctg
   384
   Glu Asp Asp Pro Thr Gly Pro Leu Gly Val Tyr Gly Ala Thr Lys Leu
           115                 120                 125
   gcc ggc gag cag gcg gtg cag gcc tcg ggt gcc acc aac gcc gtg atc
   432
   Ala Gly Glu Gln Ala Val Gln Ala Ser Gly Ala Thr Asn Ala Val Ile
       130                 135                 140
   cgg ctg gcc tgg gtc tac agc ccg ttc ggc aac aat ttc gtc aag acg
   480
   Arg Leu Ala Trp Val Tyr Ser Pro Phe Gly Asn Asn Phe Val Lys Thr
   145                 150                 155                 160
   atg ctc cgc ctc gcc gag acg cgc gac gcg ctg aac gtc gtg gag gac
   528
   Met Leu Arg Leu Ala Glu Thr Arg Asp Ala Leu Asn Val Val Glu Asp
                   165                 170                 175
   cag tgg ggc tgc ccc agt tcg gcg ctg gac atc gcg acc gcg atc ctg
   576
   Gln Trp Gly Cys Pro Ser Ser Ala Leu Asp Ile Ala Thr Ala Ile Leu
               180                 185                 190
   acg gtg gtc ggg cac tgg cag cag gac ggc gcg acg agc ggc ctc tac
   624
   Thr Val Val Gly His Trp Gln Gln Asp Gly Ala Thr Ser Gly Leu Tyr
           195                 200                 205
   cat ttc gcc ggc acc ggc gag acc aac tgg gcc gac ttc gca tcg acg
   672
   His Phe Ala Gly Thr Gly Glu Thr Asn Trp Ala Asp Phe Ala Ser Thr
       210                 215                 220
   atc ttc gcc gag agc gcc aag cgc ggt ggc ccc tcg gcc acc gtc acc
   720
   Ile Phe Ala Glu Ser Ala Lys Arg Gly Gly Pro Ser Ala Thr Val Thr
   225                 230                 235                 240
   ggc att ccc agc tcg ggc tat ccg act ccg gcc acg cgc ccg gcc aat
   768
   Gly Ile Pro Ser Ser Gly Tyr Pro Thr Pro Ala Thr Arg Pro Ala Asn
                   245                 250                 255
   tcg cgg ctg gac tgc acc cgc ttc gcg gag acc ttc ggc tac cgg gcg
   816
   Ser Arg Leu Asp Cys Thr Arg Phe Ala Glu Thr Phe Gly Tyr Arg Ala
               260                 265                 270
   cct gcc tgg cag gat tcg ctg aac gtc gta ctg gat cgc ctg ctc ggc
   864
   Pro Ala Trp Gln Asp Ser Leu Asn Val Val Leu Asp Arg Leu Leu Gly
           275                 280                 285
   tga
   867
    *
<210> 43
   <211> 288
   <212> PRT
   <213> Sphingomonas sp. ATCC53159
<220>
   <221> PEPTIDE
   <222> (0)...(0)
   <223> dTDP-6-deoxy-L-mannose-dehydrogenase
<400> 43
   Met Arg Ile Leu Val Thr Gly His Asp Gly Gln Val Ala Gln Ser Leu
    1               5                  10                  15
   Ala Glu Gln Ala Val Gly His Glu Leu Val Phe Thr Thr Tyr Pro Glu
               20                  25                  30
   Phe Asp Leu Ser Lys Pro Glu Thr Ile Glu Ala Gly Val Ala Arg Val
           35                  40                  45
   His Pro Asp Leu Ile Val Ser Ala Ala Ala Tyr Thr Ala Val Asp Lys
       50                  55                  60
   Ala Glu Ser Glu Pro Glu Leu Ala Met Ala Ile Asn Gly Asp Gly Pro
   65                  70                  75                  80
   Gly Val Leu Ala Arg Ala Gly Ala Lys Ile Gly Ala Pro Ile Ile His
                   85                  90                  95
   Leu Ser Thr Asp Tyr Val Phe Asp Gly Ser Leu Asp Arg Pro Trp Arg
               100                 105                 110
   Glu Asp Asp Pro Thr Gly Pro Leu Gly Val Tyr Gly Ala Thr Lys Leu
           115                 120                 125
   Ala Gly Glu Gln Ala Val Gln Ala Ser Gly Ala Thr Asn Ala Val Ile
       130                 135                 140
   Arg Leu Ala Trp Val Tyr Ser Pro Phe Gly Asn Asn Phe Val Lys Thr
   145                 150                 155                 160
   Met Leu Arg Leu Ala Glu Thr Arg Asp Ala Leu Asn Val Val Glu Asp
                   165                 170                 175
   Gln Trp Gly Cys Pro Ser Ser Ala Leu Asp Ile Ala Thr Ala Ile Leu
               180                 185                 190
   Thr Val Val Gly His Trp Gln Gln Asp Gly Ala Thr Ser Gly Leu Tyr
           195                 200                 205
   His Phe Ala Gly Thr Gly Glu Thr Asn Trp Ala Asp Phe Ala Ser Thr
       210                 215                 220
   Ile Phe Ala Glu Ser Ala Lys Arg Gly Gly Pro Ser Ala Thr Val Thr
   225                 230                 235                 240
   Gly Ile Pro Ser Ser Gly Tyr Pro Thr Pro Ala Thr Arg Pro Ala Asn
                   245                 250                 255
   Ser Arg Leu Asp Cys Thr Arg Phe Ala Glu Thr Phe Gly Tyr Arg Ala
               260                 265                 270
   Pro Ala Trp Gln Asp Ser Leu Asn Val Val Leu Asp Arg Leu Leu Gly
           275                 280                 285
<210> 44
   <211> 132
   <212> DNA
   <213> Sphingomonas sp. ATCC53159
<220>
   <221> CDS
   <222> (1)...(132)
   <223> orf7 (partial); unknown function
<400> 2
<210> 45
   <211> 43
   <212> PRT
   <213> Sphingomonas sp. ATCC53159
<400> 3

## Description

### Field of the Invention

The present invention describes the production of a diutan polysaccharide exhibiting increased viscosity properties as compared with previously produced polysaccharide of the same type of repeating units. Such an improved diutan polysaccharide is produced through the generation of a derivative of *Sphingomonas sp.* ATCC 53159 that harbors a multicopy broad-host-range plasmid into which genes for biosynthesis of diutan polysaccharide have been cloned. The plasmid provides the capability within the host *Sphingomonas* strain to produce multiple copies of genes for such polysaccharide synthesis. In such a manner, a method of not just increased production of the target diutan polysaccharide, but also production of a diutan polysaccharide of improved physical properties (of the aforementioned higher viscosity) thereof is provided. Such a diutan polysaccharide has proven particularly useful as a possible viscosifier in oilfield applications and within cement materials. The inventive methods of production of such an improved diutan polysaccharide, as well as the novel cloned genes required to produce the improved diutan within such a method, are also encompassed within this invention. Additionally, the novel engineered *Sphingomonas* strain including the needed DNA sequence is encompassed within this invention.

### Background of the Invention

Polysaccharides or gums are primarily used to thicken or gel aqueous solutions and are frequently classified into two groups: thickeners and gelling agents. Typical thickeners include starches, xanthan gum, diutan gum, welan gum, guar gum, carboxymethylcellulose, alginate, methylcellulose, gum karaya and gum tragacanth. Common gelling agents include gelatin, gellan gum, starch, alginate, pectin, carrageenan, agar and methylcellulose.

Some polysaccharides, or more particularly stated, biogums, such as xanthan, gellan, welan and diutan have been produced via fermentation from microbes for many years. Such biogums exhibit varied characteristics such as viscosity modification capabilities that have permitted their utilization in many different applications. Included within such a list are gelling agents for foods, such as confectionery jellies, jams and jellies, dessert gels, icings and dairy products, as well as components of microbiological media. Furthermore, thickening agents are utilized for myriad end-use applications to modify the viscosity of target liquids. Of particular interest is the ability of such gums to impart viscosity modification to underground and/or underwater petroleum liquids to facilitate collection thereof, although many other different possible end-uses exist (including cement production, as one example). Different biogums have been produced from different bacterial sources, such as xanthan gum, from *Xanthomonas campestris*, gellan gum, from *Sphingomonas elodea,* welan gum from *Sphingomonas sp.* ATCC 31555, and diutan gum (S-657), from *Sphingomonas sp.* ATCC 53159. Genetic modifications of such strains have been undertaken in the past to effectuate significant changes in the resultant gum materials produced through the aforementioned fermentation procedures. Such modifications have permitted such changes as removal of acyl groups to create different gum materials exhibiting different physical properties. Generally, such genetic modifications have been of the type to either alter the composition of the target biogum ultimately through altered gene expression within the host organism, or increase the yield of the target biogum, through introduction of a plasmid that exhibits gene amplification alone (such as in U.S. Patent Nos. 5,854,034, 5,985,623, and 6,284,516, to Pollock et al. and U.S. Patent No. 6,709,845 to Pollock alone).

Diutan gum (also known as heterpolysaccharide S-657) is prepared by fermentation of strain *Sphingomonas sp.* ATCC 53159 and exhibits thickening, suspending, and stabilizing properties in aqueous solutions. Diutan generally exhibits a hexameric repeat unit consisting of four sugars in the backbone (glucose-glucuronic acid-glucose-rhamnose) and a side chain of two rhamnose residues attached to one of the glucose residues. Details of the diutan gum structure may be found in an article by Chowdhury, T. A., B. Lindberg, U. Lindquist and J. Baird, Carbohydrate Research 164 (1987) 117-122. Diutan was shown to have two acetyl substituents per repeat unit within Diltz et al., Carbohydrate Research 331 (2001) 265-270. Both of these references are hereby incorporated by reference in their entirety. Details of preparing diutan gum may be found in U.S. Pat. No. 5,175,278, which is hereby incorporated by reference in its entirety. Diutan may be produced from the *Sphingomonas* strain by utilizing standard fermentation techniques such as using carbohydrate sources (glucose, maltose, and the like, as non-limiting examples), a nitrogen source, and additional salts.

The physical characteristics imparted by such a diutan biogum in its wild-type form are desired by certain industries, particularly in terms of its viscosity modification properties and/or water retention characteristics. Unfortunately, diutan has proven difficult to produce cost effectively. Furthermore, such cost issues militate against widespread utilization of diutan currently since the degree of viscosity exhibited by such a biogum is insufficient to supplant other less expensive, but effective, biogums (such as xanthan gum, as one example). As such, it has been an established need to provide a method to produce such an effective diutan at lower cost, at the very least, and/or to provide a manner of producing a biogum of the diutan type that exhibits a significant improvement in physical properties as well. To date, the only mention of production of any types of related sphingans (without any demonstrations for diutan specifically) is in terms of higher yield (within the Pollock et al. patents mentioned above). There has been no discussion or fair suggestion of any manner of providing a method for producing an improved diutan gum of higher molecular weight that exhibits any improvement in viscosity measurements via such a production method.

### Brief Description of the Invention

It has now been realized that amplification of certain novel isolated DNA sequences for diutan biosynthesis within a host *Sphingomonas* organism not only permits increased production of diutan gum therefrom, but also produces a diutan gum that exhibits increased viscosity properties. Such a novel DNA sequence (that is introduced within a host organism via any well known method, such as, without limitation, a plasmid) thus provides the desired results that have been sought after for diutan synthesis methods. A distinct advantage of such utilization of these genes amplified on a plasmid is the relatively simple nature of incorporating such an isolated DNA sequence into diutan synthesis procedures. Another advantage is the ability to produce such higher viscosity properties for the target diutan gum, while potentially increasing the fermentation production efficiency, if necessary.

Accordingly, this invention includes a diutan gum exhibiting an improvement in a number of different viscosity measurements as claimed in claim 1. Among these are: i) an intrinsic viscosity of greater than 150, preferably higher than 155, more preferably higher than 160dL/g; ii) a sea water 3 rpm viscosity greater than 35, preferably higher than 37, more preferably higher than 40, and most preferably higher than 42 dial reading; iii) a sea water 0.3 rpm viscosity greater than 35,000, preferably higher than 39,000, more preferably higher than 40,000, and most preferably higher than 41,000 centipoise (cP); and a PEG low shear rate viscosity greater than 3500, preferably higher than 3700, more preferably higher than 3900, and most preferably higher than 4000 cP. Also, this invention encompasses a method of producing such a diutan gum, through the introduction of a specific cluster of genes into a host *Sphingomonas* organism and permitting fermentation of said organism to produce a resultant diutan gum.

Furthermore, this invention encompasses the specific DNA sequence according to SEQ ID No.1 to provide multiple copies of the genes or increased expression of the genes by use of a stronger promoter, and the like.

Such a unique isolated DNA sequence has been found to require at least one diutan biosynthetic enzyme being a DpsG polymerase. In another possible embodiment, such a diutan biosynthetic enzyme will include a DpsG polymerase and a glucose-1-phosphate thymidylyltransferase; a dTDP-6-deoxy-D-glucose-3-5-epimerase; a dTDP-D-glucose-4,6-dehydratase; and a dTDP-6-deoxy-L-mannose-dehydrogenase. In yet another possible embodiment such a diutan biosynthetic enzyme will include a DpsG polymerase and a rhamnosyl transferase IV; a beta-1,4-glucuronosyl transferase II; a glucosyl isoprenylphoaphate transferase I; and a glucosyl transferase III. In still another possible embodiment, such a diutan biosynthetic enzyme comprises a dpsG polymerase and polysaccharide export proteins dpsD, dpsC, and dpsE. In yet another possible embodiment, such a diutan biosynthetic enzyme will include a rhamnosyl transferase IV; a beta-1,4-glucuronosyl transferase II; a glucosyl isoprenylphoaphate transferase I; glucosyl transferase III; a glucose-1-phosphate thymidylyltransferase; a dTDP-6-deoxy-D-glucose-3-5-epimerase; a dTDP-D-glucose-4,6-dehydratase; and a dTDP-6-deoxy-L-mannose-dehydrogenase. Generally, the diutan biosynthetic enzyme of the inventive method and within the inventive product may be selected from the group consisting of polymerase; lyase; rhamnosyl transferase IV; beta-1,4-glucuronosyl transferase II; glucosyl transferase III; polysaccharide export protein; secretion protein; glucosyl-isoprenylphosphate transferase I; glucose-1-phosphate thymidylyltransferase; dTDP-6-deoxy-D-glucose-3-5-epimerase; dTDP-D-glucose-4,6-dehydratase; dTDP-6-deoxy-L-mannose-dehydrogenase and combinations thereof. Further encompassed within this invention then is an isolated nucleic acid molecule (in addition to DNA which may be present on the target chromosome) which encodes at least one diutan biosynthetic enzyme as shown in SEQ ID NO: 5, 7, 9, 11, 13, 15, 17, 19, 21, 23, 25, 27, 29, 31, 33, 35, 37, 39, 41, and 43, or an enzyme which is at least 95 % identical to SEQ ID NO: 5, 7, 9, 11, 13, 15, 17, 19, 21, 23, 25, 27, 29, 31, 33, 35, 37, 39, 41, and 43.

The inventive method (as well as the products made thereby) thus concern sphingan gums, particularly diutan types, including, without limitation, S88, S60, and S657.

As noted above, the present invention is the culmination of development and realization that specific DNA sequences that are introduced in multiple copies within certain *Sphingomonas* strains can provide increased biosynthetic production of high viscosity diutan polysaccharide. The engineered bacteria containing such genes for increased production produce significantly greater amounts of diutan polysaccharide compared to non-engineered bacteria and create the aforementioned resultant high viscosity properties.

The DNA sequences that are introduced within the host organism (in any well known form, such as, again, as one non-limiting example, a plasmid) to generate the aforementioned increased production and increased viscosity properties (through what is believed, without any reliance upon any specific scientific theory, an increase in molecular weight range properties) according to the present invention may be isolated, recovered and cloned by techniques that are readily available in the art. Thereafter, the DNA is delivered into bacteria of the genus *Sphingomonas* in multiple copies (via plasmid, other known manner) or increased expression of the genes via a suitable, e.g., stronger promoter. After insertion into the target bacteria, the production of diutan can be determined by fermenting the engineered bacteria and comparing the yield in terms of amount produced and quality produced. Increased production and viscosity increases can both be determined by comparing diutan production via the inventive method in comparison with the wild type diutan-producing strain (ATCC 53159).

### Detailed Description of the Invention

The following terms shall be used throughout the specification in connection with the present invention and have the meaning indicated:
The term *"Sphingomonas"* is used throughout the specification to refer to strains of gram-negative bacteria from the genus *Sphingomonas.*

The term "increased producer" or "increased production" is used throughout the specification to describe engineered bacteria containing multiple copies of DNA sequences isolated from the same strain which produce significantly greater (at least about 5% more on a weight by weight basis) diutan polysaccharide compared to wild-type bacteria of the same strain.

The term "isolated" is used to describe DNA which has been removed from a microorganism and subjected to at least some degree of purification, i.e., one or more purification steps, and which can be cleaved or cut by restriction enzymes, cloned into multiple copies or inserted into plasmid vectors or otherwise inserted or incorporated into bacteria.

The term "sequence" is used to describe a specific segment of DNA which is identified by its nucleotide units. The term "inserted" is used throughout the specification to describe the process and outcome of transferring DNA segments isolated from the chromosomal DNA of a diutan-producing *Sphingomonas* strain into the *Sphingomonas* strain (via a plasmid, as one non-limiting example). Such isolated DNA may be introduced first into, again as one non-limiting possibility, the desired plasmid (here pLAFR3), by well-known techniques in the art, and then transferred, for example, by conjugation or mobilization into a recipient *Sphingomonas* bacterium. After insertion into a recipient *Sphingomonas* bacterium, the plasmid containing the relevant DNA sequence will replicate in the recipient cell to give several (at least two and usually 4-10) copies of the DNA segment necessary for increased production of high viscosity (again, believed to be high molecular weight range) diutan polysaccharide. The use of conjugation or mobilization to transfer the plasmid vectors into recipient bacteria is generally effective. Electroporation or chemical transformation of competent cells with purified DNA may also be used. Other vectors or bacteriophages can be used to transfer DNA into the host cell. Maintaining the DNA segments on plasmids (or other well known delivery vectors) in the recipient diutan-producing *Sphingomonas* is not necessary. It is routine to introduce additional copies of a DNA segment into the bacterial chromosome so that the segments are replicated each generation by the same mechanism that replicates the bacterial DNA. Alternatively, increased expression of the genes may be achieved by using stronger promoter elements.

The term "gene amplification" is used to refer to either increased copies of genes, for example by cloning the target genes on a multicopy plasmid (such as from 4 to 10 copies) or insertion of multiple copies (such as from 4 to 10) of the genes into the bacterial genome, or alternatively increased expression of genes by modification of promoter elements to increase gene expression. Both of these methods and others can result in increased amounts of the encoded proteins.

The term "biosynthesis" is used throughout the specification to describe the biological production or synthesis of diutan by *Sphingomonas* bacteria. Diutan polysaccharide is synthesized from individual carbohydrate units in a series of steps controlled by a large number of enzymes of the bacteria.

The relevant DNA sequence which is incorporated into the recipient bacteria in any selected form (such as, again, preferably, but not necessarily, plasmid form) encodes genetic information which is known to be beneficial or essential for the biosynthesis of increased production and increased molecular weight diutan polysaccharide. Additionally, though, the particular inventive DNA sequence (such as within plasmid pS8) is believed, without relying on a specific scientific theory, to induce, not just increased production, but also an increase in number of repeating units polymerized within the individual polymers of the diutan itself. As a result, it is believed that such an increase in repeating units produces the resultant high viscosity properties surprisingly provided by the diutan gum. A molecular weight increase has been hypothesized due to measured increases in intrinsic viscosity which is related to molecular weight by a power law relationship. For a linear polymer (like diutan gum), intrinsic viscosity is thus known to be essentially proportional to molecular weight in that respect.

The isolation of the relevant DNA sequences that are the basis of this inventive method and that generate the increased viscosity diutan polysaccharide is accomplished via standard techniques and methods. Such sequences may thus be generated from a diutan-producing *Sphingomonas* strain that has been cultured using standard procedures. Extraction of the DNA can then be performed, for example, through initial centrifugation and resuspension of the bacterial cells and then subsequent elution of the DNA through purification columns. After purification is completed, the isolated DNA can be digested with restriction endonucleases and cloned into the desired plasmid or other delivery vector and subsequently transferred to a recipient strain. Other techniques as are known in the art can be used without limitation.

The cloning of DNA in the present invention relies on general techniques and methods which have become standard in the art. It is noted that any number of methods may be used to clone the DNA segments according to the present invention and the present invention is not limited, for example, to the use of plasmid cloning vectors. For example, the DNA fragments may be cloned by insertion into a bacteriophage vector.

The cloned DNA sequences can be then introduced to a *Sphingomonas* strain via a plasmid or other delivery vector. The genetically modified *Sphingomonas* strain can then be used to produce diutan by fermentation. Basically, a suitable medium for fermentation is an aqueous medium which generally contains a source of carbon such as, for example, carbohydrates including glucose, lactose, sucrose, maltose or maltodextrins, a nitrogen source such as, for example, inorganic ammonium, inorganic nitrate, organic amino acids or proteinaceous materials such as hydrolyzed yeast, soy flour or casein, distiller's solubles or corn steep liquor, and inorganic salts. A wide variety of fermentation media will support the production of diutans according to the present invention.

Carbohydrates can be included in the fermentation broth in varying amounts but usually between about 1 and 10% by weight (preferably 2-8%) of the fermentation medium. The carbohydrates may be added prior to fermentation or alternatively, during fermentation. The amount of nitrogen may range from about 0.01% to about 0.4% by weight of the aqueous medium. A single carbon source or nitrogen source may be used, as well as mixtures of these sources. Among the inorganic salts which find use in fermenting *Sphingomonas* bacteria are salts which contain sodium, potassium, ammonium, nitrate, calcium, phosphate, sulfate, chloride, carbonate and similar ions. Trace metals such as magnesium, manganese, cobalt, iron, zinc, copper, molybdenum, iodide and borate may also be advantageously included.

The fermentation can be carried out at temperatures between about 25° and 40°C, with a temperature range of about 27° and 35°C preferred. The inoculum can be prepared by standard methods of volume scale-up, including shake flask cultures and small-scale submerged stirred fermentation. The medium for preparing the inoculum can be the same as the production medium or can be any one of several standard media well-known in the art, such as Luria broth or YM medium. More than one seed stage may be used to obtain the desired volume for inoculation. Typical inoculation volumes range from about 0.5% to about 10% of the total final fermentation volume.

The fermentation vessel may contain an agitator to stir the contents. The vessel also may have automatic pH and foaming controls. The production medium can be added to the vessel and sterilized in place by heating. Alternatively, the carbohydrate or carbon source may be sterilized separately before addition. A previously grown seed culture can be added to the cooled medium (generally, at the preferred fermentation temperature of about 27° to about 35°C) and the stirred culture can be fermented for about 48 to about 110 hours, producing a high viscosity broth. The diutan polysaccharide can be recovered from the broth by the standard method of precipitation with an alcohol, generally isopropanol.

### Preferred Embodiments of the Invention

### Including Detailed Descriptions of the Drawings

The following examples are provided to illustrate the present invention. The description of the examples should not be misconstrued to limit the scope of the present invention in any way.

### DNA Sequence Isolation/Plasmid Production

To undergo the initial isolation and determine the proper sequence for the inventive results described previously, a gene library of the ATCC 53159 organism was constructed as follows: Chromosomal DNA was isolated from *Sphingomonas sp.* ATCC 53159 and partially digested with Sau3AI restriction endonuclease. DNA fragments in the range of 15 to 50 kb were purified from an agarose gel and ligated into *Bam*HI digested cosmid cloning vector pLAFR3 (in accordance with Staskawicz, et al., "Molecular characterization of cloned avirulence genes from race 0 and race 1 of Pseudomonas syrinae pv. Glycinea", J. Bacteriology. 1987. 169: 5789-94), isolated from *Eschericia coli* strain JZ279 (from Harding, et al., "Genetic and physical analysis of a cluster of genes essential for xanthan gum biosynthesis in Xanthomonas campestris", J. Bacteriology. 1987. 169: 2854-61). Ligation reactions were packaged in λ phage particles (using Gigapack III Gold packaging extract, from Stratagene, La Jolla, CA) and transfected into Library Efficiency *E. coli* DH5αMCR cells (Life Technologies, Rockville, MD). Approximately 10,000 tetracycline resistant colonies were pooled to form the gene library. From this library, individual sequences were then isolated. The work undertaken in this instance involved the isolation of specific genes for polysaccharide biosynthesis from the *Sphingomonas* ATCC 53159 organism.

Such genes for polysaccharide biosynthesis are typically identified by complementation of mutants defective in polysaccharide synthesis, particularly those blocked in the first step, glycosyl transferase I. Since initially no transferase I defective mutants of ATCC 53159 were available, complementation of transferase I defective mutants of *Sphingomonas elodea* and *Xanthomonas campestris* were utilized to identify genes for diutan polysaccharide biosynthesis. Plasmid pLAFR3 can be transferred from its *E. coli* host to other gram-negative bacteria by tri-parental conjugation using a helper plasmid that supplies IncP transfer functions (in accordance with Ditta, et al., "Broad host range DNA cloning system for gram-negative bacteria: construction of a gene bank of Rhizobium meliloti", Proc. Natl. Acad. Sci. 1980. 77:7347-51.). RK2 type plasmids have an estimated copy number in *E. coli* of five to seven per chromosome (Figurski et al., "Suppression of ColEl replication properties by the Inc P-1 plasmid RK2 in hybrid plasmids constructed in vitro", J. Mol. Biol. 1979 133: 295-318.).

The gene library of ATCC 53159 chromosomal DNA in *E. coli* was transferred into a nonmucoid mutant (GPS2) of *S. elodea* ATCC 31461, by triparental conjugation, selecting for tetracycline and streptomycin resistance. The helper plasmid used was pRK2013 (in *E. coli* strain JZ279), which contains a narrow-host-range origin of replication but exhibits trans acting functions needed to mobilize pLAFR3. Plasmid pRK2013 was not replicated in *Sphingomonas* strains. *S. elodea* ATCC 31461 produces the polysaccharide gellan. Both gellan and diutan polysaccharides have the same tetrasaccharide repeat unit, comprised of [→4)-α-L-rhamnose-(1→3)-β-D-glucose-(1→4)-β-D-glucuronic acid-(1→4)-β-D-glucose-(1→]. Diutan, however, also includes a side chain comprised of two rhamnose molecules attached to one of the glucose residues, and is modified by acetyl, whereas gellan has no side chain sugars and is modified with acetyl and glyceryl. The mutant GPS2 is defective in the first step of polysaccharide biosynthesis, i.e., transfer of glucose-1-phosphate from UDP-D-glucose to the bactoprenyl phosphate lipid carrier by glucosyl transferase I enzyme. From tetracycline selection plates, polysaccharide-producing (mucoid) colonies were isolated from a background of non-mucoid colonies. Clones restoring polysaccharide production presumably contained the ATCC 53159 gene encoding glucosyl transferase I plus approximately 20-25 kb of adjacent DNA. Plasmid DNA was isolated from eight mucoid GPS2 transconjugants and transferred to *E. coli* strain DH5α (Life Technologies) by electroporation. The plasmids were isolated from *E. coli* to obtain sufficient DNA for double-digestion with restriction endonucleases *Hind*III/*Eco*RI (which cut either side of the *Bam*HI restriction endonuclease site in the polylinker), to excise the insert DNA from the vector. The sizes of the insert DNA in the clones were determined by gel electrophoresis. The end sequences of several plasmids were determined by sequencing from primers specific to plasmid sequences flanking the *Bam*HI site of the vector. The sequences were analyzed by comparison to sequences in computer databases using BLASTX. Two of these plasmids, pS8 and pS6, are presented in Fig. 1. Similarly, the ATCC 53159 gene library was transferred into a rifampicin-resistant nonmucoid *X. campestris* mutant defective in transferase I (CXC109)(such as in the Harding et al. reference noted above) through triparental conjugation selecting for resistance to tetracycline and rifampicin. *X. campestris* produces xanthan polysaccharide, the synthesis of which is also initiated by transfer of glucose-1-phosphate from UDP-D-glucose to the bactoprenyl phosphate lipid carrier by transferase I enzyme (Ielpi et al., "Sequential assembly and polymerization of the polyprenol-linked pentasaccharide repeating unit of the xanthan polysaccharide in Xanthomonas campestris", J. Bacteriology. 1993. 175: 2490-500). Plasmids were purified from mucoid transconjugants and the end sequences determined as described above. Two of these plasmids pX6 and pX4 are presented in Fig.1.

The S657 DNA cloned in plasmids pS8 and pX6 was completely sequenced by double-stranded shotgun sequencing at Lark Technologies Inc., (Houston, TX). These sequences were analyzed to identify the genes for diutan biosynthesis (presented in Figure 1). Gene functions were designated based on homology to other genes in databases, in particular to the published genes for biosynthesis of S-88 sphingan (such as within the aforementioned '516 Pollock et al. patent), GenBank accession number U51197 and gellan (GenBank AY217008 and AY220099). Genes were identified (Figure 1) that encoded the transferases for the four sugars of the backbone and four genes for dTDP-rhamnose synthesis. Genes for secretion of the polysaccharide were based on homology to genes for biosynthesis of other polysaccharides. Two genes encode proteins homologous to proteins involved in protein secretion. Two genes putatively encode a polymerase and a lyase. The insert in plasmid pX6 contained 17 genes including gene *dpsB* encoding transferase I (which initiates the first step in diutan synthesis), genes for secretion and four genes for dTDP-rhamnose synthesis, but lacks the genes for transferases II, III and IV and the putative genes for polymerase and lyase Plasmid pS8 contains 20 genes of the *dps* gene cluster, including genes for all four backbone sugar transferases, the four genes for dTDP-rhamnose synthesis, andgenes for secretion of the polysaccharide, including the putative genes for polymerase and lyase, but lacks the genes of unknown function, *orf6* and *orf7.* Plasmid pS6 contains genes for secretion and the four sugar transferases but does not have all genes for dTDP-rhamnose synthesis or the gene for polymerase. Plasmid pX4 contains only a small part of the *dps* region but includes the gene encoding transferase I and the four genes for dTDP-rhamnose synthesis that were reported by Pollock et al, to be sufficient to result in an increase in production of polysaccharide in *Sphingomonas* strains.

### Strain Production

The four plasmids described above were then introduced within *Sphingomonas* strain ATCC No. 53159 by triparental conjugation as described above to form the novel S657 engineered strains (S657/pS8, S657/pS6, S657/pX6 and S657/ρX4. Fermentation was followed, as described above, thereafter in order to produce a biogum material as noted below. All four plasmids had a beneficial effect on diutan productivity; however, the pS8 plasmid surprisingly also provided extremely large increases in diutan viscosity, and increase in molecular weight. The DNA sequence of pS8 (26278 bps)(DNA Sequence No. 1) is provided and the encoded genes are listed in Table 1 below, and in diagram form in Figure 1. The insert DNA in plasmid pS8 includes genes *dpsG* through *rmlD* and a portion of genes *dpsS* and *orf7.*

The following gene table is basically a list of the genes represented by the DNA sequence for insert in plasmid pS8.

**TABLE 1**

| *Genes on pS8 plasmid insert* | | | |
|---|---|---|---|
| **Start** | **End** | **Name** | **Description** |
| 2* | 1054 | dpsS | (partial) homologous to *gelS* |
| 2738 | 1113 C | dpsG | putative polymerase |
| 4895 | 2898 C | dpsR | putative lyase |
| 5093 | 6031 | dpsQ | putative rhamnosyl transferase IV |
| 7082 | 6111 C | dpsI | unknown |
| 7121 | 8167 | dpsK | beta-1,4-glucuronosyl transferase II |
| 8164 | 9030 | dpsL | glucosyl transferase III |
| 10467 | 9079 C | dpsJ | unknown |
| 11076 | 12374 | dpsF | unknown |
| 12389 | 13306 | dpsD | putative polysaccharide export protein |
| 13341 | 14687 | dpsC | putative polysaccharide export protein |
| 14687 | 15394 | dpsE | putative polysaccharide export protein |
| 15405 | 16286 | dpsM | putative polysaccharide export protein |
| 16270 | 16968 | dpsN | putative polysaccharide export protein |
| 18454 | 17060 C | atrD | putative secretion protein |
| 20637 | 18451 C | atrB | putative secretion protein |
| 21229 | 22641 | dpsB | glucosyl-isoprenylphosphate transferase I |
| 22757 | 23635 | rmlA | glucose-1-phosphate thymidylyltransferase |
| 23632 | 24198 | rmlC | dTDP-6-deoxy-D-glucose-3-5-epimerase |
| 24202 | 25263 | rmlB | dTDP-D-glucose-4,6-dehydratase |
| 25263 | 26129 | rmlD | dTDP-6-deoxy-L-mannose-dehydrogenase |
| 26277* | 26146 C | orf7 | (partial) unknown function |

| | | | |
|---|---|---|---|
| *First in-frame codon, the start codon is not present | | | |

### Diutan Production

Diutan production by the engineered plasmid-containing *Sphingomonas* S657 strains compared to the S657 wild-type strain without a plasmid was determined in three sets of fermentations run in the same liquid media in Applikon 20L fermentors, with agitation and aeration. For the plasmid containing strains, the antibiotic tetracycline at 5 mg/L was added throughout the fermentation to ensure retention of the plasmid. KOH was added as needed to control pH. Two seed stages were used with 1% to 6% inoculum transfers. Media used for fermentation contained corn syrup as carbohydrate source, an assimilable nitrogen source and salts. Nutrients that can be used for fermentation are well known in the art and include a carbohydrate, for example, glucose, sucrose, maltose or maltodextrins, a nitrogen source, for example inorganic nitrogen as ammonium or nitrate, organic nitrogen such as amino acids, hydrolyzed yeast extract, soy protein, or corn steep liquor, and additional salts containing for example, chloride, phosphate, sulfate, calcium, copper, iron, magnesium, potassium, sodium, or zinc.

As a measure of the resultant diutan production, broth viscosity and precipitated fibers were determined. The viscosity of the fermentation broths was measured via a Brookfield viscometer run at 60 rpm with a spindle #4, and the results are shown in Table 2. At the end of the fermentation, the broths were treated with the well known introduction of glucoamylase enzyme to hydrolyze any remaining oligosaccharides from the corn syrup. The diutan gums produced were then precipitated from an aliquot of broth with two volumes of isopropyl alcohol. The fibers were collected on a filter and dried. In Table 2, the term DWY means the total precipitable dry weight yields of biogums after hydrolysis of excess oligosaccharides from corn syrups

Clearly the resultant material is in higher yield with plasmids pX4, pX6, pS6 or pS8 carrying additional copies of genes for diutan biosynthesis present therein. However, with the pS8 plasmid, there was an unexpected high increase in broth viscosity relative to the increase in dry weight yield indicating that some factor in addition to increased amount of diutan produced was affecting the viscosity.

**TABLE 2**

| *Fermentation of plasmid-containing strains* | | | | | |
|---|---|---|---|---|---|
| **DWY** | | | | | |
| **Strain** | **Run #1** | **Run #2** | **Run#3** | **av.** | **% Increase** |
| S657 | 34.3 | 32.2 | 33.9 | 33.5 | ---- |
| S657/pS8 | 37.1 | 35.4 | 35.9 | 36.1 | 8.0% |
| S657/pX6 | 38.4 | 37.6 | 33.5 | 36.5 | 9.1% |
| S657/pS6 | | | 37.6 | | 12.3% |
| S657/pX4 | | | 36.4 | | 8.8% |

| **Broth Viscosity** | | | | | |
|---|---|---|---|---|---|
| **Strain** | **Run #1** | **Run #2** | **Run #3** | **av.** | **% Increase** |
| S657 | 5150 | 4950 | 5550 | 5217 | ---- |
| S657/pS8 | 6650 | 6850 | 6850 | 6783 | 30.0% |
| S657/pX6 | 5400 | 6250 | 5125 | 5592 | 7.2% |
| S657/pS6 | | | 6675 | | 28.0% |
| S657/pX4 | | | 5525 | | 5.9 |

Clearly, there was a higher yield of resultant material with any of the four plasmids present therein, whereas the pS8 and pS6 plasmids permitted a highly unexpected increase in broth viscosity thus indicating high product quality as well. The quality, i.e. viscosity, of the resultant diutan gum products was then determined.

### Diutan Rheology in Applications Tests

These diutan gum samples were then analyzed in terms of potential beneficial uses within two different areas: oilfield additives for oil recovery and cement additives for water retention and quick set-up.

The oilfield industry relies upon what is termed a "sea water viscosity" (SWV) test as an estimate of acceptable performance for gums for oil recovery. Such a test basically is an indicator of the effectiveness of a gum to increase viscosity in briny conditions of water (to replicate recovery from seabeds, for example).

The prediction of the viability of a resultant gum as a proper viscosity modifier for oil recovery purposes is generally accepted in terms of viscosity modification of a test sea water formulation. Such a "Synthetic Seawater" formulation is produced by mixing 419.53 grams of Sea Salt (ASTM D-1141-52) in 9800 grams deionized water. For the seawater viscosity test, 0.86 grams of the sample gum is added to 307.0 g Synthetic Seawater and mixed at approximately 11,500 rpm in a Fann Multimixer (Model 9B5, part number N5020) for 35 minutes. At the end of 35 minutes, the solution is cooled to approximately 26°C before the viscosity is measured. For the 3-rpm reading, the sample is placed on the Fann sample platform (Fann model 35A; Torsion spring MOC 34/35 F0.2b; Bob B1; Rotor R1) and the speed is adjusted to 3 rpm by turning the motor to low speed and setting the gearshift in the middle position. The reading is then allowed to stabilize and the shear stress value is read from the dial and recorded as the SWV 3 rpm dial reading (DR). For the 0.3-rpm reading, a Brookfield viscometer is used (Brookfield LV DV-II or DV-II viscometer, with LV-2C spindle) to measure the viscosity. The speed of the spindle is set to 0.3 rpm and the spindle is allowed to rotate at least 6 minutes before the viscosity is recorded as the SWV-0.3 rpm reading and expressed in centipoises (cP). For cement applications, the PEG LSRV test (a low shear rate viscosity using polyethylene glycol as dispersant as outlined below) provides an indication as to effectiveness of performance of a viscosity modifier to that industry. Such a test measures the viscosity of a 0.25% solution of biogum in Standard Tap Water (STW). STW is prepared by adding 10.0 grams NaCl and 1.47 grams CaCl₂ •2H₂O to 10 liters deionized water. For the viscosity measurement, 0.75 grams of biogum is added to 4.5 grams Polyethylene Glycol 200 (CAS 25322-68-3) in a 400-mL beaker and thoroughly dispersed. Then, 299 grams of STW are added to the beaker and mixed for approximately 4 hours using a low-pitched, propeller-style stirrer at 800 ± 20 rpm. After the 4-hr mixing time, the beaker is placed in a 25°C water bath and allowed to sit undisturbed for approximately 30 minutes. The viscosity is then measured using a Brookfield LV viscometer equipped with a 2.5+ torque spring (or equivalent instrument such as Model DVE 2.5+) at 3 rpm using the LV 1 spindle after allowing the spindle to rotate for 3 minutes and expressed in centipoises (cP).

The diutan samples produced above were tested in this manner; the results were as follows:

**TABLE 3**

| *Rheology of diutata from plasmid-containing strains* | | | | | | | | |
|---|---|---|---|---|---|---|---|---|
| **Strain** | **SWV 3 rpm DR** | | | **SWV-0.3 rpm cP** | | **PEG LSRV cP** | | |
| | **Run #1** | **Run #2** | **Run #3** | **Run #1** | **Run #2** | **Run #1** | **Run #2** | **Run #3** |
| S657 wild-type | 25 | 26 | 22 | 24400 | 28600 | 2820 | 3150 | 2280 |
| S657/pS8 | 42 | 43 | 47 | 41500 | 38800 | 4720 | 4980 | 4920 |
| S657/pX6 | 25 | 29 | 26 | 25000 | 29100 | 2860 | 3400 | 3270 |
| S657/pS6 | --- | --- | 22 | --- | --- | --- | --- | 2270 |
| S657/pX4 | --- | --- | 24.5 | --- | --- | --- | --- | 2950 |

| | | | | | | | | |
|---|---|---|---|---|---|---|---|---|
| SWV = viscosity in sea water LSRV = low shear rate viscosity | | | | | | | | |

Unexpectedly, there are definite increases in viscosity exhibited by the inventive diutan gums produced by some of the engineered plasmid-containing strains Most surprisingly, however, is that the increase in viscosity for SWV at 3 rpm for the pS8 strain is 80%, whereas the same analysis made for the pX6 strain is merely 9.6% over the wild-type results. Plasmids pS6 and pX4 had no significant increase. Likewise, the lower SWV rpm test reveals an increase of 51.5% over the wild-type for the pS8 type versus just over 2% for the pX6. Finally, the polyethylene glycol LSRV test showed that the pS8 results were in excess of 77% viscosity increase over the wild-type gum, as compared with less than 16% increase for the pX6 diutan, and 7.2% increase for pX4 and no significant increase for plasmid pS6. Again, the highly unexpected results in these terms shows the drastic improvements accorded diutan gum production via the utilization of the needed gene sequence exemplified within the pS8 plasmid, as one manner of introducing such a sequence within a target diutan-producing bacterium.

Thus, the inventive diutan produced via the introduction of pS8 exhibited surprisingly increased viscosity measurements on all three counts, particularly as compared with the wild type and pX6 plasmid-produced varieties. Thus, it was expected that such a novel diutan would function extremely well under typical oilfield conditions and within cement applications.

### Fundamental Explanation for Rheology Improvement

The previous examples showed that diutan from the S657/pS8 strain showed a significant increase in rheological parameters. Such a substantial increase in the sea water and PEG low shear rate viscosity measurements thus cannot be attributed to the increase in productivity alone since the pX6 strain also exhibited similar, if not greater, yield results. Indeed, in the prior example illustrated by Table 2, the dry weight yields (alcohol precipitable matter) increased by 8.0%, while the rheological parameters increased significantly more for the S657/pS8 strain (52-80%). A fundamental study was pursued to explain why rheological improvements are obtained with strain S657/pS8 over the wild-type strain.

Intrinsic viscosity is a well known technique in polymer science to infer the molecular weight of macromolecules (C. Tanford, 1961. Physical Chemistry of Macromolecules. John Wiley & Sons, New York). The intrinsic viscosity is obtained by plotting the reduced viscosity (viscosity normalized for concentration) versus the solution concentration, and extrapolating a linear regression of the data to zero concentration (the y-intercept of the plot). Surprisingly, the resultant gums exhibited increases in intrinsic viscosity as noted below in the following table.

Five diutan samples, two from the wild-type strain (Control 1, Control 2) and three from the S657/pS8 strain (Sample 1, Sample 2, Sample 3) were evaluated for intrinsic viscosity, neutral sugars, and organic acid analyses. These samples were purified by alcohol precipitation, re-hydrated, treated with hypochlorite, treated with glucoamylase, treated with lysozyme, and finally treated with protease (in that sequential order). They were then recovered at a 4:1 CBM:Broth ratio, dried and milled. CBM is an azeotropic isopropyl alcohol/water mixture including ∼82% by weight of the isopropyl alcohol.

The samples were tested for moisture content by performing the following: generally, two 0.7 gram aliquots of sample were tested using a Mettler HB 43 halogen moisture balance. The results from the two trials were then averaged and these results were utilized for moisture correction.

After obtaining the moisture data, a 0.2% solution of the gum was prepared in 0.01M NaCl on a moisture corrected basis. For these trials 200 grams total of the 0.2% solution were prepared. The gum was weighed on an analytical balance to the nearest ten thousandth and added to the water weighed to the nearest thousandth. The samples were stirred for two hours using a 2.5 inch diameter propeller mixer @ 1000 rpm in a 400 ml tall form beaker.

Following initial hydration, each sample was diluted to 0.02% using 0.01M NaCl. This was done by weighing 20 grams of the 0.2% solution into a 400 ml beaker, then adding back 180 mls of the diluent. The diluted samples were mixed for an additional 30 minutes. The final dilutions ultimately used for determining the intrinsic viscosity were prepared from this sample. Each diutan sample was evaluated at the following concentrations: 0.004%, 0.008%, 0.010%, and 0.012%.

Viscosity measurements were carried out using the Vilastic® VE System. Prior to measurements the Vilastic was calibrated with water to less than 2.0% error. The samples were measured using the Timer program @ 2 Hz, a strain of 1 and a shear rate of approximately 12 1/sec, all at a constant temperature of 23°C. Five measurements were made for each sample and averaged. The averaged viscosity data were then used to calculate the intrinsic viscosity. Table 4 below provides the final results of these trials.

**TABLE 4**

| *Comparison of Diutan Based on Intrinsic Viscosity Calculations* | | |
|---|---|---|
| Diutan Sample | Measured Solids | Intrinsic Viscosity |
| S657 Control1 | 93.76 | 138.3 |
| S657Control2 | 92.42 | 143 |
| S657/pS8 Sample 1 | 91.7 | 170.7 |
| S657/pS8 Sample 2 | 91.4 | 162.2 |
| S657/pS8 Sample 3 | 91.94 | 162.8 |

These results indicate that the S657/pS8 strain consistently produced diutan with significantly higher intrinsic viscosity; in fact the average reduced viscosity for the inventive strains was 165.2, whereas the control was 140.7, all at similar measured solids levels. This finding indicates that diutan produced by S657/pS8 is higher in molecular weight than the wild-type control.

To determine if the higher viscosity diutan gum from S657/pS8 had the same composition as diutan from the wild-type strain, the composition was determined by testing for neutral sugars and organic acids. The purified sample used for intrinsic viscosity measurements were used for neutral sugar analysis. An aliquot of each purified sample was hydrolyzed to component sugars by hydrolysis with trifluoroacetic acid (100°C/-18hr). The hydrolysate neutral sugars were quantified by high-performance anion-exchange chromatography with pulsed amperometric detection. The hydrolysate organic acids were quantified by high-performance ion-exclusion chromatography with chemically suppressed conductivity detection. Table 5 summarizes the results from the neutral sugar analysis. As shown, the neutral sugar profile for the S657/pS8 strain is nearly identical with the neutral sugar profile for the S657 wild-type strain. Although both results are different from the theoretical values, these results indicate that the structure of the repeat unit of the diutan gum produced using pS8 is the same as that for wild-type and that any increase in viscosity imparted by the pS8 material is due to longer chains, meaning higher molecular weight.

**TABLE 5**

| *Neutral sugars and organic acid analysis for pS8 and wildtype (control) diutan strains* | | | | |
|---|---|---|---|---|
| | **Strain** | **% Rhamnose** | **% Glucose** | **% Acetate** |
| Sample 1 | S657/pS8 | 32 | 19 | 8.9 |
| Sample 2 | S657/pS8 | 32 | 19 | 8.2 |
| Sample 3 | S657/pS8 | 32 | 17 | 8.6 |
| Control 1 | S657 wildtype | 30 | 18 | 8.6 |
| Control 1 | S657 wildtype | 33 | 20 | 8.7 |
| AVERAGE | S657/pS8 | 32 | 18.3 | 8.6 |
| AVERAGE | S657 wildtype | 31.5 | 19 | 8.65 |
| THEORETICAL | --- | 46 | 30 | 8 |

The greatly improved seawater viscosity and PEG low shear rate viscosity of the diutan produced by the S657/pS8 engineered strain is thus attributable to an increase in molecular weight or length of the diutan molecule, i.e., more repeat units per molecule and not to a change in its compositon and thus not to changes in the repeat structure itself. Nor can this improved rheology be due soley to increase in amount of diutan produced. Although four plasmids, pS6, pS8, pX4, and pX6, with different portions of the cluster of genes for diutan biosynthesis cloned, were evaluated, and all showed some increase in productivity, only plasmid pS8 showed the unexpected and very high increase in rheological parameters of the recovered diutan product.

A comparison of the genes for diutan biosynthesis cloned in the tested plasmids suggests that the most likely gene to be responsible for the increase in molecular weight is the gene *dpsG,* since this gene is present in pS8 and not in the other plasmids. Gene *dpsG* encodes a hydrophobic membrane protein with strong homology to other membrane proteins involved in polysaccharide synthesis. A portion of the protein has homology to proteins for polymerase, an enzyme which catalyzes the linkage of repeat units to form the high molecular weight polysaccharide. The homologous gene *gelG* in S60 has been postulated to function as a polymerase for gellan synthesis (Harding, N. E. et al. 2004. "Organization of genes required for gellan polysaccharide biosynthesis in Sphingomonas elodea ATCC31461". J. Ind. Microbiol. Biotech. 31:70-82. Sa-Correia, I. et al. 2002. "Gellan gum biosynthesis in Sphingomonas paucimobilis ATCC 31461: Genes, enzymes and exopolysaccharide production engineering". J. Ind. Microbiol. Biotechnol. 29: 170-176.). Homologues of *dpsG* have also been isolated from *Sphingomonas* strains ATCC 31554 and ATCC 21423 producing polysaccharides S88 and S7 (Pollock et al. U.S. Patent Nos. 5,854,034, 5,985,623 and 6,284,516, and Pollock, T. J. U.S. Patent No. 6,709,845). It is thus very likely that additional copies of the gene for polymerase may have an effect on increasing the molecular length of the diutan molecule. It cannot be ruled out that other genes in the diutan biosynthetic gene cluster may be required in combination with *dpsG* to achieve the observed increase in viscosity. Likely candidates would be the genes *dpsB, dpsL, dpsK* and *dpsQ* encoding the sugar transferases I, II, III, and IV, in particular the gene *dpsB* which encodes transferase I that adds the first sugar of the repeat unit to the lipid carrier. Other important genes may be *dpsD, dpsC* and *dpsE,* which are homologous to genes *gumB* and *gumC* that have been shown to increase the molecular weight of xanthan when amplified on a multicopy plasmid. It is possible that all genes cloned in plasmid pS8 may be required to achieve the dramatic increase in viscosity.

While the invention will be described and disclosed in connection with certain preferred embodiments and practices, it is in no way intended to limit the invention to those specific embodiments, rather it is intended to cover structural equivalents and all alternative embodiments and modifications as may be defined by the scope of the appended claims and equivalence thereto.

### Deposits

The following bacterial strain was deposited with the Patent Depository at the American Type Culture Collection at 10801 University Boulevard, Manassas, Va. 20110, on October 21, 2005, pursuant to the Budapest Treaty for the International Recognition of the Deposit of Microrganisms:
*Sphingomonas* strain S657 with plasmid pS8.

## Claims

1. A diutan gum exhibiting an intrinsic viscosity of greater than 150 deciL/g when intrinsic viscosity is obtained by plotting the reduced viscosity versus the solution concentration, and extrapolating a linear regression of the data to zero concentration,
and exhibiting a sea water 3 rpm viscosity greater than or equal to 42 dial reading when the reading is taken by combining sample gum with synthetic seawater and mixing, then cooling the solution to approximately 26°C and placing on a Fann sample platform where the speed is adjusted to 3rpm and reading from the dial; and
wherein the diutan gum is made using *Sphingomonas* strain ATCC No.53159 comprising a plasmid comprising a nucleic acid sequence according to SEQ ID NO: 1.

2. The diutan gum of Claim 1 exhibiting a sea water 0.3 rpm viscosity greater than 35,000 cp when the reading is taken using a Brookfield viscometer with LV-2C spindle to measure the viscosity, the speed of the spindle being set to 0.3rpm and the spindle being allowed to rotate at least 6 minutes before the viscosity is recorded.

3. The diutan gum of any of Claims 1 or 2, exhibiting a low shear rate viscosity in the presence of polyethylene glycol dispersant of greater than 3500 cp when the viscosity measurement is taken by measuring the viscosity of a 0.25% solution of biogum in Standard Tap Water (STW) using a Brookfield LV viscometer equipped with a 2.5+ torque spring at 3rpm using the LV 1 spindle after allowing the spindle to rotate for 3 minutes.

4. A method of producing the diutan gum of Claim 1, comprising: introducing a plasmid comprising a nucleic acid sequence according to SEQ ID NO: 1 into a host diutan producing *Sphingomonas* organism of strain ATCC No. 53159; culturing the host organism under fermentation conditions.

5. An isolated nucleic acid molecule which comprises the nucleic acid sequence according to SEQ ID NO: 1.

## Patentansprüche

1. Ein Diutangummi, das eine Grenzviskosität von mehr als 150 dl/g aufweist, wenn die Grenzviskosität durch Auftragen der reduzierten Viskosität über der Lösungskonzentration und Extrapolieren einer linearen Regression der Daten zur Nullkonzentration erhalten wird,
und das eine Meerwasserviskosität bei 3 U/min von mehr als oder gleich einer Skalenablesung von 42 aufweist, wenn die Ablesung vorgenommen wird, indem Probengummi mit synthetischem Meerwasser kombiniert und gemischt wird, dann die Lösung auf ungefähr 26 °C gekühlt und auf eine Fann-Probenplattform platziert wird, bei der die Geschwindigkeit auf 3 U/min eingestellt ist, und die Skala abgelesen wird; und
wobei das Diutangummi unter Verwendung eines *Sphingomonas*-Stamms mit der ATCC-Nr. 53159, der ein Plasmid beinhaltet, welches eine Nukleinsäuresequenz gemäß SEQ ID NO: 1 beinhaltet, hergestellt wird.

2. Diutangummi gemäß Anspruch 1, das eine Meerwasserviskosität bei 0,3 U/min von mehr als 35 000 cP aufweist, wenn die Ablesung unter Verwendung eines Brookfield-Viskosimeters mit LV-2C-Spindel vorgenommen wird, um die Viskosität zu messen, wobei die Geschwindigkeit der Spindel auf 0,3 U/min gesetzt ist und sich die Spindel mindestens 6 Minuten drehen kann, bevor die Viskosität aufgezeichnet wird.

3. Diutangummi gemäß einem der Ansprüche 1 oder 2, das eine Viskosität bei niedriger Schergeschwindigkeit in Gegenwart eines Polyethylenglykol-Dispersionsmittels von mehr als 3500 cP aufweist, wenn die Viskositätsmessung vorgenommen wird, indem die Viskosität einer 0,25%igen Lösung von Biogummi in Standardleitungswasser (STW) unter Verwendung eines Brookfield-LV-Viskosimeters, das mit einer 2,5+-Drehkraft-Feder ausgestattet ist, bei 3 U/min unter Verwendung der LV-1-Spindel gemessen wird, nachdem sich die Spindel 3 Minuten drehen konnte.

4. Ein Verfahren zum Produzieren des Diutangummis gemäß Anspruch 1, das Folgendes beinhaltet: Einführen eines Plasmids, das eine Nukleinsäuresequenz gemäß SEQ ID NO: 1 beinhaltet, in einen Diutan produzierenden *Sphingomonas*-Wirtsorganismus des Stammes ATCC-Nr. 53159; Kultivieren des Wirtsorganismus unter Fermentationsbedingungen.

5. Ein isoliertes Nukleinsäuremolekül, das die Nukleinsäuresequenz gemäß SEQ ID NO: 1 beinhaltet.

## Revendications

1. Une gomme de diutane présentant une viscosité intrinsèque supérieure à 150 déciL/g lorsque la viscosité intrinsèque est obtenue en traçant la viscosité réduite en fonction de la concentration de la solution, et en extrapolant une régression linéaire des données jusqu'à la concentration zéro,
et présentant une viscosité à 3 tr/min dans de l'eau de mer supérieure ou égale à une lecture par cadran de 42 lorsque la lecture est prise en combinant de la gomme échantillon avec de l'eau de mer synthétique et en mélangeant, puis en refroidissant la solution jusqu'à approximativement 26 °C et en la plaçant sur une plate-forme pour échantillons Fann où la vitesse a été réglée à 3 tr/min et en lisant sur le cadran ; et
la gomme de diutane étant réalisée en utilisant la souche de *Sphingomonas* ATCC N° 53159 comprenant un plasmide comprenant une séquence d'acide nucléique selon SEQ ID N° : 1.

2. La gomme de diutane de la revendication 1 présentant une viscosité à 0,3 tr/min dans de l'eau de mer supérieure à 35 000 cP lorsque la lecture est prise en utilisant un viscosimètre Brookfield avec un mobile LV-2C pour mesurer la viscosité, la vitesse du mobile étant fixée à 0,3 tr/min et le mobile étant laissé à tourner pendant au moins 6 minutes avant que la viscosité soit enregistrée.

3. La gomme de diutane de n'importe lesquelles des revendications 1 ou 2, présentant une viscosité à faible taux de cisaillement en présence d'agent dispersant de polyéthylène glycol supérieure à 3 500 cP lorsque la mesure de la viscosité est prise en mesurant la viscosité d'une solution à 0,25 % de biogomme dans de l'eau du robinet standard (STW) en utilisant un viscosimètre Brookfield LV équipé d'un ressort de couple 2,5+ à 3 tr/min en utilisant le mobile LV 1 après avoir laissé le mobile tourner pendant 3 minutes.

4. Une méthode de production de la gomme de diutane de la revendication 1, comprenant : l'introduction d'un plasmide comprenant une séquence d'acide nucléique selon SEQ ID N° : 1 dans un organisme du genre *Sphingomonas* produisant du diutane hôte de souche ATCC N° 53159 ; la mise en culture de l'organisme hôte dans des conditions de fermentation.

5. Une molécule d'acide nucléique isolée qui comprend la séquence d'acide nucléique selon SEQ ID N° : 1.
